# EUROPEAN PATENT APPLICATION

(11) **EP 3 406 632 A1**
(43) Date of publication of application: **28.11.2018**
(21) Application number: 17425051.4
(22) Date of filing: 23.05.2017
(51) Int. Cl.: C07K 16/28, A61P 25/16, A61P 25/28

(54) **LIGANDS BINDING TO PRION PROTEIN FOR USE IN THE TREATMENT OF SYNUCLEINOPATHIES**

(71) Applicant: S.I.S.S.A. Scuola Internazionale Superiore di Studi Avanzati, 34136 Trieste (IT)
(72) Inventor: Legname, Giuseppe Antonio, 34151 Trieste (IT)
(74) Representative: Petraz, Gilberto Luigi

(57) **Abstract**

The present invention provides ligands capable of binding to prion protein, such as anti-prion protein antibodies and antigen-binding fragment thereof, for the prevention and/or treatment of synucleinopathies, such as Parkinson's disease. The present invention also provides pharmaceutical compositions comprising such ligands and methods for treating synucleinopathies or for reducing the uptake of α-synuclein fibrils.

## Description

The present invention relates to the field of synucleinopathies, in particular to immunotherapy of synucleinopathies, such as Parkinson's disease (PD).

Parkinson's disease (PD) is the world's second most common neurodegenerative disease and most common movement disorder. In 2015, PD affected 6.2 million people and resulted in about 117,400 deaths globally. PD is a long-term neurodegenerative disease that mainly affects the motor system. Usually, the symptoms emerge slowly over time. Early in the disease, symptoms include shaking, rigidity, slowness of movement, and difficulty with walking. Further symptoms include cognitive and behavioral problems, dementia, sensory, sleep and emotional problems. The main motor symptoms are collectively called "parkinsonism", or a "parkinsonian syndrome". PD is characterized by a loss of dopaminergic neurons and the development of intraneuronal inclusions known as Lewy bodies (LB). Classically, PD has been thought of as a cell-autonomous disease.

Recently, however, evidence is accumulating that Parkinson's disease progresses through non-cell autonomous mechanisms - contrary to the classical hypotheses. Namely, it was observed that Lewy bodies and neurites spread from diseased tissue to young, transplanted neurons in PD patients, suggesting that PD pathology can be propagated to neighboring cells (J. H. Kordower, Y. Chu, R. A. Hauser, T. B. Freeman, and C. W. Olanow, "Lewy body-like pathology in long-term embryonic nigral transplants in Parkinson's disease," Nature Medicine, vol. 14, no. 5, pp. 504-506, 2008; J.-Y. Li, E. Englund, J. L. Holton et al., "Lewy bodies in grafted neurons in subjects with Parkinson's disease suggest host-to-graft disease propagation," Nature Medicine, vol. 14, no. 5, pp. 501-503, 2008). Subsequent research pointed to a prion-like intercellular transfer of misfolded α-synuclein (a-Syn) as key feature of PD pathology (for review see N. P. Visanji, P. L. Brooks, L. N. Hazrati, and A. E. Lang, "The prion hypothesis in Parkinson's disease: Braak to the future," Acta Neuropathologica Communications, vol. 1, p. 2, 2013; Chauhan A. and Jeans A. F., "Is Parkinson's disease truly a prion-like disorder? An appraisal of current evidence", Neurology Research International, vol. 2015, article ID 345285, 8 pages).

Alpha-synuclein is most abundant in the human brain, however, smaller amounts are found in the heart, muscles, and other tissues. In the brain, α-synuclein is found mainly in presynaptic terminals, where it interacts with phospholipids and proteins. Although the function of α-synuclein is not yet fully characterized, it is suggested to be involved in maintaining the supply of synaptic vesicles in presynaptic terminals by clustering synaptic vesicles. It is also suggested that α-synuclein plays a role in the regulation of dopamine release. Even though α-synuclein is classically considered as an unstructured soluble protein forming a stably folded tetramer, misfolded α-synuclein aggregates to form insoluble fibrils in pathological conditions characterized by Lewy bodies. Point mutations in α-synuclein as well as α-synuclein gene duplications and triplications are associated with Parkinson's disease. (See, e.g., Polymeropoulos et al (1997) Science 276:2045-2047; Kruger et al (1998) Nat Genet 18:106-108; Zarranz et al (2004) Ann Neurol 55:164-173; Kiely et al (2013) Acta Neuropathol 125:753-769; Proukakis et al (2013) Neurology 80:1062-1064; Singleton et al (2003) Science 302:841; and lbanez et al (2004) Lancet 364:1169-1171.) Additionally, α-synuclein is a major component of intracellular protein aggregates called Lewy bodies, which are pathological hallmarks of neurodegenerative disorders such as, for example, Parkinson's Disease, Lewy body disease, and multiple system atrophy. (See, e.g., Spillantini et al (1997) Nature 388:839-840; Wakabayashi et al (1997) Neurosci Lett 239:45-48; Arawaka et al (1998) Neurology 51:887-889; and Gai et al (1998) Lancet 352:547-548.)

Pathological conditions characterized by abnormal accumulation of α-synuclein aggregates are referred to as synucleinopathies or α-synucleinopathies. Synucleinopathies comprise a class of neurodegenerative disorders; the term is used broadly to designate a spectrum of progressive degenerative disorders of the human nervous system. Misfolding and intracellular aggregation of α-synuclein are thought to be crucial factors in the pathogenesis of synucleinopathies that share, among other properties, the presence of abnormal α-synuclein immunoreactive inclusion bodies in neurons and/or macroglial cells. Synucleinopathies include Parkinson's disease (PD), dementia with Lewy bodies, and multiple system atrophy. For example, evidence to date suggests that the misfolding, aggregation, and brain deposition of the alpha-synuclein protein may be triggering factors for PD pathology.

Currently, there is no effective treatment for Parkinson's disease. The most widely used treatment for more than thirty years is levodopa (L-DOPA). Since motor symptoms are produced by a lack of dopamine in the substantia nigra, the administration of L-DOPA temporarily diminishes the motor symptoms. However, only 5-10% of L-DOPA crosses the blood-brain barrier, whereas the remainder is metabolized to dopamine elsewhere, causing a variety of side effects including nausea, dyskinesias and joint stiffness. Levodopa preparations lead in the long term to the development of motor complications characterized by involuntary movements called dyskinesias and fluctuations in the response to medication. When this occurs a person with PD can change from phases with good response to medication and few symptoms ("on" state), to phases with no response to medication and significant motor symptoms ("off" state). As an alternative to L-DOPA, dopamine receptor agonists can be used as an initial therapy for motor symptoms with the aim of delaying motor complications or in late PD to reduce the off periods. Dopamine receptor agonists include bromocriptine, pergolide, pramipexole, ropinirole, piribedil, cabergoline, apomorphine and lisuride. Like dopamine agonists, MAO-B inhibitors used as monotherapy improve motor symptoms and delay the need for levodopa in early disease, but produce more adverse effects and are less effective than levodopa. Other drugs such as amantadine and anticholinergics may be useful as treatment of motor symptoms. However, the evidence supporting them lacks quality, so they are not first choice treatments. Deep brain stimulation by microelectrodes has been used to reduce motor symptoms in severe cases where drugs are ineffective. Evidence for treatments for the non-movement-related symptoms of PD, such as sleep disturbances and emotional problems, is less strong. Examples are the use of quetiapine for psychosis, cholinesterase inhibitors for dementia, and modafinil for daytime sleepiness. In summary, present treatments of PD target the symptoms, such as the motor symptoms, rather than the cause, and are thus ineffective and cannot inhibit or delay the progress of PD.

In view thereof, there is an unmet need for an antiparkinson medication, which relates to the cause and can inhibit or delay the progress of PD. It is thus an object of the present invention to provide a medication for synucleinopathies, which overcomes the drawbacks of the present antiparkinson medications outlined above.

This object is achieved by means of the subject-matter set out below and in the appended claims.

Although the present invention is described in detail below, it is to be understood that this invention is not limited to the particular methodologies, protocols and reagents described herein as these may vary. It is also to be understood that the terminology used herein is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of". The term "comprising" thus encompasses "including" as well as "consisting" *e.g*., a composition "comprising" X may consist exclusively of X or may include something additional *e.g.*, X + Y.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

The word "substantially" does not exclude "completely" *e.g.*, a composition which is "substantially free" from Y may be completely free from Y. Where necessary, the word "substantially" may be omitted from the definition of the invention.

The term "about" in relation to a numerical value x means x ± 10%.

The term "disease" as used herein is intended to be generally synonymous, and is used interchangeably with, the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

As used herein, reference to "treatment" of a subject or patient is intended to include prevention, prophylaxis, attenuation, amelioration and therapy. The terms "subject" or "patient" are used interchangeably herein to mean all mammals including humans. Examples of subjects include humans, cows, dogs, cats, horses, goats, sheep, pigs, and rabbits. Preferably, the subject/patient is a human.

As used herein (i.e., throughout the present application), the term "antibody" as used herein is intended to encompass "immunoglobulins" and derivatives and antigen-binding fragments thereof (for example, certain antibody formats lacking, e.g., the Fc region, single-chain antibody formats, etc.). Conventional Immunoglobulins typically comprise various broad classes of polypeptides that can be distinguished biochemically. In many examples, immunoglobulins consist of combination heavy chains and light chains. All immunoglobulin classes including IgM, IgA, IgD, IgE, IgG and IgY and where appropriate, their subclasses, are within the scope of the present invention. With regard to IgG, a standard immunoglobulin molecule comprises two identical light chain polypeptides of molecular weight approximately 25 kDa, and two identical heavy chain polypeptides of approximate molecular weight 50 kDa. The resulting molecule, which is conventionally referred to as an IgG "monomer" consists of identical halves and the four chains that are typically joined by disulfide bonds in a "Y" configuration wherein the light chains adjoin the heavy chains starting at the mouth of the "Y" and continuing through the variable region or domain. It is well recognized by those skilled in the art that immunoglobulins can be characterized in terms of variable and constant domains. In this regard, it will be appreciated that the variable domains of both the light (VL) and heavy (VH) chain portions determine antigen recognition and specificity. Conversely, the constant domains of the light chain (CL) and the heavy chain (normally consisting of CH1, CH2 or CH3 domains) typically confer important biological properties such as secretion, Fc receptor binding, complement binding, and the like. As indicated above, the variable region allows the antibody to selectively recognize and specifically bind epitopes on antigens. That is, the VL domain and VH domain of an antibody combine to form the variable region that defines a three dimensional antigen binding site, this site is also called the "antigen receptor" or "paratope". More specifically, the antigen binding site is typically defined by three complementarity determining regions (CDRs) on each of the VH and VL chains. Thus within the amino acid sequence of a variable domain of an antibody there are usually three CDRs (known as CDR1 , CDR2 and CDR3). Since most sequence variation associated with immunoglobulins is found in the CDRs, these regions are sometimes referred to as "hypervariable regions", among these CDRs, CDR3 shows the greatest variability. Since the antigen binding sites are typically composed of two variable domains (on two different polypeptide chains being the heavy and light chain), there are usually six CDRs (three heavy chain CDRs and three light chain CDRs) for each antigen receptor that can collectively come into contact with the antigen. Thus, a single conventional IgG molecule has typically two antigen receptors, and therefore comprises twelve CDRs. In some instances, for example certain immunoglobulin molecules derived from camelid species or engineered molecules based on camelid immunoglobulins, a complete immunoglobulin molecule may consist of heavy chains only, with no light chains. See, e.g., Hamers Casterman et al, Nature 363:446 448 (1993) .

As used herein, the term "antibody" encompasses various forms of antibodies, preferably monoclonal antibodies including, but not being limited to, whole antibodies, antibody fragments, human antibodies, chimeric antibodies, recombinant antibodies, humanized antibodies, synthetic antibodies, chemically modified antibodies and genetically engineered antibodies (variant or mutant antibodies) as long as the characteristic properties according to the invention are retained. Preferred examples of antibodies include monoclonal antibodies, bispecific antibodies, minibodies, domain antibodies, synthetic antibodies, antibody mimetics, chimeric antibodies, humanized antibodies, human antibodies, antibody fusions, antibody conjugates, single chain antibodies, antibody derivatives, antibody analogues and fragments thereof, respectively. Recombinant antibodies, in particular recombinant monoclonal antibodies, are more preferred. Techniques for the manipulation and production of recombinant antibodies may be found in Harlow and Lane 'Antibodies-A Laboratory Manual', Cold Spring Harbour press. Moreover, it is also preferred that the antibody is a multichain antibody, i.e. an antibody comprising more than one chain, which is thus different from a single chain antibody. Unless otherwise indicated, the term "antibody" includes, in addition to antibodies comprising two full-length heavy chains and two full-length light chains, also derivatives, variants, and fragments thereof. In some instances an "antibody" may include fewer chains.

Furthermore, the antibody, or the antigen-binding fragment, may be entirely or partially of human origin or humanized. Preferably, at least the (six) CDRs (complementary-determining regions) and/or the framework regions, more preferably the variable regions, are of human origin and/or humanized.

The term "human antibody", as used herein, is intended to include antibodies having variable and constant regions derived from human immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon antigen challenge (see, e.g., Jakobovits, A., et al., Proc. Natl. Acad. Sci. USA 90 (1993) 2551-2555; Jakobovits, A., et al., Nature 362 (1993) 255-258; Bruggemann, M., et al., Year Immunol. 7 (1993) 3340). Human antibodies can also be produced in phage display libraries (Hoogenboom, H. R., and Winter, G., J. Mol. Biol. 227 (1992) 381-388; Marks, J. D., et al., J. Mol. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95). The term "human antibody" as used herein also comprises such antibodies which are modified, e.g. in the variable region and/or in the Fc region, to generate the properties according to the invention. Preferred human antibodies have the amino acid sequence of a human immunoglobulin and include antibodies isolated from human immunoglobulin libraries or from animals transgenic for one or more human immunoglobulins and that do not express endogenous immunoglobulins, as described, for example in, U.S. Pat. No. 5,939,598.

Preferably, the antibodies may be humanized. Humanization of antibodies is known in the art (see, for example, Shalaby et al., J. Exp. Med. 175 (1992), 217; Mocikat et al., Transplantation 57 (1994), 405) and can be easily accomplished by the skilled worker. Further guidance regarding humanization may be found for example in the literature as published by Gregory Winter et al. and in AlmagroJ. C. and Fransson J. (2008) Humainzation of antibodies. Frontiers in Bioscience 13, 1619-1633. In one embodiment, the antibodies (or fragments) may advantageously be humanized by manufacture of chimeric antibodies. The antibodies (or fragments) may advantageously be CDR-grafted. It is also preferred that the antibodies (or fragments) may advantageously be fully humanized (to the extent that the technology permits).

Preferably, the antibody, or the antigen-binding fragment thereof, for use according to the present invention is a monoclonal antibody, or antigen-binding fragment thereof. Herein, a "monoclonal" antibody (mAb or moAb) is understood as antibody made by identical immune cells that are all clones of a unique parent cell, in contrast to polyclonal antibodies which are made from several different immune cells. Generally, it is possible to produce a monoclonal antibody that specifically bind to a specific substance.

Preferably, a "fragment" as used herein has a length of at least 10 amino acids, more preferably at least 25 amino acids, even more preferably at least 50 amino acids, still more preferably at least 100 amino acids, and most preferably at least 200 amino acids, in particular it may comprise the majority of the polypeptide of interest. Suitably a fragment may comprise a whole motif or a whole domain of the polypeptide of interest.

As used herein, the term "antigen" refers to any structural substance which serves as a target for the receptors of an adaptive immune response, in particular as a target for antibodies, T cell receptors, and/or B cell receptors. An "epitope", also known as "antigenic determinant", is the part (or fragment) of an antigen that is recognized by the immune system, in particular by antibodies, T cell receptors, and/or B cell receptors. Thus, one antigen has at least one epitope, i.e. a single antigen has one or more epitopes. An antigen may be (i) a peptide, a polypeptide, or a protein, (ii) a polysaccharide, (iii) a lipid, (iv) a lipoprotein or a lipopeptide, (v) a glycolipid, (vi) a nucleic acid, or (vii) a small molecule drug or a toxin. Thus, an antigen may be a peptide, a protein, a polysaccharide, a lipid, a combination thereof including lipoproteins and glycolipids, a nucleic acid (e.g. DNA, siRNA, shRNA, antisense oligonucleotides, decoy DNA, plasmid), or a small molecule drug (e.g. cyclosporine A, paclitaxel, doxorubicin, methotrexate, 5-aminolevulinic acid), or any combination thereof. Preferably, the antigen is selected from (i) a peptide, a polypeptide, or a protein, (ii) a polysaccharide, (iii) a lipid, (iv) a lipoprotein or a lipopeptide and (v) a glycolipid; more preferably, the antigen is a peptide, a polypeptide, or a protein. In the context of the present invention, the antigen for an "antigen-binding fragment" of an anti-PrP antibody is in particular (a fragment/epitope of) PrP.

Accordingly, in the context of the present invention, the functionality to be preserved in an antigen-binding fragment of an anti-PrP antibody (and in anti-PrP antibodies) is in particular the recognition of (i.e. effective/specific binding to) PrP, preferably to a target region of PrP. Typically this recognition/binding function is mediated by the complementarity determining regions (CDRs) of the antibody. Thus, the ligand according to the present invention is preferably an anti-PrP antibody or an antigen-binding fragment thereof. Such an antibody/antigen-binding fragment typically comprises (six) CDRs recognizing an epitope of PrP. Functional fragments include antigen-binding fragments that bind to an epitope of PrP^{C}. For example, antibody fragments capable of binding including, but not limited to Fab (e.g., by papain digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, are encompassed by the present invention. Antibody fragments are also intended to include, e.g., domain deleted antibodies, diabodies, linear antibodies, single-chain antibody molecules, and multispecific antibodies.

As used herein, the term "recombinant antibody" is intended to include all antibodies, which do not occur in nature, in particular antibodies that are prepared, expressed, created or isolated by recombinant means, such as antibodies isolated from a host cell such as for example a CHO cell or from an animal (e.g. a mouse) or antibodies expressed using a recombinant expression vector transfected into a host cell. Such recombinant antibodies may have variable and constant regions in a rearranged form as compared to naturally occurring antibodies.

As used herein, the terms "antigen binding fragment," "fragment," and "antibody fragment" are used interchangeably to refer to any fragment of an antibody of the invention that preferably retains the specific binding activity of the antibody for use according to the invention, in particular the ability to bind to prion protein and/or the ability to reduce uptake of α-synuclein fibrils. Examples of antibody fragments include, but are not limited to, sc (single chain) antibody, scFv-Fc, scFv-CH3, scDiabody-CH3, Diabody-CH3, minibody, scFv-KIH, Fab-scFv-Fc, scDiabody-Fc, Diabody-Fc, and tandem scFv-Fc (*e.g*., as described in Spiess C., Zhai Q. and Carter P.J. (2015) Molecular Immunology 67: 95-106). Fragments of the antibodies of the invention can be obtained from the antibodies by methods that include digestion with enzymes, such as pepsin or papain, and/or by cleavage of disulfide bonds by chemical reduction. Alternatively, fragments of antibodies can be obtained by cloning and expression of part of the sequences of the heavy and/or light chains. The invention also encompasses single-chain Fv fragments (scFv) including the CH3 region derived from the heavy and light chains of an antibody of the invention. For example, the invention includes a scFv-CH3 or a scFv-Fc comprising the CDRs from an antibody of the invention. Also included are heavy or light chain monomers and dimers, single domain heavy chain antibodies, single domain light chain antibodies, as well as single chain antibodies, e.g., single chain Fv in which the heavy and light chain variable domains are joined by a peptide linker. Antibody fragments of the invention are typically multivalent and may be contained in a variety of structures as described above. For instance, scFv molecules may be synthesized to create a trivalent "triabody" or a tetravalent "tetrabody." The scFv molecules preferably include a domain of the Fc region. Although the specification, including the claims, may, in some places, refer explicitly to antigen binding fragment(s), antibody fragment(s), variant(s) and/or derivative(s) of antibodies, it is understood that the term "antibody" or "antibody of the invention" includes all categories of antibodies, namely, antigen binding fragment(s), antibody fragment(s), variant(s) and derivative(s) of antibodies.

Antigen-binding fragments may comprise, for example, at least one heavy or light chain CDR of an antibody molecule. An antigen binding fragment may also comprise at least two CDRs from one or more antibody molecules. An antigen binding fragment may also comprise at least three CDRs from one or more antibody molecules. An antigen binding fragment may also comprise at least four CDRs from one or more antibody molecules. An antigen binding fragment may also comprise at least five CDRs from one or more antibody molecules. An antigen binding fragment may also comprise at least six CDRs from one or more antibody molecules. Antibodies or immunospecific fragments thereof for use according to the invention include, but are not limited to, polyclonal, monoclonal, multispecific, human, humanized, primatized, or chimeric antibodies, single chain antibodies, epitope-binding fragments, e.g., Fab, Fab' and F(ab']2, Fd, Fvs, single-chain Fvs (scFv), single-chain antibodies, disulfide-linked Fvs (sdFv), fragments comprising either a VL or VH domain, fragments produced by a Fab expression library, and anti-idiotypic (anti-Id) antibodies (including, e.g., anti-Id antibodies to binding molecules disclosed herein). ScFv molecules are known in the art and are produced using recombinant DNA technology. Immunoglobulin or antibody molecules of the invention may be of any isotype (e.g., IgG, IgE, IgM, IgD, IgA, and IgY), class (e.g., IgG1, IgG2, IgG3, IgG4, IgA1 and IgA2) or subclass of immunoglobulin molecule. Preferably antibodies are of IgG isotype. Within the IgG isotype, antibodies may be IgG1, IgG2, IgG3 or IgG4 subclass, whereby IgG1 is preferred. Antibodies of the invention may have a κ or a λ light chain.

The term "antibody" as used herein is also intended to encompass antibodies, digestion fragments, specified portions and variants thereof, including antibody mimetics or comprising portions of antibodies that mimic the structure and/or function of an antibody or specified fragment or portion thereof, including single chain antibodies and fragments thereof; each containing at least one CDR. See Qiu et al., Nature Biotechnology 25:921 -929 (2007). Functional fragments include antigen binding fragments that bind to a PrP^{C} antigen. For example, antibody fragments capable of binding to PrP or a portion thereof, including, but not limited to Fab (e.g., by papain digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, are encompassed by the present invention. Antibody fragments are also intended to include for example, domain deleted antibodies, linear antibodies, single-chain antibody molecules, multispecific antibodies formed from antibody fragments and diabodies. Diabodies are typically formed by the creation of scFvs with linker peptides that are too short for the two variable regions to fold together (about five amino acids), forcing scFvs to dimerize. Modified versions of each of these categories of recombinant antibody fragments and combinations thereof will be discernible to the skilled person and are within the scope of the present invention. Antigen-binding fragments, including single-chain antibodies, preferably comprise the variable region(s) alone or in combination with the entirety or a portion of the following: hinge region, CH1, CH2, and CH3 domains. Also included in the invention are antigen-binding fragments comprising any combination of variable region(s) with a hinge region, CH1, CH2, and CH3 domains. Antibodies or immunospecific fragments thereof may be from any animal origin including birds and mammals, however, mammals are preferred. Preferably, the antibodies are human, murine, donkey, rabbit, goat, guinea pig, camel, Ilama, horse, or chicken antibodies.

Antibodies and antibody fragments of the invention may impart monovalent or multivalent interactions and be contained in a variety of structures as described above. For instance, scFv molecules may be synthesized to create a trivalent "triabody" or a tetravalent "tetrabody." The scFv molecules may include a domain of the Fc region resulting in bivalent minibodies. In addition, the sequences of the invention may be a component of multispecific molecules in which the sequences of the invention target the epitopes of the invention and other regions of the molecule bind to other targets. Exemplary molecules include, but are not limited to, bispecific Fab2, trispecific Fab3, bispecific scFv, and diabodies (Holliger and Hudson, 2005, Nature Biotechnology 9: 1126-1136).

Antibodies according to the present invention may be provided in purified form. Typically, the antibody will be present in a composition that is substantially free of other polypeptides *e.g.*, where less than 90% (by weight), usually less than 60% and more usually less than 50% of the composition is made up of other polypeptides.

Doses are often expressed in relation to the bodyweight. Thus, a dose which is expressed as [g, mg, or other unit]/kg (or g, mg etc.) usually refers to [g, mg, or other unit] "per kg (or g, mg etc.) bodyweight", even if the term "bodyweight" is not explicitly mentioned.

The term "specifically binding" and similar reference does not encompass non-specific sticking.

As used herein, "sequence variant" (also referred to as "variant") refers to any alteration in a reference sequence, whereby a reference sequence is any of the sequences listed in the "Tables of Sequences and SEQ ID Numbers" (sequence listing), i.e. SEQ ID NO: 1 to SEQ ID NO: 8. Thus, the term "sequence variant" includes nucleotide sequence variants and amino acid sequence variants. Of note, the sequence variants referred to herein are in particular functional sequence variants, i.e. sequence variants maintaining the biological function of, for example, the PrP or of an antibody. In the context of the present invention such a maintained biological function is preferably the ability of PrP to bind to α-synuclein and/or to bind to an anti-PrP antibody or the ability of an anti-PrP antibody to bind to PrP. Preferred sequence variants are thus functional sequence variants having at least 70%, at least 75%, at least 80%, at least 85%, at least 88%, at least 90%, at least 92%, at least 95%, at least 96%, at least 97%, at least 98% or at least 99% sequence identity to a reference sequence. The phrase *"functional sequence variant thereof having at least 70%, at least 75%, at least 80%, at least 85%, at least 88%, at least 90%, at least 92%, at least 95%, at least 96%, at least 97%, at least 98% or at least 99% sequence identity",* as used herein, means (i) that the sequence variant is functional as described herein and (ii) the higher the % sequence identity, the more preferred the sequence variant. In other words, the phrase *"functional sequence variant thereof having at least 70%, at least 75%, at least 80%, at least 85%, at least 88%, at least 90%, at least 92%, at least 95%, at least 96%, at least 97%, at least 98% or at least 99% sequence identity",* means in particular that the functional sequence variant has at least 70% sequence identity, preferably at least 75% sequence identity, preferably at least 80% sequence identity, more preferably at least 85% sequence identity, more preferably at least 88% sequence identity, even more preferably at least 90 % sequence identity, even more preferably at least 92% sequence identity, still more preferably at least 95% sequence identity, still more preferably at least 96% sequence identity, particularly preferably at least 97% sequence identity, particularly preferably at least 98% sequence identity and most preferably at least 99% sequence identity to the respective reference sequence.

The term "sequence variant" includes in particular such variants that comprise mutations and/or substitutions in comparison to the reference sequence.

Sequence identity is usually calculated with regard to the full length of the reference sequence (i.e. the sequence recited in the application). Percentage identity, as referred to herein, can be determined, for example, using BLAST using the default parameters specified by the NCBI (the National Center for Biotechnology Information; http://www.ncbi.nlm.nih.gov/) [Blosum 62 matrix; gap open penalty=11 and gap extension penalty=1].

As used herein, a "nucleotide sequence variant" has an altered sequence in which one or more of the nucleotides in the reference sequence is deleted, or substituted, or one or more nucleotides are inserted into the sequence of the reference nucleotide sequence. Nucleotides are referred to herein by the standard one-letter designation (A, C, G, or T). Due to the degeneracy of the genetic code, a "nucleotide sequence variant" can either result in a change in the respective reference amino acid sequence, i.e. in an "amino acid sequence variant" or not. Preferred sequence variants are such nucleotide sequence variants, which do not result in amino acid sequence variants (silent mutations), but other non-silent mutations are within the scope as well, in particular mutant nucleotide sequences, which result in an amino acid sequence, which is at least 80%, preferably at least 90 %, more preferably at least 95% sequence identical to the reference sequence.

An "amino acid sequence variant" has an altered sequence in which one or more of the amino acids in the reference sequence is deleted or substituted, or one or more amino acids are inserted into the sequence of the reference amino acid sequence. As a result of the alterations, the amino acid sequence variant has an amino acid sequence which is at least 80% identical to the reference sequence, preferably, at least 90% identical, more preferably at least 95% identical, most preferably at least 99% identical to the reference sequence. Variant sequences which are at least 90% identical have no more than 10 alterations, i.e. any combination of deletions, insertions or substitutions, per 100 amino acids of the reference sequence.

While it is possible to have non-conservative amino acid substitutions, it is preferred that the substitutions be conservative amino acid substitutions, in which the substituted amino acid has similar structural or chemical properties with the corresponding amino acid in the reference sequence. By way of example, conservative amino acid substitutions involve substitution of one aliphatic or hydrophobic amino acids, e.g. alanine, valine, leucine and isoleucine, with another; substitution of one hydoxyl-containing amino acid, e.g. serine and threonine, with another; substitution of one acidic residue, e.g. glutamic acid or aspartic acid, with another; replacement of one amide-containing residue, e.g. asparagine and glutamine, with another; replacement of one aromatic residue, e.g. phenylalanine and tyrosine, with another; replacement of one basic residue, e.g. lysine, arginine and histidine, with another; and replacement of one small amino acid, e.g., alanine, serine, threonine, methionine, and glycine, with another.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include the fusion to the N- or C-terminus of an amino acid sequence to a reporter molecule or an enzyme.

Importantly, the alterations in the sequence variants do not abolish the functionality of the respective reference sequence, in the present case, e.g., the functionality of a sequence of PrP or of an antibody, or antigen binding fragment thereof. Guidance in determining which nucleotides and amino acid residues, respectively, may be substituted, inserted or deleted without abolishing such functionality are found by using computer programs well known in the art.

As used herein, a nucleic acid sequence or an amino acid sequence "derived from" a designated nucleic acid, peptide, polypeptide or protein refers to the origin of the nucleic acid, peptide, polypeptide or protein. Preferably, the nucleic acid sequence or amino acid sequence which is derived from a particular sequence has an amino acid sequence that is essentially identical to that sequence or a portion thereof, from which it is derived, whereby "essentially identical" includes sequence variants as defined above. Preferably, the nucleic acid sequence or amino acid sequence which is derived from a particular peptide or protein, is derived from the corresponding domain in the particular peptide or protein. Thereby, "corresponding" refers in particular to the same functionality. For example, an "extracellular domain" corresponds to another "extracellular domain" (of another protein), or a "transmembrane domain" corresponds to another "transmembrane domain" (of another protein). "Corresponding" parts of peptides, proteins and nucleic acids are thus easily identifiable to one of ordinary skill in the art. Likewise, sequences "derived from" other sequence are usually easily identifiable to one of ordinary skill in the art as having its origin in the sequence.

Preferably, a nucleic acid sequence or an amino acid sequence derived from another nucleic acid, peptide, polypeptide or protein may be identical to the starting nucleic acid, peptide, polypeptide or protein (from which it is derived). However, a nucleic acid sequence or an amino acid sequence derived from another nucleic acid, peptide, polypeptide or protein may also have one or more mutations relative to the starting nucleic acid, peptide, polypeptide or protein (from which it is derived), in particular a nucleic acid sequence or an amino acid sequence derived from another nucleic acid, peptide, polypeptide or protein may be a functional sequence variant as described above of the starting nucleic acid, peptide, polypeptide or protein (from which it is derived). For example, in a peptide/protein one or more amino acid residues may be substituted with other amino acid residues or one or more amino acid residue insertions or deletions may occur.

As used herein, the term "mutation" relates to a change in the nucleic acid sequence and/or in the amino acid sequence in comparison to a reference sequence, e.g. a corresponding genomic sequence. A mutation, e.g. in comparison to a genomic sequence, may be, for example, a (naturally occurring) somatic mutation, a spontaneous mutation, an induced mutation, e.g. induced by enzymes, chemicals or radiation, or a mutation obtained by site-directed mutagenesis (molecular biology methods for making specific and intentional changes in the nucleic acid sequence and/or in the amino acid sequence). Thus, the terms "mutation" or "mutating" shall be understood to also include physically making a mutation, e.g. in a nucleic acid sequence or in an amino acid sequence. A mutation includes substitution, deletion and insertion of one or more nucleotides or amino acids as well as inversion of several successive nucleotides or amino acids. To achieve a mutation in an amino acid sequence, preferably a mutation may be introduced into the nucleotide sequence encoding said amino acid sequence in order to express a (recombinant) mutated polypeptide. A mutation may be achieved e.g., by altering, e.g., by site-directed mutagenesis, a codon of a nucleic acid molecule encoding one amino acid to result in a codon encoding a different amino acid, or by synthesizing a sequence variant, e.g., by knowing the nucleotide sequence of a nucleic acid molecule encoding a polypeptide and by designing the synthesis of a nucleic acid molecule comprising a nucleotide sequence encoding a variant of the polypeptide without the need for mutating one or more nucleotides of a nucleic acid molecule.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

It is to be understood thatthis invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

### Ligands capable of binding prion protein

In a first aspect the present invention provides a ligand capable of binding to prion protein (PrP) for use in the prevention and/or treatment of a synucleinopathy.

The present invention is based on the surprising finding that ligands capable of binding prion protein (PrP), such as anti-prion protein antibodies, are able to reduce the uptake and spreading of α-synuclein fibrils in neuronal cells, which is a hallmark of synucleinopathies. Moreover, the present inventors have surprisingly found that binding of α-synuclein to prion protein facilitates and promotes uptake and spreading of α-synuclein fibrils. Without being bound to any theory, it is assumed that binding of prion protein to a ligand distinct from α-synuclein impairs or inhibits the binding of prion protein to α-synuclein, thereby reducing uptake and spreading of α-synuclein fibrils in neuronal cells and, thus, α-synuclein-related neurotoxicity. Since the prion-like intercellular transfer of misfolded α-synuclein is a key feature of synucleinopathies, such as Parkinson's disease (PD), reduction of cellular uptake and spreading of α-synuclein is thought to delay or inhibit the progress of synucleinopathies, such as PD, instead of targeting a selected symptom only. Moreover, because the brain has low regeneration capacity, early diagnosis of a synucleinopathy, such as PD, is crucial to permit intervention before irreversible neuropathological changes occur. Several lines of evidence indicate that the process of α-synuclein misfolding and aggregation may begin years or decades before the onset of clinical symptoms and substantial brain damage. Accordingly, reduction or blockade of α-synuclein uptake and spreading can also prevent the clinical manifestation of synucelinopathies, such as PD.

Prion protein (PrP) is a well characterized and studied protein. Major prion protein (PrP) also known as CD230 (cluster of differentiation 230) is a protein that in humans is encoded by the PRNP gene (PRioN Protein). The human PRNP gene is located on the short (p) arm of chromosome 20 between the end (terminus) of the arm and position 12, from base pair 4,615,068 to base pair 4,630,233. More than 20 mutations in the PRNP gene have been identified in people with inherited prion diseases, which include Creutzfeldt-Jakob disease, Gerstmann-Sträussler-Scheinker syndrome and fatal familial insomnia. Prion protein (PrP) is found throughout the body even in healthy individuals, in particular PrP is expressed in the brain and several other tissues. PrP found in infectious material has a different structure and is resistant to proteases. The common (non-pathological) form of PrP is cellular prion protein (PrP^{C}), whereas the infectious form is PrP^{Sc}, wherein the "Sc" refers to "scrapie". While PrP^{C} is structurally well-defined, PrP^{Sc} may be polydisperse and is presently defined only at a relatively poor level.

Cellular prion protein (PrP^{C}) is a normal protein found on the membranes of cells. In humans, it has 208 amino acids (see, for example, SEQ ID NO: 3), one disulfide bond, a molecular mass of 35-36 kDa and a mainly alpha-helical structure. Several topological forms exist; one cell surface form anchored via glycolipid and two transmembrane forms. PrP^{C} is readily digested by proteinase K.

Although the exact 3D structure of PrP^{Sc} is not known, it has a higher proportion of β-sheet structure in place of the normal α-helix structure. PrP^{Sc} is able to convert normal PrP^{C} proteins into the infectious isoform by changing their conformation, or shape; this, in turn, alters the way the proteins interconnect. PrP^{Sc} always causes prion disease. Aggregations of these abnormal isoforms form highly structured amyloid fibers, which accumulate to form plaques.

The physiological function of the prion protein remains a controversial matter. While data from *in vitro* experiments suggest many dissimilar roles, studies on PrP knockout mice have provided only limited information because these animals exhibit only minor abnormalities. Accordingly, the abolition of neuronal PrP expression in the adult murine nervous system is without serious consequence (See, e.g., Mallucci, G. et al. (2002) Post-natal knockout of prion protein alters hippocampal CA 1 properties, but does not result in neurodegeneration. EMBO J. 21, 202-210; Mallucci G. et al. (2003) Depleting neuronal PrP in prion infection prevents disease and reverses spongiosis. Science 302, 871-874) and both small molecule (See, e.g., Nicoll, A. J. & CollingeJ. Preventing prion pathogenicity by targeting the cellular prion protein. Infect. Disord. Drug Targets 9, 48-57 - 2009) and monoclonal antibody therapeutics have been extensively studied. Indeed therapeutic molecular interactions with PrP^{C} have been characterized and fully humanized anti-PrP monoclonal antibodies have been produced for clinical studies in human prion disease (See, e.g., Antonyuk, S. V. et al. Crystal structure of human prion protein bound to a therapeutic antibody. Proc. Natl Acad. Sci. USA 106, 2554-2558 - 2009; Nicoll, A. J. et al. Pharmacological chaperone for the structured domain of human prion protein. Proc. Natl. Acad. Sci. USA 107, 1 7610-17615 - 2010).

As used herein, the terms "prion," "prion protein," "PrP protein," and "PrP" are used interchangeably to refer to both the pathogenic prion protein form (also referred to as scrapie protein, pathogenic protein form, pathogenic isoform, pathogenic prion and PrP^{Sc}) and the non-pathogenic prion form (also referred to as the normal form, cellular protein form, cellular isoform, nonpathogenic isoform, nonpathogenic prion protein and PrP^{C}), as well as the denatured form and various recombinant forms of the prion protein that may not have either the pathogenic conformation or the normal cellular conformation. Preferably, the prion protein is the cellular prion protein (PrP^{C}).

One of ordinary skill in the art in view of the teachings of the present disclosure and the art can determine regions corresponding to the sequences disclosed herein in any other prion proteins, using for example, sequence comparison programs (e.g., Basic Local Alignment Search Tool (BLAST)) or identification and alignment of structural features or motifs. "Pathogenic" means that the protein actually causes the disease, or the protein is associated with the disease and, therefore, is present when the disease is present. Thus, a pathogenic protein, as used herein, is not necessarily a protein that is the specific causative agent of a disease. A "pathogenic form" of a protein means a conformation of a protein that is present when the disease is present, but it may or may not be infectious. An example of a pathogenic conformational disease protein, or a pathogenic form of a protein, is PrP^{Sc}. Accordingly, the term "non-pathogenic" or "normal form" describes a protein that does not normally cause disease or is not normally associated with causing disease. An example of a non-pathogenic or a normal form of conformational disease protein is PrP^{C}.

Prion protein may be from any mammalian species such as cow, sheep, mouse, hamster, human or any other mammal. Preferably, PrP is livestock or human PrP. Most preferably, PrP is human PrP. Accordingly, the prion protein sequence may be derived from any mammalian PRNP gene, preferably, it is derived from human or mouse PRNP gene. Preferred examples of PrP amino acid sequences include SEQ ID NOs: 1 - 3 and sequence variants thereof.






(SEQ ID NO: 1; human PrP)

MANLGYWLLALFVTMWTDVGLCKKRPKPGGWNTGGSRYPGQGSPGGNRYPPQGGTWG
QPHGGGWGQPHGGSWGQPHGGSWGQPHGGGWGQGGGTHNQWNKPSKPKTNLKH
VAGAAAAGAVVGGLGGYMLGSAMSRPMIHFGNDWEDRYYRENMYRYPNQVYYRPVDQY
SNQNNFVHDCVNITIKQHTVTTTTKGENFTETDVKMMERVVEQMCVTQYQKESQAYYDGR
RSSSTVLFSSPPVILLISFLIFLIVG
(SEQ ID NO: 2; mouse PrP)

### (SEQ ID NO: 2; mouse PrP)

Preferably, the ligand capable of binding prion protein is capable of binding human major prion protein according to SEQ ID NO: 1, or a sequence variant thereof, or mouse major prion protein according to SEQ ID NO: 2, or a sequence variant thereof. More preferably, the ligand is capable of binding human major prion protein according to SEQ ID NO: 1, or a sequence variant thereof. Since amino acids 1 - 22 of SEQ ID NO: 1 refer to a signal peptide and amino acids 231 - 253 are removed in the mature form of human PrP, it is even more preferred that the ligand capable of binding prion protein is capable of binding human major prion protein according to SEQ ID NO: 3 (which corresponds to amino acids 23 - 230 of SEQ ID NO: 1), or a sequence variant thereof:

KKRPKPGGWNTGGSRYPGQGSPGGNRYPPQGGGGWGQPHGGGWGQPHGGGWGQP
HGGGWGQPHGGGWGQGGGTHSQWNKPSKPKTNMKHMAGAAAAGAVVGGLGGYML
GSAMSRPIIHFGSDYEDRYYRENMHRYPNQVYYRPMDEYSNQNNFVHDCVNITIKQHTVTTT
TKGENFTETDVKMMERVVEQMCITQYERESQAYYQRGS
(SEQ ID NO: 3; human PrP, amino acids 23 - 230)

If it is herein referred to specific amino acid residues of prion protein (PrP), the numbering is taken using human prion protein amino acid sequence (SEQ ID NO:1) as reference sequence. The skilled person can easily determine any amino acid corresponding to a specific amino acid of SEQ ID NO: 1 by aligning the query sequence to SEQ ID NO: 1 and identifying the amino acid in the position corresponding to the specific amino acid in SEQ ID NO: 1.

Preferably, the ligand capable of binding prion protein is capable of binding to cellular prion protein (PrP^{C}), more preferably to human or mouse cellular prion protein (PrP^{C}) and most preferably to human cellular prion protein (PrP^{C}).

More preferably, the ligand capable of binding prion protein is capable of binding to the N-terminal part and/or to the C-terminal part of (cellular) prion protein, more preferably to the N-terminal part and/or to the C-terminal part of human or mouse (cellular) prion protein and most preferably to the N-terminal part and/or to the C-terminal part of human (cellular) prion protein.

Preferably, the ligand capable of binding prion protein is capable of binding to the N-terminal part of (cellular) prion protein, in particular of human PrP^{C}. The "N-terminal part" of (cellular) prion protein refers preferably to amino acids 23 - 123 of SEQ ID NO: 1 (which corresponds to the N-terminal part of SEQ ID NO: 3) or to corresponding amino acids in another prion protein sequence. The N-terminal part is also known as "flexible tail" (FT) of PrP^{C}. The N-terminal part of prion protein comprises a stretch of several the octapeptide repeats (OR; e.g. amino acids 50 - 90 of SEQ ID NO: 1), which are flanked by two positively charged clusters, namely "charged cluster 1" (CC1; e.g. amino acids 23 - 27 of SEQ ID NO: 1) and "charged cluster 2" (CC2; e.g. amino acids 95 - 110 of SEQ ID NO: 1).

Preferably, the ligand capable of binding prion protein is capable of binding to the C-terminal part of (cellular) prion protein, in particular of human PrP^{C}. The "C-terminal part" of (cellular) prion protein refers preferably to amino acids 124 - 230 of SEQ ID NO: 1 (which corresponds to the C-terminal part of SEQ ID NO: 3) or to corresponding amino acids in another prion protein sequence. The C-terminal part is also known as "globular domain" (GD) of PrP^{C}. The structure of the C-terminal part of human PrP^{C} is identical to many other mammals, and comprises three α-helices with helix 1 including amino acids 143 to 156 of SEQ ID NO: 1. Ligands capable of binding to helix 1 of the PrP^{C} are known in the art, for example antibody ICSM-18 as disclosed in WO 2004/050120 and commercially available from D-Gen Limited, UK.

More preferably, the ligand is capable of binding to two distinct epitopes/binding sites of the (cellular) prion protein, preferably of human (cellular) prion protein. Such distinct epitopes/binding sites are preferably non-overlapping. Even more preferably, the ligand is capable of binding (i) to an epitope in the N-terminal part of the (cellular) prion protein, preferably of human (cellular) prion protein, and (ii) to an epitope in the C-terminal part of the (cellular) prion protein, preferably of human (cellular) prion protein.

The ligand is preferably not (neuro)toxic. This means in particular that the ligand is preferably not (neuro)toxic *in vivo* and/or in humans.

As used herein, a "ligand" may be a single entity or it may be a combination of entities, whereby a ligand being a single entity is preferred. The ligand may be an organic or an inorganic compound. The ligand may be natural or artificial. The ligand may be a nucleic acid molecule, an amino acid molecule, a polypeptide, or a chemical derivative thereof, or a combination thereof. The ligand may be designed or obtained from a library of compounds, which may comprise peptides, as well as other compounds, such as small organic molecules. By way of example, the ligand may be a natural substance, a biological macromolecule, or an extract made from biological materials such as bacteria, fungi, or animal (particularly mammalian) cells or tissues, an organic or an inorganic molecule, a synthetic agent, a semisynthetic agent, a structural or functional mimetic, a peptide, a peptidomimetic, a derivatized agent, a peptide cleaved from a whole protein, or a peptide synthesized synthetically (such as, by way of example, either using a peptide synthesizer or by recombinant techniques or combinations thereof), a recombinant ligand, an antibody, a natural or a non-natural ligand, a fusion protein or equivalent thereof and mutants, derivatives or combinations thereof. Preferably the ligand is a molecule, i.e. an electrically charged or neutral group of two or more atoms - rather than a (monoatomic) ion. More preferably, the ligand is not copper, zinc, manganese or nickel. It is also preferred that the ligand is distinct from α-synuclein, i.e. the ligand is preferably not α-synuclein.

A variety of prion protein (PrP) ligands are known in the art. Examples of PrP ligands include Pli45, Pli110, Pli3, Pli4, Pli5, Pli6, Pli7, Pli8, stress-inducible protein 1, the G-protein coupled receptor Adgrg6, tetraspanin-7, bifunctional affinity ligands based on triazine scaffold (e.g. as described in Soto Renou et al., (2004) "The design, synthesis and evaluation of affinity ligands for prion proteins", Journal of Molecular Recognition 17(3): 248-261), and the ligands described in WO 2004/050851.

Most preferably, the ligand capable of binding to prion protein is an anti-prion protein antibody (anti-PrP antibody), or an antigen-binding fragment thereof. Anti-PrP antibodies (or antigen-binding fragments thereof) are in particular characterized in that they bind (effectively and/or specifically) to PrP, preferably to PrP^{C}, such as mammalian PrP^{C} as described herein, in particular human PrP^{C}.

Examples of anti-PrP antibodies and antigen-binding fragments thereof are well-known in the art. Examples of anti-PrP antibodies and antigen-binding fragments thereof include, but are not limited to, the following:
- the POM monoclonals, such as POM1, POM2, POM3, POM4, POM5, POM6, POM7, POM8, POM9, POM10, POM11, POM12, POM13, POM14, POM15, POM16, POM17, POM18, and POM19 and antigen-binding fragments thereof, for example as described in Polymenidou M. et al. (2008) "The POM monoclonals: a comprehensive set of antibodies to non-overlapping prion protein epitopes", PLoS one 3(12): e3872, Polymenidou et al. (2005) Lancet Neurol. 4:805-814;
- antibodies of the ICSM series, such as ICSM1, ICSM2, ICSM3, ICSM4, ICSM5, ICSM6, ICSM7, ICSM8, ICSM9, ICSM10, ICSM11, ICSM12, ICSM13, ICSM14, ICSM15, ICSM16, ICSM17, ICSM18, ICSM19, ICSM20, ICSM21, ICSM22, ICSM23, ICSM24, ICSM25, ICSM26, ICSM27, ICSM28, ICSM29, ICSM30, ICSM31, ICSM32, ICSM33, ICSM34, ICSM35, ICSM36, ICSM37, ICSM38, ICSM39, ICSM40, ICSM41, and ICSM42, for example as described in: Beringue V. et al. (2003) "Regional heterogeneity of cellular prion protein isoforms in the mouse brain", Brain 126: 2065-2073, Tayebi M. et al. (2004) "Disease-associated prion protein elicits immunoglobulin M responses in vivo", Mol Med 10: 104-111, Antonyuk S. V. et al: "Crystal structure of human prion protein bound to a therapeutic antibody", PNAS 106(8): 2554-2558, WO 2004/050120 and WO 2012/156666;
- antibody 3F4, as described in WO 00/29850 or WO 00/22438 (recognizing region 109-112 of PrP);
- antibodies of the SAF-series, such as SAF-1- SAF-90, for example as described in U.S. Patent No. 7,097,997 B1 and in Demart, S., Fournier, J. G., Creminon, C., Frobert, Y., Lamoury, F., Marce, D., Lasmezas, C., Dormont, D., Grassi, J., and Deslys, J. P. (1999) Biochem. Biophys. Res. Commun. 265, 652-657, in particular SAF-15, SAF-31, SAF-32, SAF-33, SAF-34, SAF-35 and SAF-37 (recognizing OR of PrP);
- antibodies of the DPZ series, for example as described in Krasemann S. et al. (1996) Molecular Medicine 2(6):725-734, in Krasemann, S., Groschup, M., Hunsmann, G., and Bodemer, W. (1996) J. Immunol. Methods 199, 109-118 and in Krasemann, S., Jurgens, T., and Bodemer, W. (1999) J. Biotechnol. 73, 119-129, such as antibodies 11C6, 14D3, 4F2, 8G8, 12F10, 13F10, 11B9, 3B5;
- the anti-PrP antibodies described in Pankiewicz J, Prelli F, Sy M-S, et al. Clearance and prevention of prion infection in cell culture by anti-PrP antibodies. The European journal of neuroscience. 2006;23(10):2635-2647; Zanusso G, Liu DC, Ferrari S, Hegyi I, Yin XH, Aguzzi A, Hornemann S, Liemann S, Glockshuber R, Manson JC, Brown P, Petersen RB, Gambetti P, Sy MS. Prion protein expression in different species: Analysis with a panel of new mAbs. Proc Natl Acad Sci USA. 1998;95:8812-8816; Liu T, Zwingman T, Li R, Pan T, Wong BS, Petersen RB, Gambetti P, Herrup K, Sy MS. Differential expression of cellular prion protein in mouse brain as detected with multiple anti-PrP monoclonal antibodies. Brain Res. 2001;896:118-129; Pan T, Li RR, Wong BS, Liu T, Gambetti P, Sy MS. Heterogeneity of normal prion protein in two-dimensional immunoblot: presence of various glycosylated and truncated forms. J Neurochem. 2002;81:1092-1101; Pan T, Li RL, Kang SC, Wong BS, Wisniewski T, Sy MS. Epitope scanning reveals gain and loss of strain specific antibody binding epitopes associated with the conversion of normal cellular prion to scrapie prion. J Neurochem. 2004;90:1205-1217; Pan T, Li RL, Kang SC, Pastore M, Wong BS, Ironside J, Gambetti P, Sy MS. Biochemical fingerprints of prion diseases: scrapie prion protein in human prion diseases that share prion genotype and type. J Neurochem. 2005;92:132-142; Wong BS, Li R, Sassoon J, Kang SC, Liu T, Pan T, Greenspan NS, Wisniewski T, Brown DR, Sy MS. Mapping the antigenicity of copper-treated cellular prion protein with the scrapie isoform. Cell Mol Life Sci. 2003;60:1224-1234; Wong BS, Li R, Sassoon J, Kang SC, Liu T, Pan T, Greenspan NS, Wisniewski T, Brown DR, Sy MS. Mapping the antigenicity of copper-treated cellular prion protein with the scrapie isoform. Cell Mol Life Sci. 2003;60:1224-1234, such as 6D11, 8B4, 11G5, 7H6, 7A12, 2C2, 8H4, 8F9 and 9H7;
- antibody 6H4 (recognizing amino acids 142-151), for example available from Prionics AG, Wagistrasse 27a, CH-8952 Schlieren-Zürich, Switzerland, and described in C. Korth, B. Stierli, P. Streit, M. Moser, O. Schaller, R. Fischer, W. Schulz-Schaeffer, H. Kretzschmar, A. Raeber, U. Braun, F. Ehrensperger, S. Hornemann, R. Glockshuber, R. Riek, M. Billeter, K. Wüthrich and B. Oesch, 1997, Prion (PrPSc)-specific epitope defined by amonoclonal antibody, Nature 390, 74-77;
- antibody W226 (recognizing amino acids 145-155), for example as described in Petsch, B., Muller-Schiffmann, A., Lehle, A., Zirdum, E., Prikulis, I., Kuhn, F., Raeber, A.J., Ironside, J.W., Korth, C., and Stitz, L. (2011). Biological effects and use of PrPSc- and PrP-specific antibodies generated by immunization with purified full-length native mouse prions. Journal of virology 85, 4538-4546;
- anti-PrP antibodies described in Didonna, A., Venturini, A.C., Hartman, K., Vranac, T., Curin Serbec, V., and Legname, G. (2015). Characterization of four new monoclonal antibodies against the distal N-terminal region of PrP(c). PeerJ 3, e811, such as antibody EB8 (recognizing amino acids 26-34);
- antibody 4H11, for example as described in Lefebvre-Roque M, Kremmer E, Gilch S, Zou WQ, Feraudet C, Gilles CM, et al. Toxic effects of intracerebral PrP antibody administration during the course of BSE infection in mice. Prion. 2007; 1(3):198-206;
- anti-PrP antibodies 106 and 110, for example as described in Song CH, Furuoka H, Kim CL, Ogino M, Suzuki A, Hasebe R, et al. Effect of intraventricular infusion of anti-prion protein monoclonal antibodies on disease progression in prion-infected mice. J Gen Virol. 2008; 89(Pt 6):1533-44;
- antibody 31C6, for example as described in Ohsawa N, Song CH, Suzuki A, Furuoka H, Hasebe R, Horiuchi M. Therapeutic effect of peripheral administration of an anti-prion protein antibody on mice infected with prions. Microbiol Immunol. 2013; 57(4):288-97;
- antibody 44B1, for example as described in Kim C-L, Karino A, Ishiguro N, Shinagawa M, Sato M, et al. (2004) Cell-surface retention of PrPC by anti-PrP antibody prevents protease-resistant PrP formation. The Journal of General Virology 85: 3473-3482;
- anti-PrP antibodies and antigen-binding fragments thereof described in Williamson RA, Peretz D, Pinilla C, et al. Mapping the Prion Protein Using Recombinant Antibodies. Journal of Virology. 1998;72(11):9413-9418, for example Fabs R1, R2, R5, R10, D2, D4, D5, D7, D13, D14, and D18;
- anti-PrP antibodies and antigen-binding fragments thereof disclosed in WO 2008/124098, for example 3F4, POM1, POM4, POM5, P0M6, P0M7, POM8, POM9, POM10, POM13, P0M15, P0M16, P0M17, POM 19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri308, Pri917, BAR215, BAR221, BAR224, BAR233, BAR234, Sha31, 11B9, 12F10, D18, 6H4, BDI115, POM2, POM11, P0M12, POM14, 3B5, 4F2, 13F10, SAF-15, SAF-31, SAF-32, SAF-33, SAF-34, SAF-35, and SAF-37;
- anti-PrP antibodies and antigen-binding fragments thereof described in Feraudet C. et al. (2005) The Journal of Biological Chemistry 280(12): 11247-11258, for example BAR210, BAR231, 3B5, 4F2, SAF-15, SAF-31, SAF-32, SAF-33, SAF-34, SAF-35, SAF-37, BAR238, 8G8, Pri308, BAR215, BAR221, BAR224, BAR233, BAR234, Sha31, 12F10, SAF-53, SAF-51, SAF-75. SAF-76, SAF-54, SAF-60, SAF-69, SAF-70, SAF-66, SAF-4, SAF-8, SAF-9, SAF-10, SAF-13, SAF-14, SAF-22, SAF-24, SAF-82, SAF-95, SAF-2, SAF-12, SAF-68, SAF-84, SAF-83, Pri917, 11C6, SAF-3, SAF-1, SAF-5, SAF-7, SAF-11, SAF-21, SAF-23, SAF-67, SAF-73, SAF-77, SAF-80, BAR201, BAR202, BAR203, BAR205, BAR206, BAR213, BAR216, BAR217, BAR219, BAR220, BAR229, BAR204, BAR222, BAR225, BAR232, BAR235, BAR239, BAR240, BAR207, BAR208, BAR209, BAR211, BAR214, BAR223, BAR226, BAR236, BAR241, βS4, βS12, βS14, βS23, βS39, βS42, βS8, βS16, βS18, βS29, βS31, βS36, βS37, βS39, βS41, βS43, βH1, βH2, βH3, Sha4, Sha5, Sha6, Sha7, Sha8, Sha9, Sha11, Sha13, Sha14, Sha15, Sha16, Sha17, Sha18, Sha19, Sha20, Sha22, Sha23, Sha24, Sha27, Sha28, Sha29, Sha30, Sha32, Sha33, Sha34, Sha25, Sha26, Sha39, Sha40, Sha42, Sha44, Sha45, Sha46, Sha36, Sha37, Sha38, Sha49, Sha50, Sha51, and Sha52; and
- antibody PRN100, the humanized form of ICSM18, for example as described in Klyubin I. et al., 2014, The Journal of Neuroscience 34(18):6140-6145.

Among the above examples of anti-PrP antibodies, the POM monoclonals are preferred. Accordingly, it is preferred that the ligand, in particular the antibody or an antigen-binding fragment thereof, is a POM monoclonal, for example as described in Polymenidou M. et al. (2008) "The POM monoclonals: a comprehensive set of antibodies to non-overlapping prion protein epitopes", PLoS one 3(12): e3872, Polymenidou et al. (2005) Lancet Neurol. 4:805-814, or an antigen-binding fragment or derivative thereof. Preferred examples of POM monoclonals include POM1, POM2, POM3, POM4, POM5, POM6, POM7, POM8, POM9, POM10, POM11, POM12, POM13, POM14, POM15, POM16, POM17, POM18, and POM19 and antigen-binding fragments and derivatives thereof.

Even more preferably, the ligand, in particular the anti-PrP antibody or an antigen-binding fragment or derivative thereof, is a humanized POM monoclonal or an antigen-binding fragment or derivative thereof.

For most of the known anti-PrP antibodies and antigen-binding fragments thereof, the epitopes of PrP, to which the antibodies bind to, are well-known. In view thereof, antibodies and antigen-binding fragments thereof binding to the N-terminal part of PrP and antibodies and antigen-binding fragments thereof binding to the C-terminal part of PrP can be easily identified.

Examples of antibodies and antigen-binding fragments thereof binding to the N-terminal part of PrP include, but are not limited to POM2, POM3, POM11, P0M12, POM14, 3B5, 4F2, 13F10, SAF-15, SAF-31, SAF-32, SAF-33, SAF-34, SAF-35, SAF-37, EB8, D13, 8B4, 6D11, BAR210, BAR231, 3B5, 4F2, SAF-15, SAF-31, SAF-32, SAF-33, SAF-34, SAF-35, SAF-37, BAR238, 8G8, D13, ICSM35, and Pri308. Preferred antibodies and antigen-binding fragments thereof binding to the N-terminal part of PrP include POM2, POM3, POM11, P0M12, and POM14, and antigen-binding fragments and derivatives thereof, in particular humanized forms thereof.

Examples of antibodies and antigen-binding fragments thereof binding to the N-terminal part of PrP include, but are not limited to 3F4, POM1, POM4, POM5, P0M6, P0M7, POM8, POM9, POM10, POM13, P0M15, P0M16, POM17, POM19, SAF-2, SAF-4, SAF-8, SAF-9, SAF-10, SAF-12, SAF-13, SAF-14, SAF-22, SAF-24, SAF-53, SAF-54, SAF-60, SAF-61, SAF-66, SAF-68, SAF-69, SAF-70, SAF-75, SAF-76, SAF-82, SAF-83, SAF-84, SAF-95, Pri917, BAR215, BAR221, BAR224, BAR233, BAR234, Sha31, 11B9, 12F10, D18, 6H4, BDI115, W226, SAF-53, SAF-51, SAF-75. SAF-76, SAF-54, SAF-60, SAF-69, SAF-70, SAF-66, SAF-4, SAF-8, SAF-9, SAF-10, SAF-13, SAF-14, SAF-22, SAF-24, SAF-82, SAF-95, SAF-2, SAF-12, SAF-68, SAF-84, SAF-83, 7H6, 7A12, 2C2, 8H4, 9H7, 8F9, ICSM15, ICSM17, ICSM18, ICSM30, ICSM31, ICSM32, and PRN100. Preferred antibodies and antigen-binding fragments thereof binding to the C-terminal part of PrP include POM1, POM4, POM5, P0M6, P0M7, POM8, POM9, POM10, POM13, P0M15, P0M16, P0M17, and POM19, and antigen-binding fragments and derivatives thereof, in particular humanized forms thereof.

For example, antibodies binding to the C-terminal part of PrP, in particular to helix 1, include ICSM17 (recognizing amino acids 131-150; available from D-Gen Ltd, UK), ICSM18 (recognizing amino acids 142-153; available from D-Gen Ltd, UK), ICSM 30 (recognizing amino acids 136-143; available from D-Gen Ltd, UK), ICSM 31 (recognizing amino acids 136-143; available from D-Gen Ltd, UK), ICSM 32 (recognizing amino acids 131 -150; available from D-Gen Ltd, UK), Sha31 (recognizing amino acids 145-152; Alier et al. 2011 J. Neurosci. 31, p. 16292-16297; available from Bertin Pharma (subsidiary of Bertin Technologies), France, as product number A03213), 6H4 (e.g. from Prionics AG, Wagistrasse 27a, CH-8952 Schlieren-Zurich, Switzerland), E12/2 (e.g. as published in Cernilec et al. 2007 Immunol Lett. 113(1): 29-39), and D18 (e.g. as published in Peretz et al. 2001 Nature 412: 739-743). A preferred example is ICSM-18. More preferably, such an antibody is a humanized, such as the humanized version of ICSM-18, namely PRN100.

Preferably, the ligand for use according to the present invention, in particular the anti-PrP antibody, is a multispecific antibody or antigen-binding fragment thereof, more preferably a bispecific antibody or antigen-binding fragment thereof.

As used herein, the term "multispecific" refers to the ability to bind to at least two different epitopes, e.g. on different antigens or on the same antigen. Preferably, the multispecific antibody according to the present invention is capable of binding to at least two distinct, preferably non-overlapping, epitopes of PrP, in particular of human PrP^{C}. Terms like "bispecific", trispecific", "tetraspecific" etc. refer to the number of different epitopes to which the antibody can bind to. For example, conventional monospecific IgG-type antibodies have two identical epitope binding sites (paratopes) and can, thus, only bind to identical epitopes (but not to different epitopes). A multispecific antibody, in contrast, has at least two distinct types of paratopes and can, thus, bind to at least two different epitopes. As used herein, "paratope" refers to an epitope-binding site of the antibody. Moreover, a single "specificity" may refer to one, two, three or more identical paratopes in a single antibody (the actual number of paratopes in one single antibody molecule is referred to as "valency"). For example, a single native IgG antibody is monospecific and bivalent, since it has two identical paratopes. Accordingly, a multispecific antibody comprises at least two (different) paratopes. Thus, the term "multispecific antibodies" refers to antibodies having more than one paratope and the ability to bind to two or more different epitopes. The term "multispecific antibodies" comprises in particular bispecific antibodies, but typically also protein, e.g. antibody, scaffolds, which bind in particular to three or more different epitopes, i.e. antibodies with three or more paratopes.

In particular, the multispecific antibody, or the antigen binding fragment thereof, may comprise two or more paratopes, wherein some paratopes may be identical so that all paratopes of the antibody belong to at least two different types of paratopes and, hence, the antibody has at least two specificities. For example, the multispecific antibody or antigen binding fragment thereof according to the present invention may comprise four paratopes, wherein each two paratopes are identical (i.e. have the same specificity) and, thus, the antibody or fragment thereof is bispecific and tetravalent (two identical paratopes for each of the two specificities). Thus, "one specificity" refers in particular to one or more paratopes exhibiting the same specificity (which typically means that such one or more paratopes are identical) and, thus, "two specificities" may be realized by two, three, four five, six or more paratopes as long as they refer to only two specificities. Most preferably a multispecific antibody comprises one single paratope for each (of the at least two) specificity, i.e. the multispecific antibody comprises in total at least two paratopes. For example, a bispecific antibody comprises one single paratope for each of the two specificities, i.e. the antibody comprises in total two paratopes. It is also preferred that the antibody comprises two (identical) paratopes for each of the two specificities, i.e. the antibody comprises in total four paratopes. Preferably the antibody comprises three (identical) paratopes for each of the two specificities, i.e. the antibody comprises in total six paratopes.

Preferably, the antibody, or the antigen binding fragment thereof, for use according to the present invention is a bispecific antibody or a bispecific antigen binding fragment thereof.

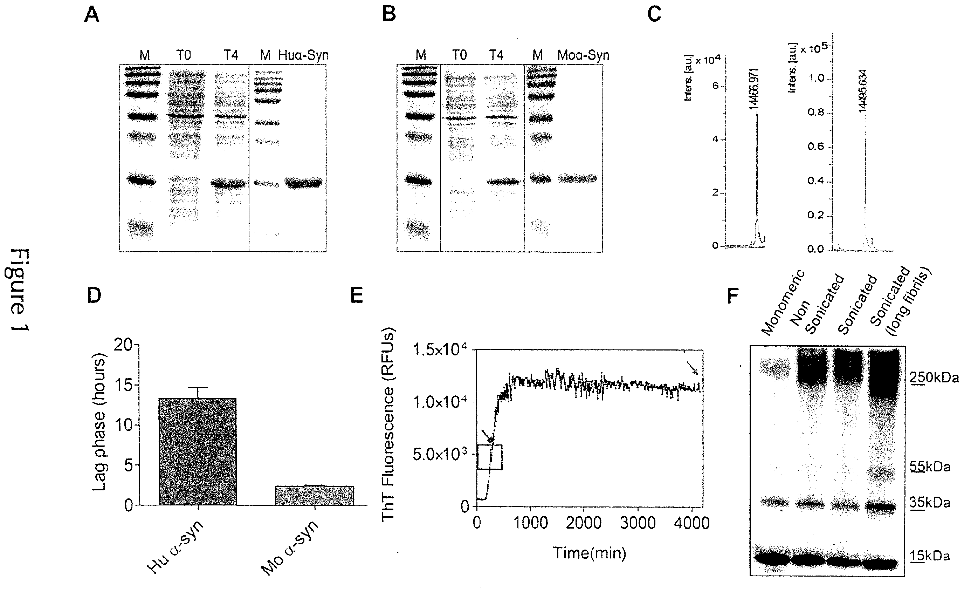

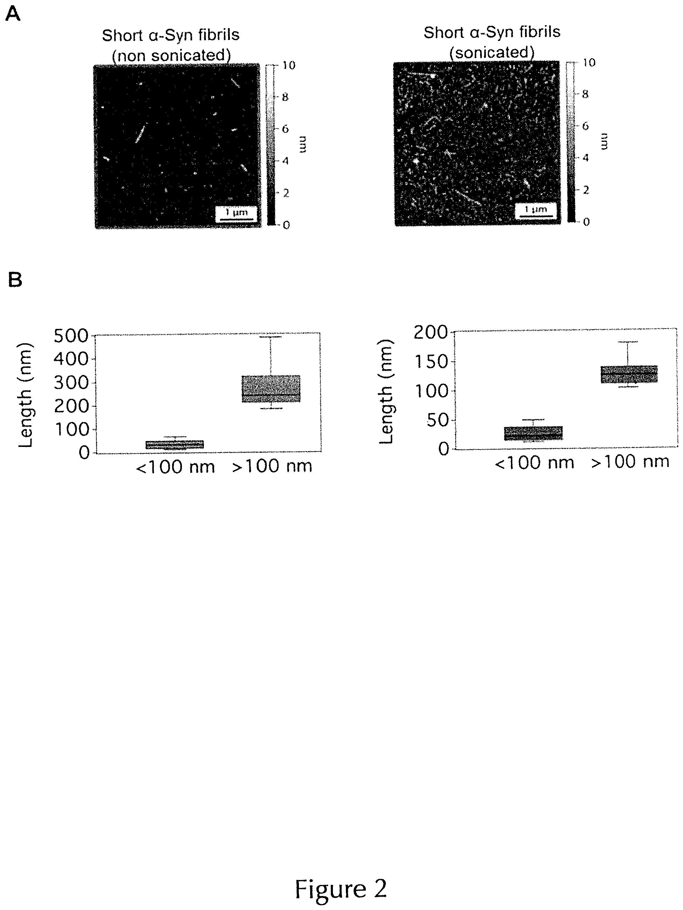

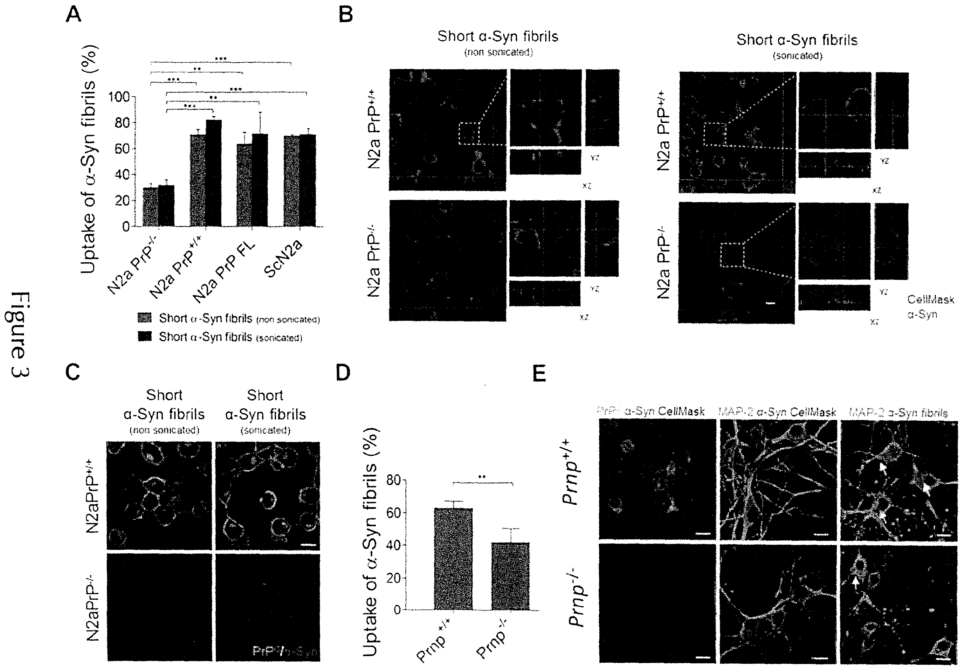

The antibody, or the antigen binding fragment thereof, for use according to the present invention may be of any antibody format. In particular, multispecific antibodies preferably encompass "whole" antibodies, such as whole IgG- or IgG-like molecules, while antigen binding fragments in the context of the present invention preferably refer to small recombinant formats, such as bispecific T-cell engagers (BiTes), tandem single chain variable fragment molecules (taFvs), diabodies (Dbs), single chain diabodies (scDbs) and various other derivatives of these (cf. bispecific antibody formats as described by Byrne H. et al. (2013) Trends Biotech, 31 (11): 621-632 with Figure 2 showing various bispecific antibody formats; Weidle U.H. et al. (2013) Cancer Genomics and Proteomics 10: 1-18, in particular Fig. 1 showing various bispecific antibody formats; and Chan, A.C. and Carter, P.J. (2010) Nat Rev Immu 10: 301-316 with Fig. 3 showing various bispecific antibody formats). Examples of bispecific antibody formats include, but are not limited to, quadroma, chemically coupled Fab (fragment antigen binding), and BiTE® (bispecific T cell engager). In one embodiment of the present invention the antibody used is preferably a BiTE® (bispecific T cell engager).

Thus, the antibody, or the antigen binding fragment thereof, for use according to the present invention may be selected from the group comprising hybrid hybridoma (quadroma); Multispecific anticalin platform (Pieris); Diabodies; Single chain diabodies; Tandem single chain Fv fragments; TandAbs, Trispecific Abs (Affimed) (105-110 kDa); Darts (dual affinity retargeting; Macrogenics); Bispecific Xmabs (Xencor); Bispecific T cell engagers (Bites; Amgen; 55kDa); Triplebodies; Tribody = Fab-scFv Fusion Protein (CreativeBiolabs) multifunctional recombinant antibody derivates (110 kDa); Duobody platform (Genmab); Dock and lock platform; Knob into hole (KIH) platform; Humanized bispecific IgG antibody (REGN1979) (Regeneron); Mab² bispecific antibodies (F-Star); DVD-Ig = dual variable domain immunoglobulin (Abbvie); kappa-lambda bodies; TBTI = tetravalent bispecific tandem Ig; and CrossMab.

The antibody, or the antigen binding fragment thereof, for use according to the present invention may be selected from bispecific IgG-like antibodies (BsIgG) comprising CrossMab; DAF (two-in-one); DAF (four-in-one); DutaMab; DT-IgG; Knobs-in-holes common LC; Knobs-in-holes assembly; Charge pair; Fab-arm exchange; SEEDbody; Triomab; LUZ-Y; Fcab; κλ-body; and Orthogonal Fab. These bispecific antibody formats are shown and described for example in Spiess C., Zhai Q. and Carter P.J. (2015) Molecular Immunology 67: 95-106, in particular Fig. 1 and corresponding description, e.g. p. 95-101.

Preferably, the antibody, or the antigen binding fragment thereof, for use according to the present invention may be selected from IgG-appended antibodies with an additional antigen-binding moiety comprising DVD-IgG; IgG(H)-scFv; scFv-(H)IgG; IgG(L)-scFv; scFV-(L)IgG; IgG(L,H)-Fv; IgG(H)-V; V(H)-IgG; IgG(L)-V; V(L)-IgG; KIH IgG-scFab; 2scFv-IgG; IgG-2scFv; scFv4-Ig; scFv4-Ig; Zybody; and DVI-IgG (four-in-one). These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

Preferably, the antibody, or the antigen binding fragment thereof, for use according to the present invention may be selected from bispecific antibody fragments comprising Nanobody; Nanobody-HAS; BiTE; Diabody; DART; TandAb; scDiabody; sc-Diabody-CH3; Diabody-CH3; Triple Body; Miniantibody; Minibody; TriBi minibody; scFv-CH3 KIH; Fab-scFv; scFv-CH-CL-scFv; F(ab')2; F(ab')2-scFv2; scFv-KIH; Fab-scFv-Fc; Tetravalent HCAb; scDiabody-Fc; Diabody-Fc; Tandem scFv-Fc; and Intrabody. These bispecific antibody formats are shown and described for example in Spiess C., Zhai Q. and Carter P.J. (2015) Molecular Immunology 67: 95-106, in particular Fig. 1 and corresponding description, e.g. p. 95-101.

Preferably, the antibody, or the antigen binding fragment thereof, for use according to the present invention does not comprise a binding site for an Fc receptor, in particular the antibody, or the antigen binding fragment thereof, does not comprise an Fc moiety such as an Fc region.

Preferably, the antibody, or the antigen binding fragment thereof, for use according to the present invention may be selected from bispecific fusion proteins comprising Dock and Lock; ImmTAC; HSAbody; scDiabody-HAS; and Tandem scFv-Toxin. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

In particular, the antibody, or the antigen binding fragment thereof, for use according to the present invention may be selected from bispecific antibody conjugates comprising IgG-IgG; Cov-X-Body; and scFv1-PEG-scFv2. These bispecific antibody formats are shown and described for example in Spiess C., Zhai Q. and Carter P.J. (2015) Molecular Immunology 67: 95-106, in particular Fig. 1 and corresponding description, e.g. p. 95 - 101.

Preferably, the antibody, or the antigen binding fragment thereof, for use according to the present invention comprises a binding site for an Fc receptor. More preferably, the antibody, or the antigen binding fragment thereof, for use according to the present invention comprises an Fc moiety, in particular an Fc region.

As used herein, the term "Fc moiety" refers to a sequence derived from the portion of an immunoglobulin heavy chain beginning in the hinge region just upstream of the papain cleavage site and ending at the C-terminus of the immunoglobulin heavy chain. Preferably, the "Fc moiety" comprises a binding site for an Fc receptor. However, it is also preferred that an Fc moiety may mediate a functionality different from binding to an Fc receptor, for example binding to a protein of the complement system. Accordingly, an "Fc moiety" may be a complete Fc region or a part (e.g., a domain) thereof. Preferably, the "Fc moiety" mediates the full functionality of a complete Fc region, e.g. including Fc receptor binding and, optionally, binding to a protein from the complement system. Thus, the antibody as used according to the present invention may preferably comprise a complete Fc region, whereby a complete Fc region comprises at least a hinge domain, a CH2 domain, and a CH3 domain. The Fc moiety may also comprise one or more amino acid insertions, deletions, or substitutions relative to a naturally-occurring Fc region. For example, at least one of a hinge domain, CH2 domain or CH3 domain (or portion thereof) may be deleted. For example, an Fc moiety may comprise or consist of: (i) hinge domain (or portion thereof) fused to a CH2 domain (or portion thereof), (ii) a hinge domain (or portion thereof) fused to a CH3 domain (or portion thereof), (iii) a CH2 domain (or portion thereof) fused to a CH3 domain (or portion thereof), (iv) a hinge domain (or portion thereof), (v) a CH2 domain (or portion thereof), or (vi) a CH3 domain or portion thereof.

In the context of the present invention, bispecific antibodies (BiAbs) comprise (exactly) two specificities. They are the most preferred type of multispecific antibodies and antigen binding fragments thereof. A bispecific antibody in the context of the present invention may be of any bispecifc antibody format. For example, BiAbs may be whole antibodies, such as whole IgG-like molecules, or fragments thereof which are not whole antibodies but retain antibody properties. These may be small recombinant formats, e.g. as tandem single chain variable fragment molecules (taFvs), diabodies (Dbs), single chain diabodies (scDbs), and various other derivatives of these (cf. e.g. Byrne H. et al. (2013) Trends Biotech, 31 (11): 621-632 with Figure 2 showing various bispecific antibody formats).

Preferably, the multispecific, in particular bispecific, antibody, or the antigen binding fragment thereof is at least bivalent, i.e. it has at least two paratopes. More preferably, the multispecific, in particular bispecific, antibody, or the antigen binding fragment thereof is bivalent, trivalent, tetravalent, or hexavalent. Even more preferably, the multispecific, in particular bispecific, antibody, or the antigen binding fragment thereof is bivalent or tetravalent. Most preferably, the antibody, or the antigen binding fragment thereof, for use according to the present invention is a bispecific, bivalent antibody, i.e. an antibody having exactly two distinct paratopes.

Preferably at least one specificity, in particular at least one paratope, of the multispecific, in particular bispecific, antibody (or an antigen-binding fragment thereof) is capable of binding to (an epitope in) the N-terminal part of PrP, in particular human PrP^{C}. It is also preferred that at least one specificity, in particular at least one paratope, of the multispecific, in particular bispecific, antibody (or an antigen-binding fragment thereof) is capable of binding to (an epitope in) the C-terminal part of PrP, in particular human PrP^{C}. It is also preferred that the multispecific, in particular bispecific, antibody (or an antigen-binding fragment thereof) is capable of binding to two distinct, preferably non-overlapping epitopes of PrP, in particular human PrP^{C}. Most preferably, the multispecific, in particular bispecific antibody (or an antigen-binding fragment thereof) comprises (i) at least one specificity, in particular at least one paratope, capable of binding to (an epitope in) the N-terminal part of PrP, in particular human PrP^{C} and (ii) at least one specificity, in particular at least one paratope, capable of binding to (an epitope in) the C-terminal part of PrP, in particular human PrP^{C}. Accordingly, it is preferred that the ligand for use according to the present invention, in particular the anti-PrP antibody or the antigen-binding fragment thereof, may be a multispecific antibody, in particular a bispecific antibody, or an antigen-binding fragment thereof comprising
(a) a specificity (at least one paratope) against an epitope in the N-terminal part of PrP, in particular of human PrP^{C}; and
(b) a specificity (at least one paratope) against an epitope in the C-terminal part of PrP, in particular of human PrP^{C}.

It is also preferred that the ligand for use according to the present invention, in particular the anti-PrP antibody or the antigen-binding fragment thereof, may be a multispecific antibody or an antigen-binding fragment thereof comprising
(a) at least one specificity (at least one paratope) against at least one epitope in PrP, in particular in human PrP^{C}; and
(b) a specificity of an antibody capable of crossing the blood-brain barrier, in particular of an antibody capable of undergoing receptor-mediated transcytosis (RMT).

Such antibodies provide an increased uptake in the CNS due to facilitated passage across the blood-brain barrier. Antibodies capable of crossing the blood-brain barrier, in particular antibodies capable of undergoing receptor-mediated transcytosis (RMT) are known in the art. Preferred examples of such antibodies include anti-transferrin receptor (TfR) antibodies, such as OX-26 (for example, as described in Friden PM, Walus LR, Musso GF, Taylor MA, Malfroy B, Starzyk RM. Anti-transferrin receptor antibody and antibody drug conjugates cross the blood-brain barrier. Proc Natl Acad Sci U S A 1991;88:4771-5); anti-insulin receptor (InsR) antibodies, such as 83-14 (for example as described in Pardridge WM, Kang YS, Buciak JL, Yang J. Human insulin receptor monoclonal antibody undergoes high affinity binding to human brain capillaries in vitro and rapid transcytosis through the blood-brain barrier in vivo in the primate. Pharm Res 1995;12: 807-16; Boado RJ, Zhang Y, Zhang Y, PardridgeWM. Humanization of antihuman insulin receptor antibody for drug targeting across the human blood-brain barrier. Biotechnol Bioeng 2007; 96:381-91); anti-low-density lipoprotein receptor-related protein 1 (Lrp1) antibodies; anti-low-density lipoprotein receptor-related protein 2 (Lrp2) antibodies; and single domain (sd) antibodies FC5 and FC44 (for example, as described in Muruganandam A, Tanha J, Narang S, Stanimirovic D. Selection of phage-displayed llama single-domain antibodies that transmigrate across human blood-brain barrier endothelium. FASEB J Off Publ Fed Am Soc Exp Biol 2002; 16: 240-2; Abulrob A, Sprong H, Van Bergen en Henegouwen P, Stanimirovic D. The blood-brain barrier transmigrating single domain antibody: mechanisms of transport and antigenic epitopes in human brain endothelial cells. J Neurochem 2005;95:1201-14).

Multispecific antibodies comprising a specificity of an antibody capable of crossing the blood-brain barrier, in particular of an antibody capable of undergoing receptor-mediated transcytosis (RMT) may be engineered, for example, as described in: Stanimirovic D. et al (2014) Engineering and pharmacology of blood-brain barrier-permeable bispecific antibodies. Advances in Pharmacology 71:301-35, or in: Farrington G. K. et al. (2014)A novel platform for engineering blood-brain barrier-crossing bispecific biologics. FASEB J. 28(11):4764-78).

Preferably, the ligand for use according to the present invention, in particular the anti-PrP antibody or the antigen-binding fragment thereof, is a monoclonal antibody or antigen-binding fragment thereof.

It is also preferred that the ligand for use according to the present invention, in particular the anti-PrP antibody or the antigen-binding fragment thereof, is a human or humanized antibody or antigen-binding fragment thereof.

### Conjugate comprising ligands, in particular anti-PrP antibodies

In a further aspect, the present invention also provides a conjugate comprising the ligand as described above and an agent facilitating the passage across the blood-brain barrier for use in the prevention and/or treatment of a synucleinopathy.

Preferred agents facilitating the passage across the blood-brain barrier include in particular agents undergoing adsorptive mediated transport (AMT) and agents undergoing receptor-mediated transcytosis (RMT). Due to their higher specificity, agents undergoing receptor-mediated transcytosis (RMT) are more preferred.

Preferred examples of agents undergoing adsorptive mediated transport (AMT) include sugar molecules (for example for glycation), diamines and polyamines (for example for polyamination), and cell penetrating peptides. Examples of diamines and polyamines include hexamethylenediamine, putrescine, spermidine and spermine. Examples of cell penetrating peptides include penetratin (derived from Antennapedia protein), TAT protein (HIV-1 transactivating transcriptor), FBP (fusion sequence-based peptide), syn-B (derived from a natural mammalian antimicrobial peptide) and poly-arginine peptides.

Preferred examples of agents undergoing receptor-mediated transcytosis (RMT) include antibodies undergoing RMT. Preferred examples of such antibodies include anti-transferrin receptor (TfR) antibodies, such as OX-26 (for example, as described in Friden PM, Walus LR, Musso GF, Taylor MA, Malfroy B, Starzyk RM. Anti-transferrin receptor antibody and antibody drug conjugates cross the blood-brain barrier. Proc Natl Acad Sci U S A 1991;88:4771-5); anti-insulin receptor (InsR) antibodies, such as 83-14 (for example as described in Pardridge WM, Kang YS, Buciak JL, Yang J. Human insulin receptor monoclonal antibody undergoes high affinity binding to human brain capillaries in vitro and rapid transcytosis through the blood-brain barrier in vivo in the primate. Pharm Res 1995;12: 807-16; Boado RJ, Zhang Y, Zhang Y, PardridgeWM. Humanization of antihuman insulin receptor antibody for drug targeting across the human blood-brain barrier. Biotechnol Bioeng 2007; 96:381-91); anti-low-density lipoprotein receptor-related protein 1 (Lrp1) antibodies; anti-low-density lipoprotein receptor-related protein 2 (Lrp2) antibodies; and single domain (sd) antibodies FC5 and FC44 (for example, as described in Muruganandam A, Tanha J, Narang S, Stanimirovic D. Selection of phage-displayed llama single-domain antibodies that transmigrate across human blood-brain barrier endothelium. FASEB J Off Publ Fed Am Soc Exp Biol 2002;16: 240-2; Abulrob A, Sprong H, Van Bergen en Henegouwen P, Stanimirovic D. The blood-brain barrier transmigrating single domain antibody: mechanisms of transport and antigenic epitopes in human brain endothelial cells. J Neurochem 2005;95:1201-14).

Further preferred examples of agents undergoing receptor-mediated transcytosis (RMT) include transferrin, CRM197 (a non-toxic mutant of diphtheria toxin), and agents targeting low-density lipoprotein receptor related proteins, such as melanotransferrin, receptor-associated protein, p97 (for example as described in Karkan D, Pfeifer C, Vitalis TZ, Arthur G, Ujiie M, Chen Q, et al. A unique carrier for delivery of therapeutic compounds beyond the blood-brain barrier. PLoS One 2008;3:e2469), LRP binding domain of the apolipoprotein B and angiopep-2 (AN-2) (for review see Yu Y. J. and Watts R. J. (2013) Developing therapeutic antibodies for neurodegenerative disease Neurotherapeutics 10:459-472).

For example, the agent facilitating the passage across the blood-brain barrier may be angiopep-2 (AN-2). AN-2 is a 19-mer peptide associating with the LRP1 receptor on blood-brain barrier capillary endothelial cells and enters the brain via RMT. In particular, AN-2 has an amino acid sequence according to SEQ ID NO: 4:

### TFFYGGSRGKRNNFKTEEY

### (SEQ ID NO: 4)

Conjugation of the ligand and the agent facilitating the passage across the blood-brain barrier is not particularly limited and may be achieved by any appropriate method known to the skilled person. Preferably conjugation is achieved directly or via one or more linker agents. Conjugation may be obtained by covalent linkage.

A "covalent linkage" (also covalent bond), as used in the context of the present invention, refers to a chemical bond that involves the sharing of electron pairs between atoms. A "covalent linkage" (also covalent bond) in particular involves a stable balance of attractive and repulsive forces between atoms when they share electrons. For many molecules, the sharing of electrons allows each atom to attain the equivalent of a full outer shell, corresponding to a stable electronic configuration. Covalent bonding includes many kinds of interactions, including for example σ-bonding, π-bonding, metal-to-metal bonding, agostic interactions, and three-center two-electron bonds.

Conjugating may be accomplished via a coupling or conjugating agent including standard peptide synthesis coupling reagents such as HOBt, HBTU, DICI, TBTU. There are several intermolecular cross-linking agents which can be utilized, see for example, Means and Feeney, Chemical Modification of Proteins, Holden-Day, 1974, pp. 39-43. Among these reagents are, for example, N-succinimidyl 3-(2-pyridyldithio)propionate (SPDP) or N,N'-(1,3-phenylene)bismaleimide; N,N'-ethylene-bis-(iodoacetamide) or other such reagent having 6 to 11 carbon methylene bridges; and 1,5-difluoro-2,4-dinitrobenzene. Other cross-linking agents useful for this purpose include: p,p'-difluoro-m,m'-dinitrodiphenylsulfone; dimethyl adipimidate; phenol-1,4-disulfonylchloride; hexamethylenediisocyanate or diisothiocyanate, or azophenyl-p-diisocyanate; glutaraldehyde and disdiazobenzidine. Cross-linking agents may be homobifunctional, i.e., having two functional groups that undergo the same reaction. A preferred homobifunctional cross-linking agent is bismaleimidohexane (BMH). BMH contains two maleimide functional groups, which react specifically with sulfhydryl-containing compounds under mild conditions (pH 6.5-7.7). The two maleimide groups are connected by a hydrocarbon chain. Therefore, BMH is useful for irreversible cross-linking of proteins (or polypeptides) that contain cysteine residues. Cross-linking agents may also be heterobifunctional. Heterobifunctional cross-linking agents have two different functional groups, for example an amine-reactive group and a thiol-reactive group, that will cross-link two proteins having free amines and thiols, respectively. Examples of heterobifunctional cross-linking agents are Succinimidyl-4-(N-maleimidomethyl)-cyclohexane-1-carboxylate (SMCC), m-maleimidobenzoyl-N-hydroxysuccinimide ester (MBS), and succinimide 4-(p-maleimidophenyl)butyrate (SMPB), an extended chain analog of MBS. The succinimidyl group of these cross-linkers reacts with a primary amine, and the thiol-reactive maleimide forms a covalent bond with the thiol of a cysteine residue. Because cross-linking agents often have low solubility in water, a hydrophilic moiety, such as a sulfonate group, may be added to the cross-linking agent to improve its water solubility. Sulfo-MBS and sulfo-SMCC are examples of cross-linking agents modified for water solubility. Many cross-linking agents yield a conjugate that is essentially non-cleavable under cellular conditions. Therefore, some cross-linking agents contain a covalent bond, such as a disulfide, that is cleavable under cellular conditions. For example, Traut's reagent, dithiobis (succinimidylpropionate) (DSP), and N-succinimidyl 3-(2-pyridyldithio)propionate (SPDP) are well-known cleavable cross-linkers. The use of a cleavable cross-linking agent permits the ligand and the agent facilitating the passage across the blood-brain barrier to separate from each other after delivery into the CNS. For this purpose, direct disulfide linkage may also be useful. Chemical cross-linking may also include the use of spacer arms. Spacer arms provide intramolecular flexibility or adjust intramolecular distances between conjugated moieties and thereby may help preserve biological activity. A spacer arm may be in the form of a protein (or polypeptide) moiety that includes spacer amino acids, e.g. proline. Alternatively, a spacer arm may be part of the cross-linking agent, such as in "long-chain SPDP" (Pierce Chem. Co., Rockford, III., cat. No. 21651 H). Numerous cross-linking agents, including the ones discussed above, are commercially available. Detailed instructions for their use are readily available from the commercial suppliers. More detailed information on protein cross-linking and conjugate preparation, can be retrieved from: Wong, Chemistry of Protein Conjugation and Cross-Linking, CRC Press (1991).

Cross-linking agents for peptide or protein crosslinking include for example (i) amine-to-amine crosslinkers, e.g. homobifunctional amine-specific protein crosslinking reagents based on NHS-ester and imidoester reactive groups for selective conjugation of primary amines; available in short, long, cleavable, irreversible, membrane permeable, and cell surface varieties; (ii) sulfhydryl-to-carbohydrate crosslinkers, e.g. crosslinking reagents based on maleimide and hydrazide reactive groups for conjugation and formation of covalent crosslinks; (iii) sulfhydryl-to-sulfhydryl crosslinkers, e.g. homobifunctional sulfhydryl-specific crosslinking reagents based on maleimide or pyridyldithiol reactive groups for selective covalent conjugation of protein and peptide thiols (reduced cysteines) to form stable thioether bonds; (iv) photoreactive crosslinkers, e.g. aryl azide, diazirine, and other photo-reactive (light-activated) chemical heterobifunctional crosslinking reagents to conjugate proteins, nucleic acids and other molecular structures involved in receptor-ligand interaction complexes via two-step activation; (v) amine-to-sulfhydryl crosslinkers, e.g. heterobifunctional protein crosslinking reagents for conjugation between primary amine (lysine) and sulfhydryl (cysteine) groups of proteins and other molecules; available with different lengths and types of spacer arms; and (vi) amine-to-amine crosslinkers, e.g. carboxyl-to-amine crosslinkers, e.g. Carbodiimide crosslinking reagents, DCC and EDC (EDAC), for conjugating carboxyl groups (glutamate, aspartate, C-termini) to primary amines (lysine, N-termini) and also N-hydroxysuccinimide (NHS) for stable activation of carboxylates for amine-conjugation.

An example for conjugating an agent facilitating passage across the blood-brain barrier and an antibody is described in Regina A. et al. (2015) ANG4043, a Novel Brain-Penetrant Peptide-mAb Conjugate, Is Efficacious against HER2-Positive Intracranial Tumors in Mice. Mol Cancer Ther 14(1): 129-140. Accordingly, for example the anti-PrP antibody or the antigen-binding fragment thereof may be conjugated, for example to AN-2 as described by Regina et al., in particular as shown in Fig. 1A of Regina et al.. It is thus preferred to use copper-free click chemistry and a two-step procedure. In such a two-step procedure in the first step MFCO-N-hydroxysuccinimide ester may be coupled to the anti-PrP antibody or the antigen-binding fragment thereof and in the second step the modified anti-PrP antibody or the antigen-binding fragment thereof may be coupled, for example, to AN-2-N₃.

### Nucleic acids encoding ligands, in particular anti-PrP antibodies

In second aspect, the present invention provides a nucleic acid molecule comprising a polynucleotide encoding the ligand as defined above for use in the prevention and/or treatment of a synucleinopathy, wherein the ligand is a (poly)peptide, in particular an anti-prion protein antibody or an antigen-binding fragment thereof.

Examples of nucleic acid molecules and/or polynucleotides include, e.g., a recombinant polynucleotide, a vector, an oligonucleotide, an RNA molecule such as an rRNA, an mRNA, an miRNA, an siRNA, or a tRNA, or a DNA molecule such as a cDNA. Nucleic acid sequences encoding an anti-PrP antibody or an antigen-binding fragment thereof, as described above, are preferred. Nucleic acid molecules encoding part or all of the light and heavy chains and CDRs of the anti-PrP antibodies are also preferred. Preferably provided herein are thus nucleic acid sequences encoding part or all of the light and heavy chains, in particular VH and VL sequences and CDRs of anti-PrP antibodies as described above.

A nucleic acid molecule is a molecule comprising, preferably consisting of nucleic acid components. The term nucleic acid molecule preferably refers to DNA or RNA molecules. In particular, it is used synonymous with the term "polynucleotide". Preferably, a nucleic acid molecule is a polymer comprising or consisting of nucleotide monomers which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

In general, the nucleic acid molecule may be manipulated to insert, delete or alter certain nucleic acid sequences. Changes from such manipulation include, but are not limited to, changes to introduce restriction sites, to amend codon usage, to add or optimize transcription and/or translation regulatory sequences, etc. It is also possible to change the nucleic acid to alter the encoded amino acids. For example, it may be useful to introduce one or more (*e.g.,* 1, 2, 3, 4, 5, 6, 7, 8, 9, 10, etc.) amino acid substitutions, deletions and/or insertions into the antibody's amino acid sequence. Such point mutations can modify effector functions, antigen-binding affinity, post-translational modifications, immunogenicity, etc., can introduce amino acids for the attachment of covalent groups (e.g., labels) or can introduce tags (e.g., for purification purposes). Mutations can be introduced in specific sites or can be introduced at random, followed by selection (e.g., molecular evolution). For instance, one or more nucleic acids encoding any of the CDR regions, a VH sequence and/or a VL sequence of an anti-PrP antibody can be randomly or directionally mutated to introduce different properties in the encoded amino acids. Such changes can be the result of an iterative process wherein initial changes are retained and new changes at other nucleotide positions are introduced. Further, changes achieved in independent steps may be combined. Different properties introduced into the encoded amino acids may include, but are not limited to, enhanced affinity.

Preferably, the nucleic acid molecule is a vector, for example, an expression vector. The term "vector" refers to a nucleic acid molecule, preferably to a recombinant nucleic acid molecule, i.e. a nucleic acid molecule which does not occur in nature. A vector in the context of the present invention may be suitable for incorporating or harboring a desired nucleic acid sequence. Such vectors may be storage vectors, expression vectors, cloning vectors, transfer vectors etc. Thus, the vector may comprise a sequence corresponding, e.g., to a desired antibody or antibody fragment thereof. An expression vector may be used for production of expression products such as RNA, e.g. mRNA, or peptides, polypeptides or proteins. For example, an expression vector may comprise sequences needed for transcription of a sequence stretch of the vector, such as a promoter sequence. A vector in the context of the present invention may be, e.g., an RNA vector or a DNA vector.

### Pharmaceutical compositions

In a third aspect the present invention provides a pharmaceutical composition comprising
(i) the ligand as described above, in particular the anti-PrP antibody or an antigen-binding fragment thereof, the conjugate as described above, or the nucleic acid molecule as described above, and
(ii) optionally, a pharmaceutically acceptable carrier, diluent and/or excipient
for use in the prevention and/or treatment of a synucleinopathy.

In other words, the present invention also provides a pharmaceutical composition comprising the ligand as described above, in particular the anti-PrP antibody or an antigen-binding fragment thereof, the conjugate as described above, or the nucleic acid molecule as described above for use in the prevention and/or treatment of a synucleinopathy.

The pharmaceutical composition may preferably also contain a pharmaceutically acceptable carrier, diluent and/or excipient. Although the carrier or excipient may facilitate administration, it should not itself induce the production of antibodies harmful to the individual receiving the composition. Nor should it be toxic. Suitable carriers may be large, slowly metabolized macromolecules such as proteins, polypeptides, liposomes, polysaccharides, polylactic acids, polyglycolic acids, polymeric amino acids, amino acid copolymers and inactive virus particles. In general, pharmaceutically acceptable carriers in a pharmaceutical composition according to the present invention may be active components or inactive components. Preferably, the pharmaceutically acceptable carrier in a pharmaceutical composition according to the present invention is not an active component in respect to a synucleinopathy.

The pharmaceutical composition may contain a pharmaceutically acceptable carrier and/or excipient, that facilitates processing of the active compounds into preparations designed for delivery to the site of action. For example, the pharmaceutical composition may comprise one or more agents capable of promoting penetration of a ligand across the blood-brain barrier.

Preferably, the pharmaceutical composition may be formulated as aqueous solution of the active compound in water-soluble form, for example, water-soluble salts. Pharmaceutically acceptable salts include mineral acid salts, such as hydrochlorides, hydrobromides, phosphates and sulphates, or salts of organic acids, such as acetates, propionates, malonates and benzoates. Aqueous injection suspensions may contain substances that increase the viscosity of the suspension include, for example, sodium carboxymethyl cellulose, sorbitol and dextran. In addition, the pharmaceutical composition may be formulated as oily injection suspension. Suitable lipophilic solvents or vehicles include fatty oils, for example, sesame oil, or synthetic fatty acid esters, for example, ethyl oleate or triglycerides. Optionally, the suspension may also contain stabilizers. Liposomes also can be used to encapsulate the ligands for delivery into cells or interstitial spaces.

Examples of pharmaceutically acceptable carriers, diluents and/or excipients include, but are not limited to physiologically compatible solvents, dispersion media, coatings, antibacterial and antifungal agents, isotonic and absorption delaying agents, water, saline, phosphate buffered saline, dextrose, glycerol, ethanol, isotonic agents, for example, sugars, polyalcohols such as mannitol, sorbitol, or sodium chloride, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and/or wool fat.

Pharmaceutically acceptable carriers in a pharmaceutical composition may additionally contain liquids such as water, saline, glycerol and ethanol. Additionally, auxiliary substances, such as wetting or emulsifying agents or pH buffering substances, may be present in such compositions. Such carriers enable the pharmaceutical compositions to be formulated as tablets, pills, dragees, capsules, liquids, gels, syrups, slurries and suspensions, for ingestion by the subject.

Preferably, the pharmaceutical composition comprises a vehicle. A vehicle is typically understood to be a material that is suitable for storing, transporting, and/or administering a compound, such as a pharmaceutically active compound, in particular the antibodies according to the present invention. For example, the vehicle may be a physiologically acceptable liquid, which is suitable for storing, transporting, and/or administering a pharmaceutically active compound, in particular the antibodies according to the present invention. Once formulated, the compositions of the invention may be administered directly to the subject. In one embodiment the compositions are adapted for administration to mammalian, e.g., human subjects.

Pharmaceutical compositions may include an antimicrobial, particularly if packaged in a multiple dose format. They may comprise detergent e.g., a Tween (polysorbate), such as Tween 80. Detergents are generally present at low levels e.g., less than 0.01%. Compositions may also include sodium salts (e.g., sodium chloride) to give tonicity. For example, a concentration of 10±2mg/ml NaCl is typical.

Further, pharmaceutical compositions may comprise a sugar alcohol (e.g., mannitol) or a disaccharide (e.g., sucrose or trehalose) e.g., at around 15-30 mg/ml (e.g., 25 mg/ml), particularly if they are to be lyophilized or if they include material which has been reconstituted from lyophilized material. The pH of a composition for lyophilization may be adjusted to between 5 and 8, or between 5.5 and 7, or around 6.1 prior to lyophilization.

In general, pharmaceutical compositions of the invention have preferably a pH between 5 and 8.5, in some embodiments this may be between 6 and 8, and in other embodiments about 7. The pH may be maintained by the use of a buffer. The composition may be sterile and/or pyrogen free. The composition may be isotonic with respect to humans. In one embodiment pharmaceutical compositions of the invention are supplied in hermetically-sealed containers.

Pharmaceutical compositions of the invention may be prepared in various forms, for example depending on the route of administration. Within the scope of the invention are compositions present in several forms of administration; including but not limited to, oral, intravenous, intramuscular, intra-arterial, intramedullary, intraperitoneal, intrathecal, intraventricular, transdermal, transcutaneous, topical, subcutaneous, intranasal, enteral, sublingual, or intra-CSF routes. Hyposprays may also be used to administer the pharmaceutical compositions of the invention.

It is preferred that the active ingredient in the composition is an anti-PrP antibody, an antigen-binding fragment thereof, or variants and derivatives thereof, in particular the active ingredient in the composition is preferably an anti-PrP antibody, an antigen-binding fragment thereof, or variants and derivatives thereof. As such, it may be susceptible to degradation in the gastrointestinal tract. Thus, if the composition is to be administered by a route using the gastrointestinal tract, the composition may contain agents which protect the antibody from degradation but which release the antibody once it has been absorbed from the gastrointestinal tract.

The composition can be formulated as a solution, microemulsion, dispersion, liposome, or other ordered structure suitable to high drug concentration. For example, the compositions may be prepared as injectables, either as liquid solutions or suspensions.

Administration forms suitable for parenteral administration, e.g., by injection or infusion, include for example by bolus injection or continuous infusion. Where the product is for injection or infusion, it may take the form of a suspension, solution or emulsion in an oily or aqueous vehicle and it may contain formulatory agents, such as suspending, preservative, stabilizing and/or dispersing agents. Alternatively, the antibody molecule may be in dry form, for reconstitution before use with an appropriate sterile liquid, for example as described above.

For injection, e.g. intravenous, cutaneous or subcutaneous injection, or injection at the site of affliction, the active ingredient will preferably be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as required. Whether it is a polypeptide, peptide, or nucleic acid molecule, other pharmaceutically useful compound according to the present invention that is to be given to an individual, administration is preferably in a "prophylactically effective amount" or a "therapeutically effective amount" (as the case may be), this being sufficient to show benefit to the individual. The actual amount administered, and rate and time-course of administration, will depend on the nature and severity of what is being treated. For injection, the pharmaceutical composition according to the present invention may be provided for example in a pre-filled syringe.

Sterile injectable solutions can be prepared by incorporating the active ingredient in the required amount in an appropriate solvent with one or a combination of ingredients enumerated above, as required, followed by filtered sterilization. Sterile injectable forms of the compositions described may be aqueous or oleaginous suspension. These suspensions may be formulated according to techniques known in the art using suitable dispersing or wetting agents and suspending agents. The sterile, injectable preparation may be a sterile, injectable solution or suspension in a non-toxic parenterally acceptable diluent or solvent, for example as a suspension in 1,3-butanediol. Among the acceptable vehicles and solvents that may be employed are water, Ringer's solution and isotonic sodium chloride solution, hi addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents which are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation. Generally, dispersions are prepared by incorporating the active ingredient into a sterile vehicle that contains a basic dispersion medium and the required other ingredients from those enumerated above.

Solid forms suitable for solution in, or suspension in, liquid vehicles prior to injection can also be prepared (e.g., a lyophilized composition, similar to Synagis™ and Herceptin™, for reconstitution with sterile water containing a preservative). Accordingly, the composition may be in kit form, designed such that a combined composition is reconstituted just prior to administration to a subject. For example, a lyophilized antibody may be provided in kit form with sterile water or a sterile buffer.

In the case of sterile powders for the preparation of sterile injectable solutions, the preferred methods of preparation are vacuum drying and freeze-drying that yields a powder of the active ingredient plus any additional desired ingredient from a previously sterile-filtered solution. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

The ligand according to the invention can be formulated with a controlled-release formulation or device. Examples of such formulations and devices include implants, transdermal patches, and microencapsulated delivery systems. Biodegradable, biocompatible polymers can be used, for example, ethylene vinyl acetate, polyanhydrides, polyglycolic acid, collagen, polyorthoesters, and polylactic acid. Methods for the preparation of such formulations and devices are known in the art. See e.g., Sustained and Controlled Release Drug Delivery Systems, J. R. Robinson, ed., Marcel Dekker, Inc., New York, 1978.

Injectable depot formulations can be made by forming microencapsulated matrices of the drug in biodegradable polymers such as polylactide-polyglycolide. Depending on the ratio of drug to polymer, and the nature of the polymer employed, the rate of drug release can be controlled. Other exemplary biodegradable polymers are polyorthoesters and polyanhydrides. Depot injectable formulations also can be prepared by entrapping the drug in liposomes or microemulsions.

It is also preferred that the pharmaceutical composition may be prepared for oral administration, e.g. as tablets or capsules or as injectable, e.g. as liquid solutions or suspensions, whereby it is particularly preferred that the pharmaceutical composition is an injectable. Solid forms suitable for solution in, or suspension in, liquid vehicles prior to injection are also be preferred, e.g. that the pharmaceutical composition is in lyophilized form. Orally acceptable dosage forms include, but are not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient, i.e. the inventive transporter cargo conjugate molecule as defined above, is combined with emulsifying and suspending agents. If desired, certain sweetening, flavoring or coloring agents may also be added.

The inventive pharmaceutical composition may also be administered topically. For topical applications, the inventive pharmaceutical composition may be formulated in a suitable ointment, cream or powder containing the inventive pharmaceutical composition, particularly its components as defined above, suspended or dissolved in one or more carriers. Carriers for topical administration include, but are not limited to, mineral oil, liquid petrolatum, white petrolatum, propylene glycol, polyoxyethylene, polyoxypropylene compound, emulsifying wax and water. Alternatively, the inventive pharmaceutical composition can be formulated in a suitable lotion or cream. In the context of the present invention, suitable carriers include, but are not limited to, mineral oil, sorbitan monostearate, polysorbate 60, cetyl esters wax, cetearyl alcohol, 2-octyldodecanol, benzyl alcohol and water.

A thorough discussion of pharmaceutically acceptable carriers is available in Remington: The Science and Practice of Pharmacy, 20th edition, 2000, ISBN: 0683306472.

Pharmaceutical compositions typically include an "effective" amount of the ligand, in particular of the anti-PrP antibody or of an antigen-binding fragment thereof, i.e. an amount that is sufficient to treat, ameliorate, attenuate or prevent a synucleinopathy, or to exhibit a detectable therapeutic effect. Therapeutic effects also include reduction or attenuation in pathogenic potency or physical symptoms. The precise effective amount for any particular subject will depend upon their size, weight, and health, the nature and extent of the condition, and the therapeutics or combination of therapeutics selected for administration. The effective amount for a given situation is determined by routine experimentation and is within the judgment of a clinician.

Moreover, the pharmaceutical composition according to the present invention may also comprise an additional active component, which may be a further antibody or a component, which is not an antibody. Accordingly, the pharmaceutical composition according to the present invention may comprise one or more of the additional active components, e.g. as described below in the context of a combination therapy, for example an antiparkinson medication as described below.

In one embodiment, a composition of the invention may include anti-PrP antibodies or antigen binding fragments thereof, wherein the antibodies or the antigen-binding fragments may make up at least 50% by weight (*e.g.,* 60%, 70%, 75%, 80%, 85%, 90%, 95%, 96%, 97%, 98%, 99% or more) of the total protein in the composition. In such a composition, the antibodies or the antigen binding fragments are preferably in purified form.

The present invention also provides a method of preparing a pharmaceutical composition comprising the steps of: (i) preparing a ligand as described above, in particular an anti-PrP antibody or an antigen binding fragment thereof; and (ii) admixing the purified antibody with one or more pharmaceutically-acceptable carriers.

### Prevention and/or treatment of synucleinopathies

According to the present invention the ligand, in particular the anti-PrP antibody or the antigen-binding fragment thereof, the conjugate, the nucleic acid molecule or the pharmaceutical composition are provided for use in the prevention and/or treatment of a synucleinopathy.

Synucleinopathies (also called α-Synucleinopathies) are characterized by the abnormal accumulation of aggregates of α-synuclein in neurons, nerve fibres or glial cells. Typically, synucleinopathies are neurodegenerative diseases, at least in the later stages. There are three main types of synucleinopathies, Parkinson's disease (PD), dementia with Lewy bodies (DLB), and multiple system atrophy (MSA), with other rare disorders also having α-synuclein pathologies, such as various neuroaxonal dystrophies. Accordingly, the ligand, in particular the anti-PrP antibody or the antigen-binding fragment thereof, the conjugate, the nucleic acid molecule or the pharmaceutical composition are preferably used in subjects showing (i) an accumulation of aggregates of α-synuclein, in particular Lewy bodies and/or Lewy neurites, and/or (ii) other symptoms of a synucleinopathy, such as Parkinson's disease, dementia with Lewy bodies, and multiple systems atrophy. Accumulation of aggregates of α-synuclein, in particular Lewy bodies and/or Lewy neurites, may occur in particular in the central nervous system (CNS), in the peripheral nervous system (PNS) and in the enteric nervous system (ENS).

Preferably, the synucleinopathy is selected from Parkinson's disease, dementia with Lewy bodies, and multiple systems atrophy. More preferably, the synucleinopathy is Parkinson's disease.

In particular, the ligand, in particular the anti-PrP antibody or the antigen-binding fragment thereof, the conjugate, the nucleic acid molecule or the pharmaceutical composition may be preferably used in incidental Lewy body disease (ILBD). ILBD typically refers to subjects showing Lewy bodies, but who are otherwise clinically asymptomatic (regarding parkinsonism). ILBD is under discussion as precursor or very early stage of PD (Dickson DW, Fujishiro H, DelleDonne A, Menke J, Ahmed Z, Klos KJ, Josephs KA, Frigerio R, Burnett M, Parisi JE, Ahlskog JE: Evidence that incidental Lewy body disease is pre-symptomatic Parkinson's disease. Acta Neuropathol 2008, 115:437-444). Since the present inventors found that anti-PrP antibodies reduce uptake of α-synuclein fibrils, the ligand, in particular the anti-PrP antibody or the antigen-binding fragment thereof, for use according to the present invention may be very useful in particular in "arresting" or delaying the progress of a synucleinopathy. This even enables the prevention of, for example, Parkinson's disease, dementia with Lewy bodies, and/or multiple systems atrophy, since the spreading of α-synuclein fibrils can be prevented or reduced even at a very early, (otherwise) clinically asymptomatic stage (which is usually not yet diagnosed as Parkinson's disease, dementia with Lewy bodies, and/or multiple systems atrophy, such as in ILBD).

Even though in ILBD lewy bodies are mostly reported in the brain, a recent study reported patients who were found to have α-syn staining in bowel biopsy samples obtained 2-5 years before they presented with signs of PD (Shannon KM, Keshavarzian A, Mutlu E, Dodiya HB, Daian D, Jaglin JA, Kordower JH: Alpha-synuclein in colonic submucosa in early untreated Parkinson's disease. Mov Disord 2012, 27:709-715). It is generally well established that Lewy pathology occurs in the gastrointestinal tract in early, mid- and late PD (Braak H, de Vos RA, Bohl J, Del Tredici K: Gastric alpha-synuclein immunoreactive inclusions in Meissner's and Auerbach's plexuses in cases staged for Parkinson's disease-related brain pathology. Neurosci Lett 2006, 396:67-72; Lebouvier T, Chaumette T, Damier P, Coron E, Touchefeu Y, Vrignaud S, Naveilhan P, Galmiche JP: Bruley des Varannes S, Derkinderen P, Neunlist M: Pathological lesions in colonic biopsies during Parkinson's disease. Gut 2008, 57:1741-1743; Lebouvier T, Neunlist M, Bruley des Varannes S, Coron E, Drouard A, N'Guyen JM, Chaumette T, Tasselli M, Paillusson S, Flamand M, et al: Colonic biopsies to assess the neuropathology of Parkinson's disease and its relationship with symptoms. PLoS One 2010, 5:e12728; Shannon KM, Keshavarzian A, Mutlu E, Dodiya HB, Daian D, Jaglin JA, Kordower JH: Alpha-synuclein in colonic submucosa in early untreated Parkinson's disease. Mov Disord 2012, 27:709-715; Wakabayashi K, Takahashi H, Takeda S, Ohama E, Ikuta F: Parkinson's disease: the presence of Lewy bodies in Auerbach's and Meissner's plexuses. Acta Neuropathol 1988, 76:217-221). Accordingly, enteral routes of administration, in particular oral administration, are preferred in the context of the present invention for the prevention and/or treatment of a synucleinopathy, such as Parkinson's disease.

In general, the ligand, in particular the anti-PrP antibody or the antigen-binding fragment thereof, the conjugate, the nucleic acid molecule or the pharmaceutical composition may be used for the prevention and/or treatment at any stage of a synucleinopathy. Even though "regeneration" of nerve cells, once damaged, occurs rather rarely, in particular in the CNS - and thus a complete cure at late stages of, for example, PD is rather unlikely, the ligand, in particular the anti-PrP antibody or the antigen-binding fragment thereof, for use according to the present invention is - without being bound to any theory - thought to "arrest" or delay the progress of the synuceinopathy, which is typically a key issue at every stage of a synucleinopathy.

For example, Parkinson's disease may be staged according to Braak ("Braak staging"; Braak H, Del Tredici K, et al. (2003). "Staging of brain pathology related to sporadic Parkinson's disease." Neurobiol Aging. 24(2): 197-211). According to the Braak staging Lewy bodies first appear in the olfactory bulb, medulla oblongata and pontine tegmentum, individuals at this stage being asymptomatic. As the disease evolves, Lewy bodies later attain the substantia nigra, areas of the midbrain and basal forebrain, and finally reach areas of the neocortex. Briefly, during presymptomatic Braak stages 1-2, inclusion body pathology is confined to the medulla oblongata/pontine tegmentum and olfactory bulb/anterior olfactory nucleus. In Braak stages 3-4, the substantia nigra and other nuclear grays of the midbrain and forebrain become the focus of initially slight and, then, severe pathological changes. At this point, most individuals probably cross the threshold to the symptomatic phase of the illness. In the end-stages 5-6, the process enters the mature neocortex, and the disease manifests itself in all of its clinical dimensions (Braak H, Ghebremedhin E, Rub U, Bratzke H, Del Tredici K. Stages in the development of Parkinson's disease-related pathology. Cell Tissue Res. 2004; 318:121-134). Accordingly, it is preferred to use the ligand, in particular the anti-PrP antibody or the antigen-binding fragment thereof, the conjugate, the nucleic acid molecule or the pharmaceutical composition for treatment and/or prevention of PD Braak stage 1. It is also preferred to use the ligand, in particular the anti-PrP antibody or the antigen-binding fragment thereof, the conjugate, the nucleic acid molecule or the pharmaceutical composition for treatment and/or prevention of PD Braak stage 2. It is also preferred to use the ligand, in particular the anti-PrP antibody or the antigen-binding fragment thereof, the conjugate, the nucleic acid molecule or the pharmaceutical composition for treatment and/or prevention of PD Braak stage 3. It is also preferred to use the ligand, in particular the anti-PrP antibody or the antigen-binding fragment thereof, the conjugate, the nucleic acid molecule or the pharmaceutical composition for treatment and/or prevention of PD Braak stage 4. It is also preferred to use the ligand, in particular the anti-PrP antibody or the antigen-binding fragment thereof, the conjugate, the nucleic acid molecule or the pharmaceutical composition for treatment and/or prevention of PD Braak stage 5. It is also preferred to use the ligand, in particular the anti-PrP antibody or the antigen-binding fragment thereof, the conjugate, the nucleic acid molecule or the pharmaceutical composition for treatment and/or prevention of PD Braak stage 6. However, it is noted that Braak staging of PD is currently under discussion, in particular regarding whether or not asymptomatic early Braak stages are indeed to be classified as PD, or whether PD may only be diagnosed at later, symptomatic Braak stages (Burke RE, Dauer WT, Vonsattel JPG. A Critical Evaluation of The Braak Staging Scheme for Parkinson's Disease. Annals of neurology. 2008;64(5):485-491). In summary, the ligand, in particular the anti-PrP antibody or the antigen-binding fragment thereof, the conjugate, the nucleic acid molecule or the pharmaceutical composition may be used for treatment and/or prevention of early, mid or late PD.

In order to prevent and/or treat a synucleinopathy, the ligand, in particular the anti-PrP antibody or the antigen-binding fragment thereof, the conjugate, the nucleic acid molecule or the pharmaceutical composition may be administered by any suitable method, e.g., parenterally, orally, by inhalation spray, topically, rectally, nasally, buccally, intra-CSF or via an implanted reservoir. The term "parenteral" as used herein includes subcutaneous, intravenous, intramuscular, intra-articular, intra-synovial, intrastemal, intrathecal, intrahepatic, intralesional and intracranial injection or infusion techniques.

Preferably, the ligand, in particular the anti-PrP antibody or the antigen-binding fragment thereof, the conjugate, the nucleic acid molecule or the pharmaceutical composition may be administered in such a way that it crosses the blood-brain barrier. This crossing can result from the physico-chemical properties inherent in the ligand molecule itself, tagging or linking the ligand to a vehicle to facilitate crossing the blood-brain barrier, or from other components in a pharmaceutical formulation, or from the use of a mechanical device such as a needle, cannula or surgical instrument to breach the blood-brain barrier. Where the ligand is a molecule that does not inherently cross the blood-brain barrier, e.g. a fusion to a moiety that facilitates the crossing, suitable routes of administration are, e.g., intra-CSF, intrathecal or intracranial. Where the ligand is a molecule that inherently crosses the blood-brain barrier, the route of administration may be by one or more of the various routes described above. Preferably, the ligand, in particular the anti-PrP antibody or the antigen-binding fragment thereof, the conjugate, the nucleic acid molecule or the pharmaceutical composition may be administered intracerebral. More preferably, the ligand, in particular the anti-PrP antibody or the antigen-binding fragment thereof, the conjugate, the nucleic acid molecule or the pharmaceutical composition may be administered peripherally e.g. intravenously or subcutaneously, for example by injection. It is also preferred to administer the ligand, in particular the anti-PrP antibody or the antigen-binding fragment thereof, the conjugate, the nucleic acid molecule or the pharmaceutical composition intravenously or intramuscularly, for example by injection. The ligand, in particular the anti-PrP antibody or the antigen-binding fragment thereof, the conjugate, the nucleic acid molecule or the pharmaceutical composition is preferably administered orally, for example if aggregates of α-synuclein, such as Lewy bodies, are found in the gastrointestinal tract or in the vicinity thereof. Moreover, the ligand, in particular the anti-PrP antibody or the antigen-binding fragment thereof, the conjugate, the nucleic acid molecule or the pharmaceutical composition may also be administered topically, for example if aggregates of α-synuclein, such as Lewy bodies, are found in certain areas of the PNS, which are accessible via the topical route.

Preferably, the ligand, in particular the anti-PrP antibody or the antigen-binding fragment thereof, the conjugate, the nucleic acid molecule or the pharmaceutical composition is administered once or repeatedly. Accordingly, dosage treatment may be a single dose schedule or a multiple dose schedule. In particular the pharmaceutical composition may be provided as single-dose product. Preferably, the amount of the ligand, in particular the anti-PrP antibody or the antigen-binding fragment thereof, in the pharmaceutical composition - in particular if provided as single-dose product - does not exceed 200 mg, more preferably does not exceed 100 mg, and even more preferably does not exceed 50 mg.

For example, the ligand, in particular the anti-PrP antibody or the antigen-binding fragment thereof, the conjugate, the nucleic acid molecule or the pharmaceutical composition may be administered daily, e.g. once or several times per day, e.g. once, twice, three times or four times per day, preferably once or twice per day, more preferable once per day, for 1, 2, 3, 4, 5, 6, 7, 8, 9, 10, 11, 12, 13, 14, 15, 16, 17, 18, 19, 20 or 21 or more days, e.g. daily for 1, 2, 3, 4, 5, 6 months. Preferably, the ligand, in particular the anti-PrP antibody or the antigen-binding fragment thereof, the conjugate, the nucleic acid molecule or the pharmaceutical composition may be administered weekly, e.g. once or twice per week, for 1, 2, 3, 4, 5, 6, 7, 8, 9, 10, 11, 12, 13, 14, 15, 16, 17, 18, 19, 20 or 21 or more weeks, e.g. weekly for 1, 2, 3, 4, 5, 6, 7, 8, 9, 10, 11, or 12 months or weekly for 2, 3, 4, or 5 years. Moreover, the ligand, in particular the anti-PrP antibody or the antigen-binding fragment thereof, the conjugate, the nucleic acid molecule or the pharmaceutical composition may be preferably administered monthly, e.g. once per month or, more preferably, every second month for 1, 2, 3, 4, or 5 or more years. It is also preferred that the administration continues for the lifetime. In addition, also one single administration only is envisaged.

In particular, it is preferred that for a single dose, e.g. a daily, weekly or monthly dose, preferably for a weekly dose, the amount of the ligand, in particular the anti-PrP antibody or the antigen-binding fragment thereof, (in the pharmaceutical composition according to the present invention) does not exceed 1 g, preferably does not exceed 500 mg, more preferably does not exceed 200 mg, even more preferably does not exceed 100 mg, and particularly preferably does not exceed 50 mg.

For purposes of the present invention, an effective dose will generally be from about 0.005 to about 100 mg/kg, preferably from about 0.0075 to about 50 mg/kg, more preferably from about 0.01 to about 10 mg/kg, and even more preferably from about 0.02 to about 5 mg/kg, of the ligand, in particular the anti-PrP antibody or the antigen-binding fragment thereof, (e.g. amount of the antibody in the pharmaceutical composition) in relation to the bodyweight (e.g., in kg) of the individual to which it is administered.

Doses discussed are for human subjects unless otherwise indicated . Doses for other species are adjusted accordingly.

The ligand, in particular the anti-PrP antibody or the antigen-binding fragment thereof, may be used as part of a pharmaceutical kit, which may further comprise an administration means. Means for administration include, but are not limited to syringes and needles, catheters, biolistic injectors, particle accelerators, i.e., "gene guns," pneumatic "needleless" injectors, gelfoam sponge depots, other commercially available depot materials, e.g., hydrogels, osmotic pumps, and decanting, polynucleotide coated sutures, skin patches, or topical applications during surgery. Each of the pharmaceutical kits may further comprise an instruction sheet for administration of the composition to a mammal, in particular a human. If the ligand is provided in lyophilized form, the dried powder or cake may also include any salts, auxiliary agents, transfection facilitating agents, and additives of the composition in dried form. Such a kit may further comprise a container with an exact amount of sterile pyrogen-free water, for precise reconstitution of the lyophilized components of the composition.

When administering nucleic acid(s) for protein expression, suitably those are administered in amounts capable of supporting levels of expression corresponding to the administration levels mentioned for proteins. Polynucleotides of the invention may be administered directly as naked nucleic acid. When the polynucleotides/vectors are administered as naked nucleic acid, the amount of nucleic acid administered may typically be in the range of from 1 mg to 10 mg, preferably from 100 ig to 1 mg.

Accordingly, the present invention also provides a method for treating and/or preventing a synucleinopathy comprising administering to a subject the ligand as described above, in particular the anti-PrP antibody or the antigen-binding fragment thereof, the conjugate, the nucleic acid molecule or the pharmaceutical composition as described above. Preferably, the synucleinopathy is selected from Parkinson's disease, dementia with Lewy bodies, and multiple systems atrophy. More preferably, the synucleinopathy is Parkinson's disease.

It has been surprisingly found that PrP binds to α-synuclein, which facilitates uptake of α-synuclein fibrils. Moreover, it has been surprisingly found that anti-PrP antibodies reduce uptake of α-synuclein fibrils, presumably - without being bound to any theory - by blocking the binding of PrP to α-synuclein. In view thereof, the present invention also provides a method for reducing uptake of α-synuclein fibrils comprising administering to a subject the ligand as described above, in particular the anti-PrP antibody or the antigen-binding fragment thereof, the conjugate, the nucleic acid molecule or the pharmaceutical composition as described above. Preferably, the subject suffers from a synucleinopathy, in particular characterized by an abnormal accumulation of aggregates of α-synuclein, more preferably, the subject shows additionally signs of neurodegeneration.

### Combination therapy and Kits

In a further aspect the present invention provides a combination of
(i) the ligand as described above, the conjugate as described above, the nucleic acid molecule as described above or the pharmaceutical composition as described above, and
(ii) an antiparkinson medication
for use in the prevention and/or treatment of a synucleinopathy, in particular of Parkinson's disease.

In general, a "combination" of the ligand as described above, the conjugate as described above, the nucleic acid molecule as described above or the pharmaceutical composition as described above and of the antiparkinson medication means that the therapy with the ligand as described above, the conjugate as described above, the nucleic acid molecule as described above or the pharmaceutical composition as described above is combined with the therapy with the antiparkinson medication. In other words, even if one component ((i) the ligand as described above, the conjugate as described above, the nucleic acid molecule as described above or the pharmaceutical composition as described above; or (ii) the antiparkinson medication) is not administered, e.g., at the same day as the other component (the other of (i) the ligand as described above, the conjugate as described above, the nucleic acid molecule as described above or the pharmaceutical composition as described above and (ii) the antiparkinson medication), their treatment schedules are intertwined. This means that "a combination" in the context of the present invention does in particular not include the start of a therapy with one component ((i) the ligand as described above, the conjugate as described above, the nucleic acid molecule as described above or the pharmaceutical composition as described above; or (ii) the antiparkinson medication) after the therapy with the other component (the other of (i) the ligand as described above, the conjugate as described above, the nucleic acid molecule as described above or the pharmaceutical composition as described above and (ii) antiparkinson medication) is finished. In more general, an "intertwined" treatment schedule of (i) the ligand as described above, the conjugate as described above, the nucleic acid molecule as described above or the pharmaceutical composition as described above and (ii) the antiparkinson medication - and, thus, a combination of (i) the ligand as described above, the conjugate as described above, the nucleic acid molecule as described above or the pharmaceutical composition as described above and (ii) the antiparkinson medication - means that:
(a) not every administration of (i) the ligand as described above, the conjugate as described above, the nucleic acid molecule as described above or the pharmaceutical composition as described above (and therefore the complete therapy with the ligand as described above, the conjugate as described above, the nucleic acid molecule as described above or the pharmaceutical composition as described above) is completed for more than one week (preferably for more than 3 days, more preferably for more than 2 days, even more preferably for more than a day) before the first administration of the antiparkinson medication (and therefore the complete therapy with the antiparkinson medication) starts; or
(b) not every administration of the antiparkinson medication (and therefore the complete therapy with the antiparkinson medication) is completed for more than one week (preferably for more than 3 days, more preferably for more than 2 days, even more preferably for more than a day) before the first administration of the ligand as described above, the conjugate as described above, the nucleic acid molecule as described above or the pharmaceutical composition as described above (and therefore the complete therapy with the ligand as described above, the conjugate as described above, the nucleic acid molecule as described above or the pharmaceutical composition as described above) starts.

For example, in the combination of (i) the ligand as described above, the conjugate as described above, the nucleic acid molecule as described above or the pharmaceutical composition as described above and (ii) of the antiparkinson medication, one component ((i)the ligand as described above, the conjugate as described above, the nucleic acid molecule as described above or the pharmaceutical composition as described above or (ii) the antiparkinson medication) may be administered once a week and the other component (the other of (i) the ligand as described above, the conjugate as described above, the nucleic acid molecule as described above or the pharmaceutical composition as described above and (ii) antiparkinson medication) may be administered once a month. To achieve in this example "a combination" in the sense of the present invention the monthly administered component is to be administered at least once in the same week, in which also the weekly administered other component is administered.

The goal of the most common antiparkinson medications is to either replace the dopamine levels in the brain, or mimic the actions of dopamine. The main categories of antiparkinson medications are anticholinergic drugs and dopaminergic drugs. Anticholinergic drugs block the action of acetylcholine, compensating for the low levels of dopamine, whereas dopaminergic drugs aim to replace dopamine or inhibit the degradation of dopamine in the brain. Preferably, the term "antiparkinson medication" does not include the PrP ligands as described herein. In particular, the term "antiparkinson medication" does not include anti-PrP antibodies or antigen-binding fragments thereof. Preferably, the antiparkinson medication is a conventional and/or symptomatic antiparkinson medication. Symptomatic treatment usually refers to the treatment of specific symptoms of PD (such as motor symptoms etc.), but not to treatment of the underlying cause of PD. It may be also preferred that the antiparkinson medication targets α-synuclein and is, thus, useful in the treatment of synucleinopathies in general, including Parkinson's disease and other synucleinopathies.

Preferably, the ligand as described above, in particular the anti-PrP antibody or the antigen-binding fragment thereof, and the antiparkinson medication provide an additive therapeutic effect or, preferably, a synergistic therapeutic effect. The term "synergy" is used to describe a combined effect of two or more active agents that is greater than the sum of the individual effects of each respective active agent. Thus, where the combined effect of two or more agents results in "synergistic inhibition" of an activity or process, it is intended that the inhibition of the activity or process is greater than the sum of the inhibitory effects of each respective active agent. The term "synergistic therapeutic effect" refers to a therapeutic effect observed with a combination of two or more therapies wherein the therapeutic effect (as measured by any of a number of parameters) is greater than the sum of the individual therapeutic effects observed with the respective individual therapies.

In general, the ligand as described above, in particular the anti-PrP antibody or the antigen-binding fragment thereof, can be present either (a) in the same pharmaceutical composition as the antiparkinson medication, or, preferably, the ligand, in particular the antibody, or (b) the ligand as described above, in particular the anti-PrP antibody or the antigen-binding fragment thereof, is comprised by a first pharmaceutical composition and the antiparkinson medication is comprised by a second pharmaceutical composition different from the first pharmaceutical composition. Accordingly, if more than one additional active component is envisaged, each additional active component and the ligand as described above, in particular the anti-PrP antibody or the antigen-binding fragment thereof, is preferably comprised by a distinct pharmaceutical composition. Such distinct pharmaceutical compositions may be administered either combined/simultaneously or at separate times or at separate locations (e.g. separate parts of the body). Preferably, (i) the ligand as described above, in particular the anti-PrP antibody or the antigen-binding fragment thereof, and (ii) the antiparkinson medication are administered separately. The ligand as described above, in particular the anti-PrP antibody or the antigen-binding fragment thereof, and the antiparkinson medication may be administered via distinct routes of administration or via the same route of administration. For example, the ligand as described above, in particular the anti-PrP antibody or the antigen-binding fragment thereof, may be administered intravenously or intramuscularly and the antiparkinson medication may be administered orally.

In the combination of (i) the ligand as described above, the conjugate as described above, the nucleic acid molecule as described above or the pharmaceutical composition as described above and (ii) the antiparkinson medication, (i) the ligand as described above, the conjugate as described above, the nucleic acid molecule as described above or the pharmaceutical composition as described above and (ii) the antiparkinson medication are preferably administered at about the same time.

"At about the same time", as used herein, means in particular simultaneous administration or that
(a) directly after administration of (i) the ligand as described above, the conjugate as described above, the nucleic acid molecule as described above or the pharmaceutical composition as described above, (ii) the antiparkinson medication is administered or
(b) directly after administration of (ii) the antiparkinson medication, (i) the ligand as described above, the conjugate as described above, the nucleic acid molecule as described above or the pharmaceutical composition as described above is administered.

The skilled person understands that "directly after" includes the time necessary to prepare the second administration - in particular the time necessary for exposing and disinfecting the location for the second administration as well as appropriate preparation of the "administration device" (e.g., syringe, pump, etc.). Simultaneous administration also includes if the periods of administration of (i) the ligand as described above, the conjugate as described above, the nucleic acid molecule as described above or the pharmaceutical composition as described above and of (ii) the antiparkinson medication overlap or if, for example, one component ((i)the ligand as described above, the conjugate as described above, the nucleic acid molecule as described above or the pharmaceutical composition as described above or (ii) antiparkinson medication) is administered over a longer period of time, such as 30 min, 1 h, 2 h or even more, e.g. by infusion, and the other component ((i)the ligand as described above, the conjugate as described above, the nucleic acid molecule as described above or the pharmaceutical composition as described above or (ii) antiparkinson medication) is administered at some time during such a long period. Administration of (i) the ligand as described above, the conjugate as described above, the nucleic acid molecule as described above or the pharmaceutical composition as described above and of (ii) the antiparkinson medication at about the same time is in particular preferred if different routes of administration and/or different administration sites are used.

It is also preferred in the combination of (i) the ligand as described above, the conjugate as described above, the nucleic acid molecule as described above or the pharmaceutical composition as described above and of (ii) the antiparkinson medication that (i) the ligand as described above, the conjugate as described above, the nucleic acid molecule as described above or the pharmaceutical composition as described above and (ii) the antiparkinson medication are administered consecutively. For example, the ligand as described above, the conjugate as described above, the nucleic acid molecule as described above or the pharmaceutical composition as described above is preferably administered before the antiparkinson medication. It is also preferred that the ligand as described above, the conjugate as described above, the nucleic acid molecule as described above or the pharmaceutical composition as described above is administered after the antiparkinson medication.

In consecutive administration, the time interval between administration of the first component ((i) the ligand as described above, the conjugate as described above, the nucleic acid molecule as described above or the pharmaceutical composition as described above or (ii) the antiparkinson medication) and administration of the second component (the other of (i) the ligand as described above, the conjugate as described above, the nucleic acid molecule as described above or the pharmaceutical composition as described above and (ii) the antiparkinson medication) is preferably no more than one week, more preferably no more than 3 days, even more preferably no more than 2 days and most preferably no more than 24 h. It is particularly preferred that (i) the ligand as described above, the conjugate as described above, the nucleic acid molecule as described above or the pharmaceutical composition as described above and (i) the antiparkinson medication are administered at the same day with the time between administration of the first component ((i) the ligand as described above, the conjugate as described above, the nucleic acid molecule as described above or the pharmaceutical composition as described above or (ii) the antiparkinson medication) and administration of the second component (the other of (i) the ligand as described above, the conjugate as described above, the nucleic acid molecule as described above or the pharmaceutical composition as described above and (ii) the antiparkinson medication) being preferably no more than 6 hours, more preferably no more than 3 hours, even more preferably no more than 2 hours and most preferably no more than 1 h.

Preferably, the antiparkinson medication is selected from dopaminergic precursors, COMT inhibitors, peripheral aromatic L-amino acid decarboxylase inhibitors, selective monoamine oxidase B inhibitors, dopamine receptor agonists, anticholinergics, positive allosteric modulators of mGluR4, and anti-α-synuclein antibodies. More preferably, the antiparkinson medication is a dopaminergic precursor, most preferably levodopa (L-DOPA).

Preferably, the antiparkinson medication is a dopaminergic precursor. Dopaminergic precursors are precursors of dopamine. From such precursors of dopamine dopamine itself can be easily produced by the body. Dopaminergic precursors may be administered in combination with another antiparkinson medication. Preferred examples of dopaminergic precursors include tyrosine and L-DOPA. L-DOPA (levodopa) is most preferred. L-DOPA can cross the blood-brain barrier, whereas dopamine itself cannot. L-DOPA is presently the standard treatment for PD. L-DOPA causes the person's remaining dopaminergic neurons to produce and secrete more dopamine, thereby counteracting the effects of Parkinson's disease. However, eventually the nigrostriatal dopaminergic neurons in the brain drop to a low enough count where the symptoms of Parkinson's disease become worse. This is due to the short half-life of L-DOPA in the body; typically 1.5-2 hours.

Preferably, the antiparkinson medication is a COMT inhibitor (inhibitors of catechol-O-methyl transferase). COMT inhibitors prevent the peripheral metabolism of levodopa by COMT (catechol-O-methyl transferase) and hence increase brain levels of L-DOPA. Accordingly COMT-inhibitors are preferably administered in combination with another antiparkinson medication, such as L-DOPA. Preferred examples of COMT-inhibitors include entacapone, tolcapone, opicapone and nitecapone.

Preferably, the antiparkinson medication is a peripheral aromatic L-amino acid decarboxylase inhibitor. Peripheral aromatic L-amino acid decarboxylase inhibitors (DOPA decarboxylase inhibitors) prevent the peripheral metabolism of levodopa by decarboxylases and hence increase brain levels of L-DOPA. Accordingly a peripheral aromatic L-amino acid decarboxylase inhibitors are preferably administered in combination with another antiparkinson medication, such as L-DOPA. Preferred examples of peripheral aromatic L-amino acid decarboxylase inhibitors include benserazide and Carbidopa.

Preferably, the antiparkinson medication is a selective monoamine oxidase B inhibitor. Selective monoamine oxidase B inhibitors prevent the metabolism of dopamine by MAO_{B} and hence increase its brain levels. Selective monoamine oxidase B inhibitors may be administered in combination with another antiparkinson medication, such as L-DOPA. Preferred examples of selective monoamine oxidase B inhibitors include deprenyl, selegiline and rasagiline.

Preferably, the antiparkinson medication is a dopamine receptor agonist. Dopamine receptor agonists directly increase the activity of the dopamine system. Dopamine receptor agonists may be administered in combination with another antiparkinson medication, such as L-DOPA. Preferred examples of dopamine receptor agonists (also referred to as "dopamine agonists") include apomorphine, bromocriptine, pramipexole, ropinirole, and rotigotine.

Preferably, the antiparkinson medication is an anticholinergic. Anticholinergics block the action of acetylcholine, thereby compensating for the low levels of dopamine. Anticholinergics may be administered in combination with another antiparkinson medication, such as L-DOPA. Preferred examples of anticholinergics include antimuscarinics, benzhexol, orphenadrine, benzatropine, diphenhydramine, dimenhydrinate, and scopolamine.

Preferably, the antiparkinson medication is a positive allosteric modulator of mGluR4 (metabotropic glutamate receptor 4). Positive allosteric modulators of mGluR4 were recently suggested as antiparkinson medications (Niswender CM, Johnson KA, Weaver CD, Jones CK, Xiang Z, Luo Q, Rodriguez AL, Marlo JE, de Paulis T, Thompson AD, Days EL, Nalywajko T, Austin CA, Williams MB, Ayala JE, Williams R, Lindsley CW, Conn PJ (November 2008). "Discovery, characterization, and antiparkinsonian effect of novel positive allosteric modulators of metabotropic glutamate receptor 4". Molecular Pharmacology. 74 (5): 1345-58). Positive allosteric modulators of mGluR4 may be administered in combination with another antiparkinson medication, such as L-DOPA. A preferred example of a positive allosteric modulator of mGluR4 is N-phenyl-7-(hydroxylimino)cyclopropa[b]chromen-1a-carboxamide (PHCCC).

It is also preferred that the antiparkinson medication is an anti-α-synuclein antibody. One example of anti-α-synuclein antibodies is PRX002, an anti-α-synuclein antibody developed by Prothena corporation for the treatment of Parkinson's disease, which is currently in clinical trials. Further examples of anti-α-synuclein antibodies, which may be suitable in the context of the present invention, are disclosed in Jeśko H, Lenkiewicz AM, Adamczyk A. Treatments and compositions targeting α-synuclein: a patent review (2010-2016). Expert Opin Ther Pat. 2017 Apr;27(4):427-438; Lee JS, Lee SJ. Mechanism of Anti-α-Synuclein Immunotherapy. J Mov Disord. 2016 Jan;9(1):14-9; Bergström AL, Kallunki P, Fog K. Development of Passive Immunotherapies for Synucleinopathies. Mov Disord. 2016 Feb;31(2):203-13; George S, Brundin P. Immunotherapy in Parkinson's Disease: Micromanaging Alpha-Synuclein Aggregation. J Parkinsons Dis. 2015;5(3):413-24; US 2009/208487 A1; EP 2450056; EP 2583978; EP 2771031; and US 2016/251416 A1.

Furthermore, more than one, for example two or more, antiparkinson medications may be selected from the group consisting of dopaminergic precursors, COMT inhibitors, peripheral aromatic L-amino acid decarboxylase inhibitors, selective monoamine oxidase B inhibitors, dopamine receptor agonists, anticholinergics, positive allosteric modulators of mGluR4, and anti-α-synuclein antibodies.

In a further aspect the present invention also provides a kit comprising
(i) the ligand as described above, the conjugate as described above, the nucleic acid molecule as described above or the pharmaceutical composition as described above; and
(ii) an antiparkinson medication.

Preferably, the antiparkinson medication is selected from dopaminergic precursors, COMT inhibitors, peripheral aromatic L-amino acid decarboxylase inhibitors, selective monoamine oxidase B inhibitors, dopamine receptor agonists, anticholinergics, positive allosteric modulators of mGluR4, and anti-α-synuclein antibodies. More preferably, the antiparkinson medication is a dopaminergic precursor, most preferably levodopa (L-DOPA).

Such a kit may be particularly useful in the combination therapy as described above. In particular, the kit may be used in the prevention and/or treatment of a synucleinopathy as described herein. The synucleinopathy is preferably selected from Parkinson's disease, dementia with Lewy bodies, and multiple systems atrophy. Most preferably, the synucleinopathy is PD.

### BRIEF DESCRIPTION OF THE FIGURES

In the following a brief description of the appended figures will be given. The figures are intended to illustrate the present invention in more detail. However, they are not intended to limit the subject matter of the invention in any way.
- Figure 1: shows for Example 1 the expression and purification of recombinant α-Syn proteins. (A) Purification steps of human (Hu α-Syn) and (B) mouse (Mo α-Syn,) α-Syn proteins. M: protein molecular weight markers; T0: whole-cell lysate from un-induced cells; T4: whole-cells lysate after 4 hours induction with 0.6 mM IPTG; Hu/Mo-Syn: purified α-Syn proteins (human and mouse). (C) Mass spectrometry confirmed molecular masses of the proteins (Hu α-Syn and Mo α-Syn). (D) Lag phases of fibril formation for human and mouse α-Syn protein. The mouse α-Syn protein forms fibrils much faster compared to human protein (13.3 ± 2.3 hours vs 2.4 ± 0.23 hours). Data are presented as mean ± SD of three experiments, four replicas for each. (E) Fibrillation curve of recombinant mouse α-Syn protein with the time course of ThT changes. The red-blue arrows and square indicates the collection of short α-Syn fibrils, while the pink arrow shows the final time point for the collection of long α-Syn fibrils. (F) Western blot depicts the biochemical profile of short amyloid fibrils before and after 5 min sonication, together with long amyloid fibrils that were sonicated (Sonicated long fibrils).
- Figure 2: shows for Example 1 AFM analysis of α-Syn fibrils. (A) AFM images of fibrillar α-Syn particles (left hand panel - non sonicated, right hand panel - sonicated). (B) Length quantification of fibrillar α-Syn before and after sonication process (left hand panel - non sonicated, right hand panel - sonicated). Data are represented as mean ± SD.
- Figure 3: shows for Example 2 the uptake of mouse α-Syn amyloid fibrils in N2a cells. (A) Uptake quantification after 24 hours incubation with neuroblastoma (N2a) cells that express and that were ablated for the PrP^{C} expression show that 82.1 ± 2.9 % of N2a PrP+/+ are able to uptake α-Syn amyloid fibrils in comparison to only 31.8 ± 4.7 % of N2a PrP-/- cells. Data are shown as mean ± SD (**P < 0.01, ***P < 0.0001 for two-way ANOVA with Bonferroni's posttests, N=3 experiments with total of four hundred cells), (B) with relative orthogonal views of confocal images. Interaction accounts for 1.21% of the total variance; F = 0.66. (C) Non-sonicated and sonicated α-Syn amyloid fibrils (in red) co-localize with the endogenous membrane-bound PrP^{C} (in green) on the surface of neuroblastoma cells, while α-Syn fibrillar species did not even bind plasma membrane of N2a PrP-/- cells. Scale bars 15 µm. (D) Uptake quantification after 24 hours incubation with primary cultures of hippocampal neurons deriving from FVB Prnp+/+ and FVB Prnp-/- mice. A total of four hundred cells were counted. Data are shown as mean ± SD. Data were evaluated by unpaired Students' *t*-test. Statistical analysis is indicated as: ** *P* < 0.01. (E) Representative images of control and α-Syn fibril treated hippocampal neurons after 24 hours. In red, α-syn fibrils; in green, MAP-2; and in blue, nuclei and cytoplasm (CellMask staining). Scale bars represent 10 µm.

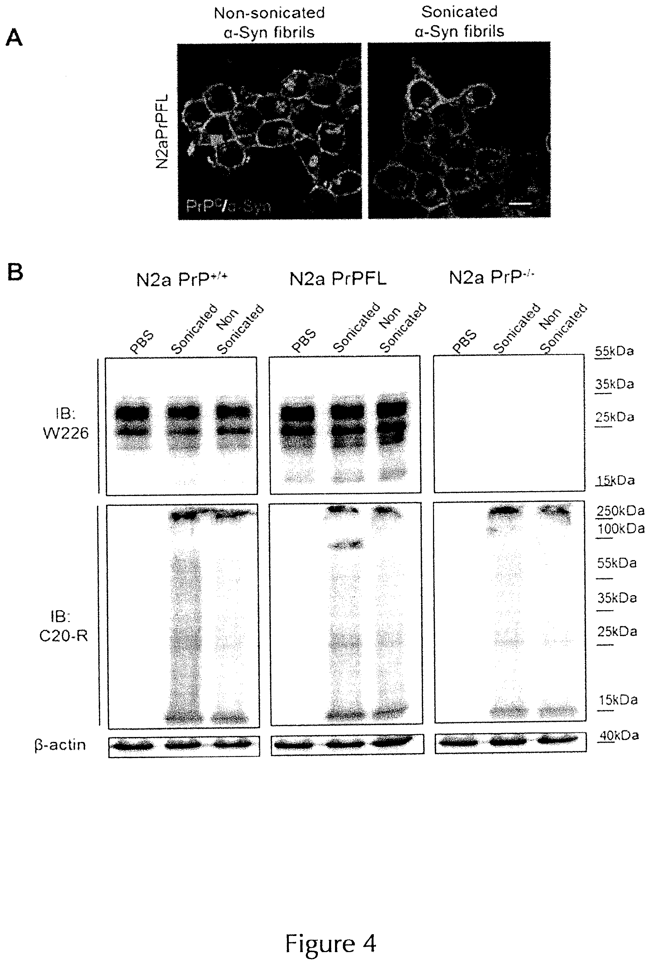

- Figure 4: shows for Example 2 PrP and α-Syn proteins after the incubation with non-sonicated and sonicated mouse α-Syn fibrils. (A) Immunofluorescence of N2aPrP-/- cells transfected with full-length PrP (N2aPrPFL, green) shows the co-localization with the exogenously added α- Syn amyloid fibrils (red) on the cell membrane. Scale bar 15 µm. (B) Western blots depict PrP^{C} and α-Syn protein levels after treatment with α-Syn amyloid preparations. Each line was loaded with 30 pg of total protein. β-Actin was used as a loading control.

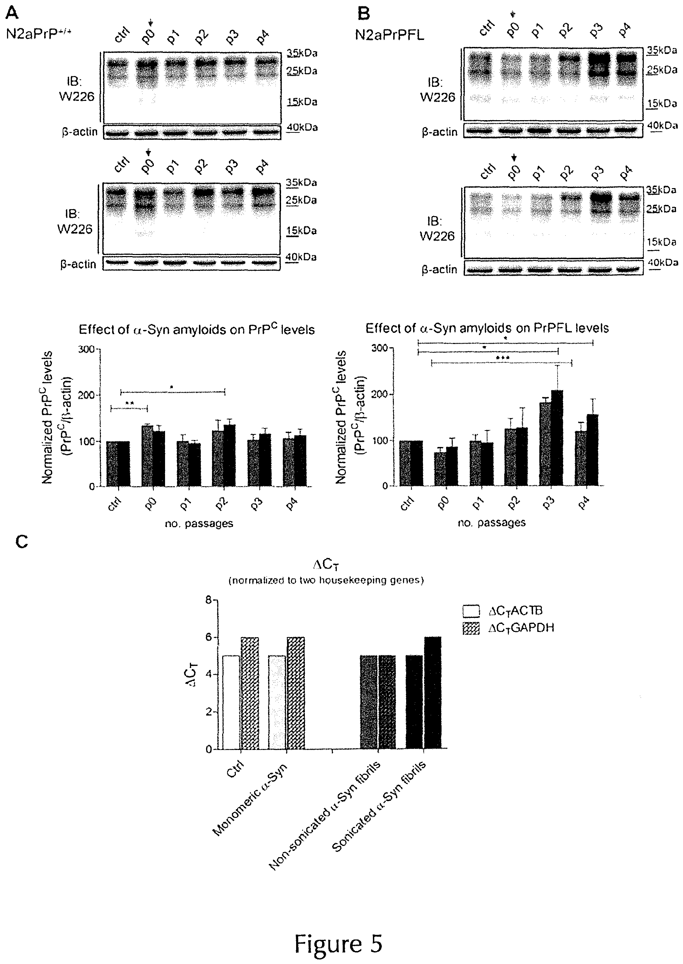

- Figure 5: shows for Example 2 the effect of mouse α-Syn preparations on endogenous PrP^{C} and transfected PrPFL protein levels in N2a cells. (A) The cell lysates of N2a PrP+/+ cells and (B) transfected N2aPrPFL cells were prepared and analyzed by SDS-PAGE and WB, using W226 anti-PrP antibody. Arrows at P0 indicate the treatment with non-sonicated and sonicated α-Syn amyloid fibrils. Each line was loaded with 30 pg of total protein. Lower graphs show the quantification of three independent experiments, after treatment with non-sonicated α-Syn amyloids (red columns) and sonicated α-Syn amyloids (blue columns). The values are shown as a percentage of total PrP relative to actin. Data are represented as mean ± SD. Data were evaluated by unpaired *t*-test. Statistical analysis is indicated as: * P < 0.05, ** P < 0.01, *** P < 0.001. (C) RT-PCR analysis of N2a non-treated and treated samples. ΔCT values for *Prnp* gene shows no variability among control and α-Syn treated samples. Normalization of RT-qPCR data was performed on two housekeeping genes, ACTB (empty column) and GAPDH (diagonal brick pattern).

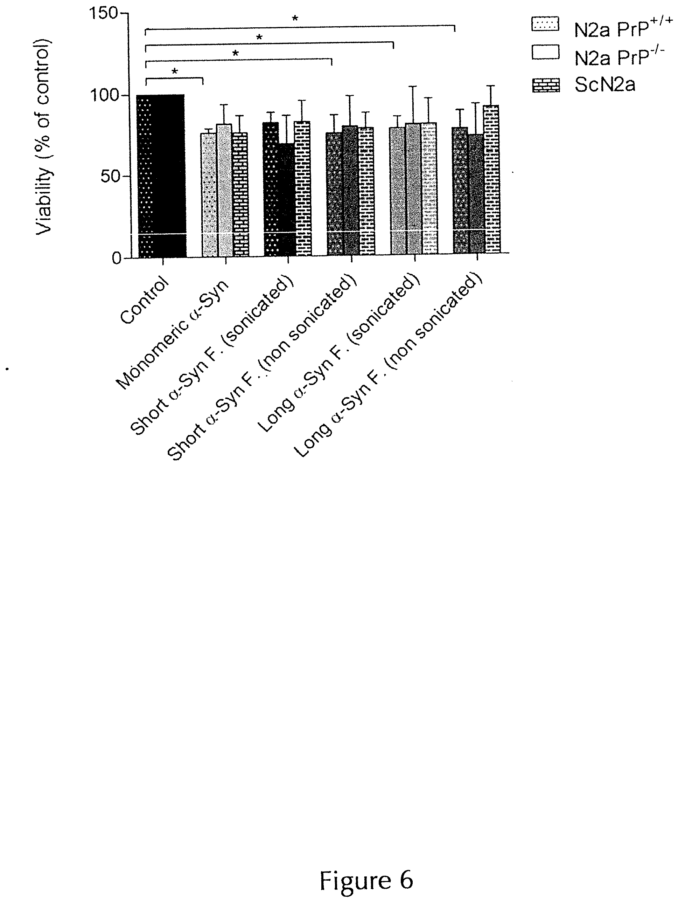

- Figure 6: shows for Example 2 *in-vitro* cell cytotoxicity. Effect of different α-Syn forms (monomeric and fibrils) on growth of N2a PrP+/+, N2a PrP-/-, ScN2a cells measured by the MTT assay. Data are shown as mean ± SD of three separate experiments, each performed in six replicates. Treatments significantly different from the untreated controls at P <0.05 are presented as *.

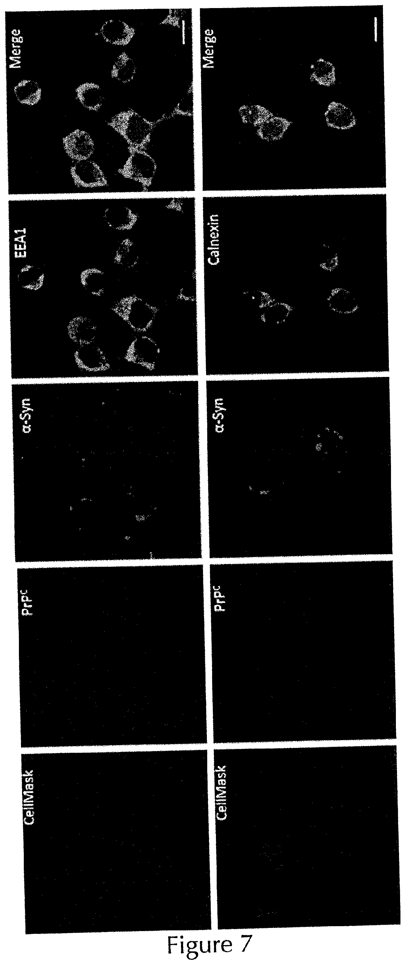

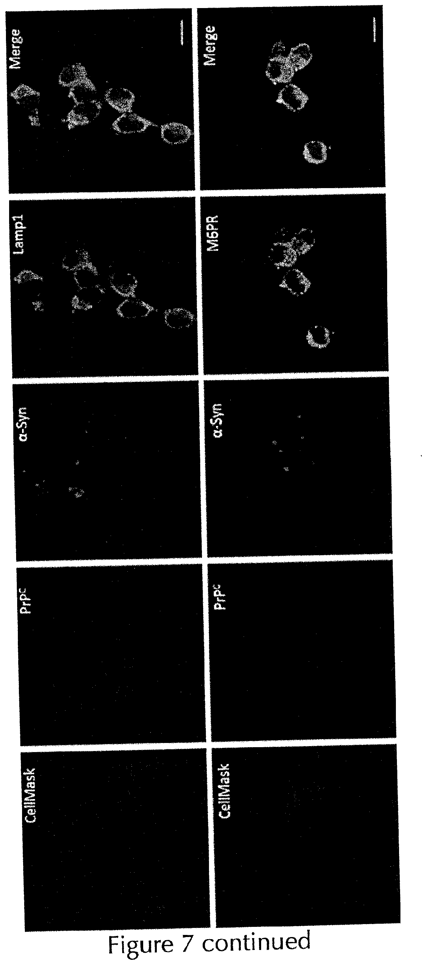

- Figure 7: shows for Example 2 the detection of α-Syn fibrils in cellular compartments by immunofluorescence in N2a PrP-/- cells. Confocal quadruple-labeled immunofluorescent images on N2a PrP-/- cells show the localization of the α-Syn fibrils (red) outside the cells. Other cellular compartments were investigated (EE1, early endosome; Calnexin, endoplasmatic reticulum; LAMP1, lysosomes; Mannose 6 Phosphate Receptor-M6PR, Golgi apparatus). Scale bars 10 µm.

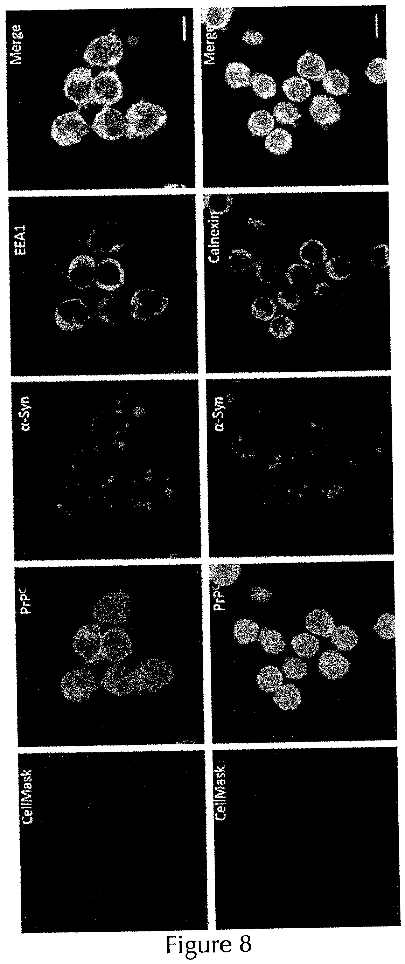

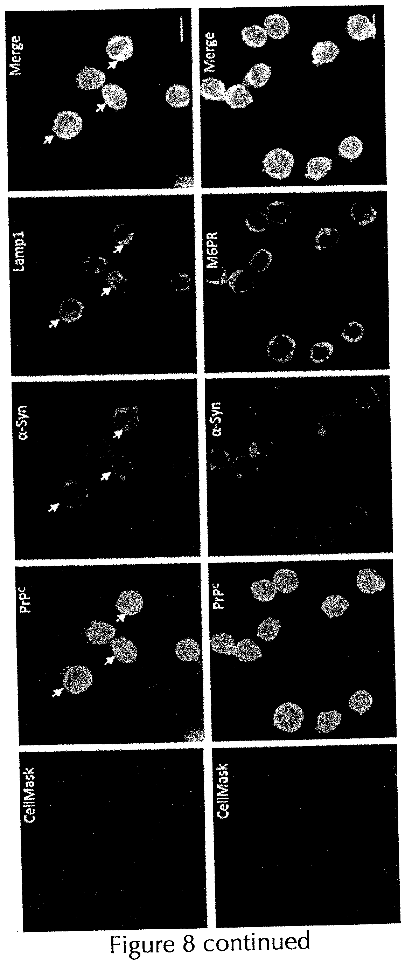

- Figure 8: shows for Example 2 the detection of α-Syn fibrils in cellular compartments by immunofluorescence in N2a PrP+/+ cells. Confocal quadruple-labeled immunofluorescent images on N2a PrP+/+ cells show the localization of the α-Syn fibrils (red) in the lysosomal compartments (LAMP1, cyan). Other cellular compartments were investigated (EE1, early endosome; Calnexin, endoplasmatic reticulum; Mannose 6 Phosphate Receptor-M6PR, Golgi apparatus). Scale bars 10 µm.

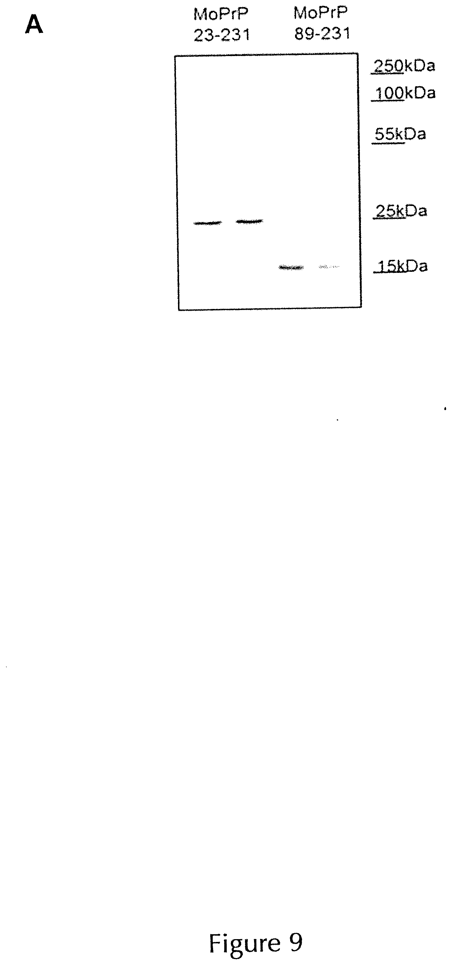

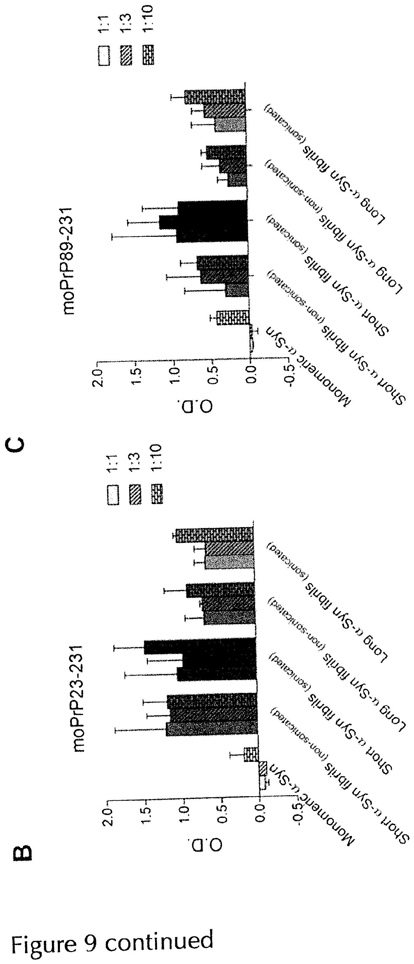

- Figure 9: shows for Example 3 that recombinant PrP protein binds α-Syn amyloids. (A) Western blot of recombinant full-length and truncated PrP protein (MoPrP(23-231) and MoPrP(89-231)); (B and C) ELISA plates were precoated with 50 ng of recPrP and three different ratios of different forms α-Syn (1:1, 1:3, 1:10) were incubated for 30min at 37°C.

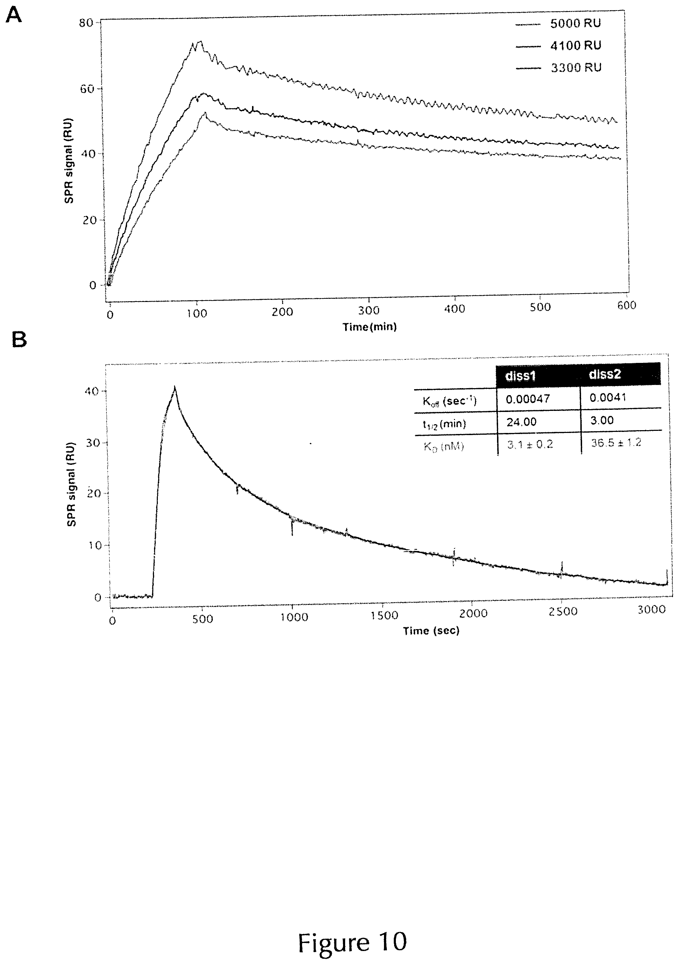

- Figure 10: shows for Example 3 the binding of monomeric and fibrillar α-Syn particles to immobilized sonicated α-Syn fibrils on CM5 biosensor chip. (A) Addition of monomeric α-Syn to sonicated α-Syn amyloid surfaces. Three different densities (3300, 4100, and 5000 RU of sonicated α-Syn amyloid fibrils were immobilized in separate flow cells. Monomeric α-Syn was injected at a concentration of 3 µM over the biosensor chip for 3 min at 50 µL/min association phase) and afterwards flushing with running buffer (dissociation phase). Figure shows the interaction with monomeric α-Syn as a positive control. (B) SPR sensogram shows MoPrP(23-231) binding to α-Syn amyloid fibrils immobilized on CM5 biosensor chip. After the immobilization, soluble recombinant full-length MoPrP (100 nM) was injected across the biosensor chip (binding phase) followed by the injection of buffer alone (dissociation phase). The sensogram showed that the full-length MoPrP binds to the sonicated α-Syn amyloid fibrils. Association and dissociation phases of full-length MoPrP interaction with sonicated α-Syn amyloid fibrils were elaborated using double referencing and analyzed separately: the association phase was fitted with a single exponential equation, determining a unique value of k*on;* the dissociation phase was analyzed with a double exponential equation, since the sensogram was more adequately fitted by a biphasic model (significantly improved error parameters). The included table indicates the KD values calculated using the formula KD=K*on*/K*off.*

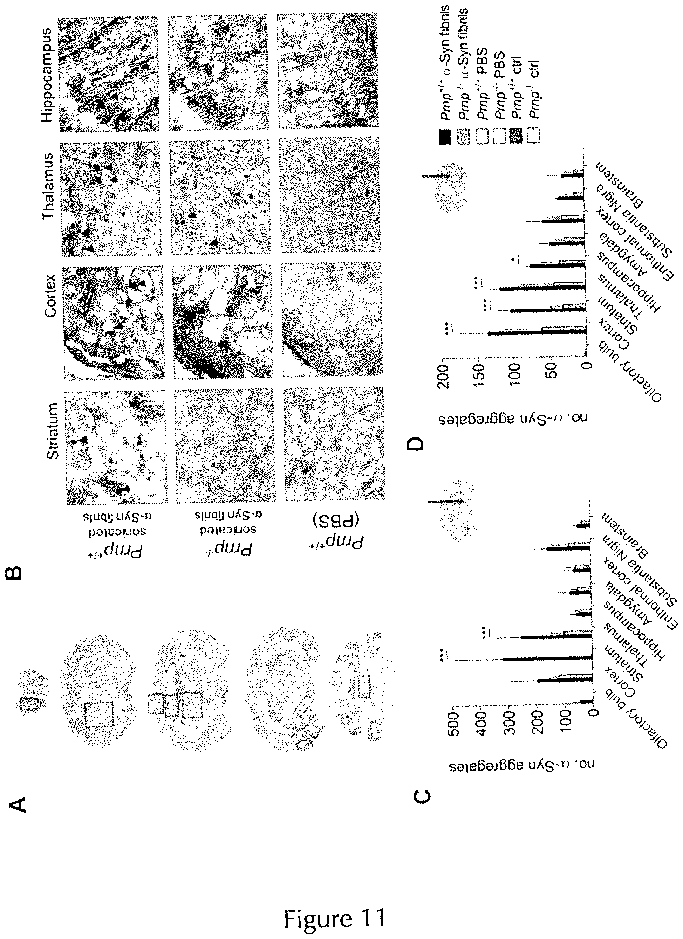

- Figure 11: shows for Example 4 that stereotaxic inoculation of sonicated α-Syn amyloid fibrils seeds the aggregation of endogenous mouse α-Syn in FVB mice. (A) Four different levels of CNS considered for the counting of α-Syn deposits (olfactory bulb; striatum; motor cortex, M1, M2; hippocampus CA1, CA2, CA3; thalamus; amygdala; Substantia nigra; enthorinal cortex; brainstem). (B) Accumulation of PK-resistant α-Syn deposits in striatum, cerebral cortex, thalamus, and hippocampus in mice injected in Substatia nigra. Scale bar 50 µm. (C and D) Quantification of PK-resistant α-Syn deposits in all considered brain areas show that *Prnp*+/+ FVB mice are able to accumulate more α-Syn deposits compared with *Prnp-*/*-. Prnp*+/+ FVB mice accumulate more PK-resistant α-Syn when injected in the *Substantia nigra* (C), or in the striatum (D) compared to *Prnp-*/*-.* Data are represented as mean ± SD, for two-way ANOVA with Bonferroni's posttests, *N*=3 animals per group. For (C) interaction accounts for 27.82% of the total variance; F = 4.86. The *P* value is < 0.0001. For (D) interaction accounts for 22.09% of the total variance; F = 5.57. The *P* value is < 0.0001).

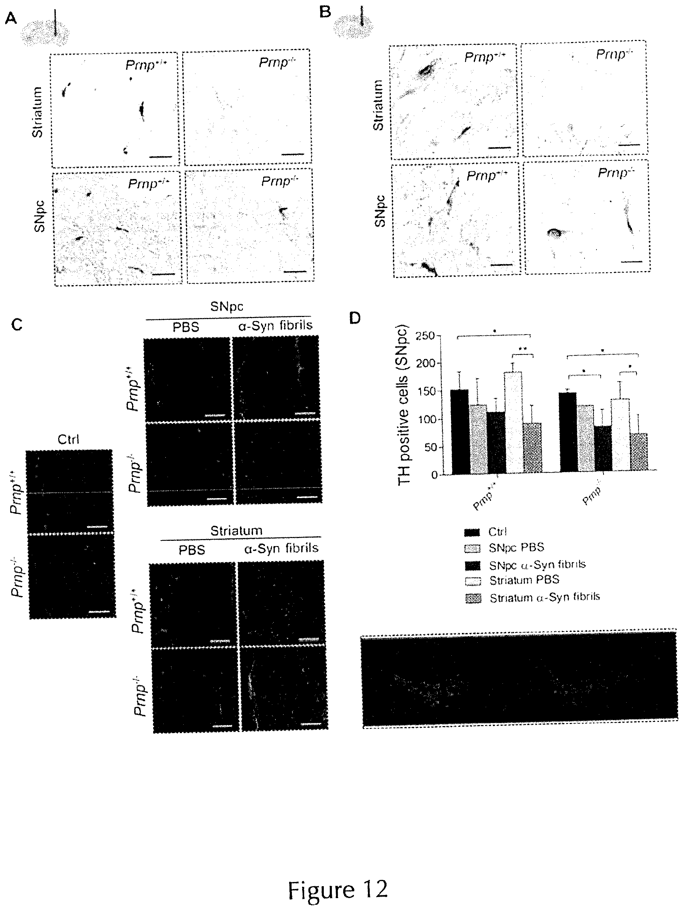

- Figure 12: shows for Example 4 that stereotaxic inoculation of sonicated α-Syn amyloid fibrils seeds the aggregation of phosphorylated α-Syn in the CNS, leading to astroglia activation and loss of DA neurons in the Substantia nigra (5 mpi). (A and B) Immunohistochemical staining with an antibody against phosphorylated α-Syn revealed the presence of pathologic α-Syn deposits in both, Substantia nigra and in the striatum when the mice were inoculated in the Substantia nigra, and in the striatum. Scale bars 25 µm. (C) Representative images of GFAP-immunostained samples show that there is a higher astroglial activation in α-Syn inoculated mice compared to PBS-inoculated or non-inoculated controls. Scale bars 100 µm. (D) Tyrosine hydroxylase (TH) immunoreactivity quantification in *Substantia nigra* and in ventral tegmental area (VTA, in Fig. 7) neurons show that seeded α-Syn pathology leads to loss of DA neurons after nigral or striatal inoculation. Values are given as means ± SD. Statistical significance was determined by using one-way ANOVA followed by Turkey's test. **P* < 0.05, ***P* < 0.01, ****P* < 0.001.

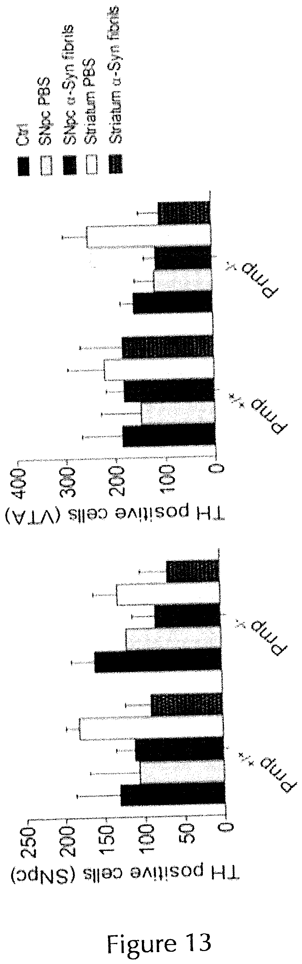

- Figure 13: shows for Example 4 that stereotaxic inoculation of sonicated α-Syn amyloid fibrils leads to loss of DA neurons in the Substantia nigra (5 mpi). Tyrosine hydroxylase (TH) immunoreactivity quantification in ventral tegmental area (VTA) neurons show that seeded α-Syn pathology leads to loss of DA neurons after nigral or striatal inoculation. Values are given as mean ± SD.

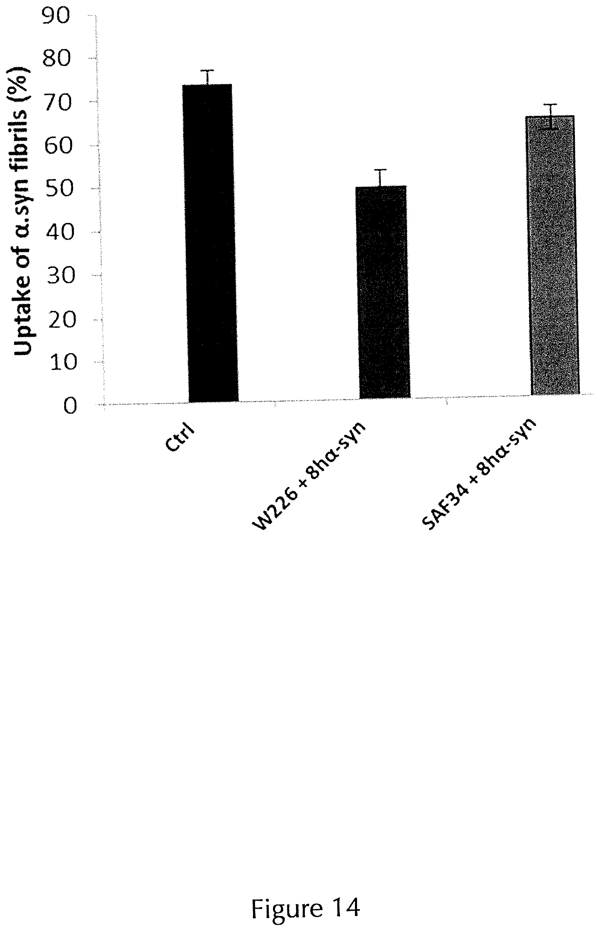

- Figure 14: shows for Example 5 that anti-PrP antibodies W226 and SAF34 reduce uptake of α-Syn fibrils.

### EXAMPLES

In the following, particular examples illustrating various embodiments and aspects of the invention are presented. However, the present invention shall not to be limited in scope by the specific embodiments described herein. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become readily apparent to those skilled in the art from the foregoing description, accompanying figures and the examples below. All such modifications fall within the scope of the appended claims.

### Example 1: Expression, purification and characterization of recombinant α-Syn proteins

First, highly pure rec human and mouse α-Syn protein were produced and subjected to the fibrillation process.

To this end, recombinant α-Synuclein (a-Syn) protein was purified from *Escherichia coli* BL21 (DE3) cells expressing mouse α-Syn construct from the pET11a expression vector. *E.coli* cells were grown in minimal medium at 37 °C in the presence of ampicillin (100 µg/mL) until OD600 of about 0.6, followed by induction with 0.6 mM IPTG for 5 hours. The protein was extracted from periplasm by osmotic shock as previously described (Huang C, Ren G, Zhou H, Wang CC. A new method for purification of recombinant human alpha-synuclein in Escherichia coli. Protein Expr Purif. 2005 Jul;42(1):173-7), followed by boiling for 20 min and ammonium sulfate precipitation. The protein was next purified by anion exchange chromatography (HiTrap Q FF column, GE Healthcare) and fractions were analyzed by SDS-PAGE. Finally, the protein was dialyzed against water, lyophilized and stored at -80 °C.

Prior to fibrillation, the protein was filtered with 0.22 µm syringe filter and the concentration was determined by absorbance measured at 280 nm. Purified mouse α-Syn (1.5 mg/mL) was incubated in the presence of 100 mM NaCl, 20 mM Tris-HCl pH 7.4 and 10 µM ThioflavinT (ThT). Reactions were performed in black 96-well plates with a clear bottom (Perkin Elmer), in the presence of one 3-mm glass bead (Sigma) in a final reaction volume of 200 µL. Plates were sealed and incubated in BMG FLUOstar Omega plate reader at 37 °C with cycles of 50 sec of shaking (400 rpm, double-orbital) and 10 sec of rest. ThT fluorescence measurements (excitation: 450 nm, emission 480 nm, bottom read) were taken every 15 min.

Obtained α-Syn proteins and fibrils were analyzed and characterized as shown in Fig. 1A-C. As shown in Fig. 1D, mouse α-Syn protein forms amyloid fibrils much faster compared to human α-Syn sequence. The resulting mouse α-Syn fibrils were subjected to biochemical analysis by Western blot. Results are shown in Fig. 1F.

Finally, the amyloids were structurally characterized by atomic force microscopy (AFM). Atomic Force Microscopy (AFM) was used to acquire high resolution three-dimensional reconstructions of α-Syn preparations. All AFM images were acquired using a commercially available microscope (Solver Pro AFM from NTMDT - NT-MDT Co. - Moscow - Russia) endowed with a closed-loop scanner. Measurements were carried out in air at room temperature working in dynamic mode. Cantilevers, characterized by a resonant frequency of about 90 kHz and a force constant of about 1.74 nN/nm (NSG03 series from NT-MDT - NT-MDT Co. - Moscow - Russia) were used working at low oscillation amplitudes with half free-amplitude set-point. High resolution images of 10×10 µm2 were 512×512 pixels frames acquired at 1 lines/second scan speed. All AFM data were analysed using Gwyddion, free SPM data analysis software (D. Nečas, P. Klapetek (2011), Gwyddion: an open-source software for SPM data analysis. Central European Journal of Physics 10, 181-188). Fibril length was evaluated as linear fibril's end-to-end distance and analyzed using commercial data analysis software (lgor Pro, Wavemetrics, US). Samples for AFM imaging were prepared by drop deposition of fibril solution on a ultra-flat mica surface. Briefly, 20 µL of solution were spotted onto a freshly cleaved piece of Goodfellow mica (8×8 mm2 side size) and left to adhere for 20 minutes. Subsequently a 60 µL drop of ethanol was placed on the sample to induce fibril precipitation for 5 minutes. Sample was thereafter blow-dried under a flow of nitrogen.

Results are shown in Fig. 2. Quantification showed that the sonication process breaks the fibrils into more homogenous smaller species (Fig. 2A and B).

### Example 2: α-Syn uptake is facilitated in cells expressing PrP^{C}

To assess whether PrP^{C} expression may facilitate α-Syn amyloid entrance in cells first an *in vitro* approach was used. To this end, N2a PrP^{+/+} and N2a PrP^{-/-} neuroblastoma (N2a) cells were incubated with recombinant (rec) mouse α-Syn amyloid fibrils for 24h. Uptake of α-Syn amyloid was compared in N2a PrP^{+/+} and N2a PrP^{-/-} cells, i.e. in N2a cells that constitutively express PrP^{C} and in the same cell line ablated for PrP^{C} (using CRISPR-Cas9-Based Knockout system) (M. Mehrabian et al. (2014), CRISPR-Cas9-based knockout of the prion protein and its effect on the proteome. PloS one 9, e114594).

To investigate whether mouse α-Syn fibrils are internalized by N2a cells confocal microscopy was used and the percentage of N2a cells that were able to take-up the amyloids were quantitatively analyzed. Briefly, cells were cultured on coverslips and treated with α-Syn fibrils (2 µM), afterwards the cells were fixed with 4% formaldehyde in PBS for 30 min. Cells were then washed three times with PBS (1X) followed by blocking in 5% Normal Goat Serum (NGS, ab7481, Abcam)/0.3% Triton X-100 for 1h, at room temperature (R.T.). Cells were incubated with primary antibodies diluted in 1% of blocking buffer (anti-PrP Ab W226, 1:500, anti-α-Syn Ab C-20-R, Santa Cruz, 1:1,000), followed by three washings with PBS and secondary antibody incubation (goat anti-mouse Alexa488, and goat anti-rabbit Alexa594, Life Technologies). To ensure that α-Syn preparations were within the cell cytoplasm a specific dye that labels the entire cell was used (HCS CellMask™ dye). Cells were mounted in Aqua Poly/Mount (Polysciences), and images were acquired using Leica confocal microscope (Leica TCS SP2, Wetzlar, Germany). The uptake quantification was performed in blind using Oil Immersion 63X objective on more than 200 cells per one single independent experiment (in total of *N*=3). Random fields per coverslips at 63x magnification were captured using Leica confocal microscope (Leica TCS SP2, Wetzlar, Germany). To observe internalized α-Syn fibrils, the coverslips were double-labeled with anti- α-Syn antibody and whole cytoplasmic dye CellMask. Cells considered α-Syn positive were those in which the aggregates were found in perinuclear zone. The images were acquired as 20-30 z-stacks of 0.22 µm, 1024 × 1024, and analyzed using Orthogonal Views function in Image J (NIH). Data are represented as % of total cell counted in three independent experiments.

Results are shown in Fig. 3. The data show that 82.1 ± 2.9 % of N2a PrP^{+/+} cells had α-Syn aggregates within the cytoplasm compared to only 31.8 ± 4.7 % of PrP^{-/-} after 24h of incubation. Only the removal of PrP^{C} resulted in lower α-Syn uptake, since in cells that were transfected with full-length PrP and in those infected with RML prion strain (D. A. Butler et al., Scrapie-infected murine neuroblastoma cells produce protease-resistant prion proteins. Journal of virology 62, 1558-1564 (1988)) the uptake was comparable (71.6 ± 16.5 % and 71.3 ± 4.0%, respectively). The non-sonicated α-Syn amyloids were internalized in similar percentage (Fig. 3A and B). In addition, both sonicated and non-sonicated mouse α-Syn amyloid preparations bound to the PrP^{C} on cell membrane, whereas in N2a PrP^{-/-} the interaction was hampered (Fig. 3C).

N2a PrP^{-/-} neuroblastoma (N2a) cells were transfected with full-length PrP (N2aPrPFL). As shown, in Fig. 3A and 4, reintroduction of full-length PrP into PrP^{-/-} cells rescued the hampered interaction of α-Syn amyloid and PrP^{C} observed in N2a PrP^{-/-} cells.

Next, the effect of mouse α-Syn preparations on endogenous PrP^{C} and transfected PrPFL protein levels was determined in N2a cells. To this end, cell lysates of N2a PrP^{+/+} cells and of transfected N2aPrPFL cells were prepared and analyzed by SDS-PAGE and Western blot, using W226 anti-PrP antibody. Briefly, after 4 days of treatment with different α-Syn preparations, medium was removed and the cells were washed twice with PBS 1X and lysed in lysis buffer. Total protein content of cell lysates was measured using bicinchoninic acid protein (BCA) quantification kit (Pierce) and stored at -20 °C until analysis. The total of 30 µg/mL of cell lysates were resuspended in Laemmli loading loading buffer, and boiled for 10 min at 95 °C. Subsequently the samples were loaded onto a 12% Tris-Glycine SDS-PAGE gel, and transferred onto nitrocellulose membrane (GE Healthcare), blocked using 5% non-fat milk (w/v) blocking solution for 1 h at room temperature with agitation followed by incubation with anti-PrP antibodies (W226, 1:1000; SAF43, 1:1000) or anti β-actin (1:50000, A3854 Sigma-Aldrich) diluted in blocking solution. Membranes were washed with TBST (0.1% Tween 20 in TBS), and incubated in horseradish-peroxidaseconjugated (HRP) goat anti-mouse secondary Ab (diluted 1:2000) for 1h. The membranes were washed in TBST and proteins were visualized following the manufacturer's instructions using Amersham ECL Western Blotting Detection Reagent (GE Healthcare) with UVITEC Cambridge. Quantitative densitometry analysis of proteins was performed using NIH Image software (Image) 1.50a, USA).

Results are shown in Fig. 5A and B. Even though PrP^{C} levels slightly increased after addition of α-Syn amyloids, PrP^{C} levels were maintained at basal levels in four subsequent serial passages (Fig 5A and B).

RT-PCR was performed to investigate whether or not the slight increase of protein levels were due to the mRNA increase. Total RNA extraction was performed using a ready-to-use TRIzol® Reagent (Invitrogen) following the Manufacture's instruction. Briefly, the medium was removed from plates with control cells and those treated with different α-syn preparations and the cells were washed twice with PBS 1 X. Subsequently, the cells were lysed using the TRIzol® Reagent. Following RNA isolation, a DNase I digestion was performed using 1 unit of enzyme per µg RNA for 10 min at room temperature, and RNA cleanup was implemented using RNeasy spin columns following the instructions. RNA concentration was determined using the NanoDrop system (Thermo Scientific). First-strand cDNA was synthesized using 4 µg of total RNA in a 20 µL reverse transcriptase reaction (RT+ samples) mixture following the instructor manual. For each sample a negative control was carried along by omission of the reverse transcriptase (RT- control). The cDNA was diluted to 1 ng/µL final concentration prior to Real-Time PCR reactions. Two ng RNA equivalent was added to the reaction mix including 2x iQ™ SYBR® Green Supermix (Bio-Rad Laboratories, Inc.), 400 nM of the corresponding forward and reverse primer (Sigma), and quantified in technical triplicates on an iQ5 Multicolor Real-Time PCR Detection System (Bio-Rad Laboratories, Inc.). After initial denaturation for 3 min at 95 °C, 45 cycles were performed at 95 °C for 10 sec and 60 °C for 1 min. Differential gene expression was normalized to GAPDH and ACTB expression. RT-controls were included in the plates for each primer pair and sample. The relative expression ratio was calculated using the ΔΔCT method. Significance was calculated with the unpaired Student's *t*-test (p < 0.05). The primers for *Prnp*-FW: 5'-GAGACCGATGTGAAGATGATGGA-3' (SEQ ID NO: 5) and RV: 5'-TAATAGGCCTGGGACTCCTTCTG-3' (SEQ ID NO: 6), ACTB-FW: 5'-GTTGCGTTACACCCTTTCTTG-3' (SEQ ID NO: 7), GAPDH-FW: 5'-CCTGCACCACCAACTGCTTA-3' (SEQ ID NO: 8).

Results are shown in Fig. 5C. ΔCT values for *Prnp* gene shows no variability among control and α-Syn treated samples. Accordingly, the slight increase of protein levels observed in the WB experiments was not due to the mRNA increase since levels of *Prnp* transcripts were not altered after treatment (Fig. 5C).

Next, the viability of the cell lines after exposure to exogenous α-Syn amyloids for 24 hours was tested. Cell viability was determined by 3-(4,5-dimethyl-2-thizolyl)-2,5-diphenyl-2H-tetrazolium bromide (MTT, Sigma Aldrich) assay following the manufacturer's instructions after 24 h treatment with α-Syn amyloids. Thirty thousand cells/well were treated with 2 µM (30 µg/mL) of α-syn fibrils in Costar™ 96-Well Plates (Thermo Fisher Scientific Inc.). Three independent experiments were performed in six technical replicas per condition (treated or un-treated). Water-insoluble colored formazan derivative was solubilized in DMSO : isopropanol (1 : 1). Absorbance of converted dye was measured at a wavelength of 570 nm. Viability was assessed in terms of % of control (untreated cells).

Results are shown in Fig. 6. Exposure to exogenous α-Syn amyloids (24 hours) showed no significant alteration of viability in cell lines used among different amyloid assemblies used (Fig. 6).

Cellular localization of α-Syn fibrils was investigated by confocal quadruple-labelled immunofluorescent imaging. Quadruple staining was carried out using 4% paraformaldehyde fixed cells. Nonspecific protein interactions were blocked with 10% normal goat serum (Sigma) and 0.3% Triton-X100 and incubated with the primary antibodies (D18 for PrPC, C-20-R for α-Syn, from Santa Cruz, EEA1-endosomal, Calenexin-endoplasmatic reticulum, Lamp1-lysosomal and M6PR-Golgi markers, from Abcam), in a humidified chamber at 4 °C overnight. Following washes in PBS the cells were incubated with secondary antibodies conjugated to biotin (1:500, ThermoFisher) followed by incubation with Alexa Fluor 647 Streptavidin conjugate (1:500, ThermoFisher). Coverslips were mounted in Aqua Poly/Mount (Polysciences), and images were acquired using C1 Nikon confocal microscope. Hippocampal neurons grown for 6 days *in vitro* (DIV), were fixed with 4% paraformaldehyde/PBS and immuno-stained with monoclonal MAP-2 antibody (Abcam), anti α-Syn antibody (C-20-R, Santa Cruz). Followed by the secondary antibody incubation (goat anti-mouse Alexa488, and goat anti-rabbit Alexa594, Life Technologies) and HCS CellMask™ dye (Thermo Fisher Scientific). Cells were mounted in Aqua Poly/Mount (Polysciences), and images were acquired using C1 Nikon confocal microscope.

As shown in Figures 7 (N2a PrP^{-/-} cells) and 8 (N2a PrP^{+/+} cells), α-Syn fibrils were predominantly found in lysosomal vesicles in the cytosol of N2a cells.

Next, α-Syn amyloid internalization in primary cultures of hippocampal neurons was investigated. To this end, both, PrP wild-type and knock out mice (FVB *Prnp*+/+ and *Prnp-*/*-*) were used. Briefly, hippocampi were dissected from 0-1-day-old postnatal animals. The isolated tissue was quickly sliced and digested in a digestion solution containing Trypsin (Sigma-Aldrich) and DNAse (Sigma-Aldrich). The reaction was stopped with Trypsin inhibitor (Sigma-Aldrich) and cells were mechanically dissociated in a dissection medium containing DNAse. After centrifugation, the cell pellet was resuspended in the culture medium and distributed in a 12 well Multiwell (Falcon), on coverslips (12 mm diameter) previously coated with polyornithine (50 µg/mL, Sigma-Aldrich) and Matrigel (2 % (w/v), BD). Plating was carried out at a density of 100.000 cells per coverslip. Hippocampal neurons cultures were incubated at 37 °C, in a humidified atmosphere with 5% CO2 in culture medium, consisting of MEM (Gibco), supplemented with 35 mM glucose (CarloErba Reagents), 1 mM Apo-Transferrin, 15 mM HEPES, 48 mM Insulin, 3 mM Biotin, 1 mM Vitamin B12 (Sigma-Aldrich) and 500 nM Gentamicin (Gibco) and 5-10 % dialyzed FBS (Gibco). Cortical neurons cultures were incubated at 37 °C, in a humidified atmosphere with 5 % CO2 in culture medium, consisting of Neurobasal medium (Gibco) supplemented with B27 (Gibco). With the primary cultures α-Syn uptake experiments were performed essentially as described above.

Results are shown in Fig. 3D and E. In FVB *Prnp*+/+ mice 62.9 ± 4.6 % of neurons internalized α-Syn fibrils (after 24 hours incubation), while the internalization in *Prnp*-/- neurons was less efficient (41.9 ± 8.5 %, Fig. 1D, E). Taken together these results indicate that PrP^{C} is required for the internalization of α-Syn fibrils.

### Example 3: Recombinant PrP binds α-Syn amyloids

Since confocal microscopy experiments revealed the co-localization between PrP^{C} attached to the cell membrane and exogenously added α-Syn amyloids, the nature of the molecular interaction between the two proteins was characterized in more detail. To this end, enzyme-linked immunosorbent assay (ELISA) and surface plasmon resonance (SPR) experiments were performed.

For ELISA Nunc-Immuno™ 96 MicroWell™ solid plates (Falcon) were coated o/n at +4 °C with 50uL (50ng) of MoPrP(23-231) and MoPrP(89-231) proteins in PBS. The day after wells were washed seven times with PBS-T (1X PBS + 0.3% Tween-20) and blocked with 5% BSA/PBS for 1hour at room temperature. After washing five times with PBS-T different forms of α-Syn (monomeric, non-sonicated, sonicated and long sonicated α-Syn fibrils) were added to MoPrP(23-231) and MoPrP(89-231) coated wells at different molar concentrations (1;1, 1:3, 1:10) and incubated for 30 min at 37 °C. Following α-Syn incubation wells were rinsed with PBS and incubated 1 hr with C-20-R (Santa Cruz, 1:1000) and W226 Ab (1:1,000, for control wells). After washing seven times with PBS secondary goat anti rabbit HRP, and goat anti mouse HRP were incubated at RT for 45min. HRP signal was visualized by determining the absorbance after sequential additions of 3,3',5,5'-tetramethylbenzidine (TMB, Sigma-Aldrich, 100 µL per well) and stopped with 100 µL 1 N sulfuric acid. The resulting yellow end product was read on SpectraMaxM5 (Molecular Devices) at 450 nm wavelength.

The results reveal that PrP^{C} binds fibrillary α-Syn *in vitro* (Fig. 9). Both rec full-length mouse PrP MoPrP(23-231) and truncated MoPrP(89-231), (Fig. 9) bind rec α-Syn amyloids in the biochemical study. More precisely, it was observed that the N-truncated rec PrP binds more weakly α-Syn fibrils. On the contrary, in the full-length rec PrP 1:3 dilution ratio led to a higher binding of the two proteins. These data suggest that the binding of α-Syn fibrils to PrP occurs mainly at the N-terminal part of PrP.

SPR experiments were performed to calculate binding constants. Biacore 2000 Surface Plasmon Resonance (SPR) instrument was used at a constant temperature of 25 °C. First, sonicated α-Syn fibrils (0.35 mg/mL diluited in 10 mM Na acetate, pH 4.0) were immobilized over the Biacore CM5 gold chip surface via amine coupling reaction (according to the Manufactures' instructions). Fibrils were injected in three distinct flow cells with different contact times in order to achieve different binding levels (-3300 RU, -4200 RU and -5000 RU in fc2, fc3 and fc4 respectively). Underivatized fc1 was used as reference cell and PBS buffer was flowed (5 µL/min) as running buffer over the surface. Binding affinity tests of α-Syn monomer (5 µM) and PrP (100 nM) were performed injecting analytes in running buffer at a flow rate of 50 µl/min for 3 minutes (association phase) and afterwards flushing with running buffer (dissociation phase). Binding affinity parameters were determined using the BIAevaluation software and the scientific data analysis software Igor Pro.

To illustrate the binding first sonicated fibrils were immobilized on the surface of one flow cell of a CM5 biosensor chip and monomeric α-Syn protein was added (Fig. 10A). Fig. 10B shows the binding of immobilized sonicated fibrils with rec full-length MoPrP(23-231). Two KD values (3.1 nM and 36.5 nM) were determined by the ratio between the two k*_{off}* values obtained and k*ₒₙ*. These KD values suggest that: (i) PrP - α-Syn amyloid binding occurs forming first weak interactions and then stronger interactions, and/or (ii) smaller α-Syn species establish stronger interactions with PrP while longer α-Syn amyloid fibrils form weaker interactions. The latter explanation may seem more plausible since α-Syn amyloid population is not homogeneous.

### Example 4: Detection of Proteinase K-resistant α-Syn deposits and other hallmarks of synucleinopathy in Prnp+/+ and Prnp-/- mice

Prnp^{-/-} mice neither propagate prions nor develop scrapie suggesting the central role of PrP^{C} in the development of prion diseases (H. Bueler et al., Normal development and behaviour of mice lacking the neuronal cell-surface PrP protein. Nature 356, 577-582 (1992); S. B. Prusiner et al., Ablation of the prion protein (PrP) gene in mice prevents scrapie and facilitates production of anti-PrP antibodies. Proc Natl Acad Sci U S A 90, 10608-10612 (1993)). The critical feature for the development of prion disease is a direct interaction of PrP^{C} with PrP^{Sc} which acts as a template for the conversion (L. Solforosi et al., Toward molecular dissection of PrPC-PrPSc interactions. J Biol Chem 282, 7465-7471 (2007)). However, there are no reports of role of PrP^{C} in synucleinopathies.

Therefore it was assessed whether the *in vitro* results might be recapitulated in an *in vivo* mouse model. Thus, stereotaxic injections of α-Syn amyloid fibrils were performed in Prnp^{+/+} and Prnp^{-/-} FVB mice both in the Substantia Nigra pars compacta (SNpc) and in the striatum.

To this end, female inbred FVB/N (Friend virus B-type susceptibility-NIH) FVB Prnp^{+/+} and FVB Prnp^{-/-} mice at 2 months-of-age were used. For stereotaxic surgery mice were subdivided into groups composed of 3 animals each and intraperitoneally anesthetized with a mixture of Xylazine (15 mg/kg) and Zoletil (15 mg/kg). Sonicated α-Syn short fibrils (15 µg) or sterile saline solution were stereotactically injected via a 10 µL Hamilton syringe into the Substantia Nigra pars compacta (AP -3.2, ML -1.2, DV -4.4 from Bregma) or in the striatum (AP+0.2, ML -2, DV -2.4 from Bregma) of the right hemisphere at a rate of 3 µL for 1 min, 3 µL for 2 min, 4 µL for 5 min. The needle was withdrawn of one coordinate and left for further 2 min before being totally removed. After recovery from surgery, animals were regularly monitored and sacrificed at 5 months-post-inoculation (mpi) by an overdose of Xylazine/Zoletil and transcardially perfused with 4% paraformaldehyde (PFA, pH 7.4). Brains were post fixed ON in PFA and sunk in 30% sucrose prior to be embedded in the Killik medium (WO1030799, Bio-Optica) and stored at -80 °C until use. Brains were cut with the Microm 550 cryostat to generate series of 10 µm slides thick coronal sections on Superfrost glass slides (Menzel-Gläser Adhesion Slides SuperFrost® Plus). Endogenous peroxidase inactivation was performed in 3% H₂O₂, 10% methanol in PBS for 10 minutes. For α-Syn detection, 5 µg/mL of Proteinase-K (PK) digestion was used to reveal aggregates. Blocking was performed in 0.05% Triton-X100, 5% normal goat serum (NGS, Sigma-Aldrich), 1% bovine albumin serum (BSA, Sigma-Aldrich) in PBS. Primary anti-α-Syn antibody (C20-R, Santa Cruz, 1:500) was incubated overnight. For phosphorylated α-Syn (p-α-Syn) detection, slides were previously treated with 70% formic acid for 30 min. Anti-phosphorylated Ser129 α-Syn antibody (P-Syn/81A, BioLegend, 1:700) was incubated overnight. Sections were then incubated with proper biotinylated-secondary antibodies (Sigma-Aldrich) followed by the VECTASTAIN® ABC Kit. Antibody labeling was revealed using 3'-diaminobenzidine (DAB; Sigma-Aldrich, SIGMAFAST™) as a chromogen. Slides were dehydrated as follow: 1 min in EtOH 50%, 1 min in EtOH 70%, 1 min in EtOH 90%, 1 min in EtOH 100%, 1 min in EtOH/Xilene (1:1), 2 min in Xilene, and mounted with Eukitt mounting medium (Bio Optica). Quantification of PK-resistant α-Syn aggregates was performed with ImageJ software (ImageJ 1.50a).

Results are shown in Fig. 11. Quantification of DAB-stained sections revealed presence of PK-resistant α-Syn aggregates in Prnp^{+/+} and Prnp^{-/-} mice 5 months post injection (mpi) (Fig. 11). Injection of α-Syn amyloid fibrils in mice induced the formation of LB-like aggregates in different brain areas (Fig. 11A). In agreement with *in vitro* results, the data show that in general Prnp^{-/-} mice accumulate less PK-resistant α-Syn aggregates in all areas analyzed (Fig. 11C and D).

Notably, when Prnp^{+/+} mice were injected within the SNpc, α-Syn aggregates were significantly higher (Fig. 11C). More precisely, we observed an almost complete absence of α-Syn aggregates in the striatum of mice that do not express PrP^{C}, while the mapping of PK-resistant α-Syn deposits in Prnp^{+/+} mice revealed the presence of α-Syn aggregates in the cortex, striatum, thalamus and hippocampus (Fig. 11B). In Prnp^{-/-} mice in all brain areas considered, α-Syn aggregates accumulate less (Fig. 11C). Phosphate-buffer saline (PBS) injections did not result in α-Syn aggregates accumulation in the two groups of animals. PK-resistant α-Syn was absent also in control animals.

Similarly, the stereotaxic injections in the striatum led to the formation of α-Syn aggregates in the brain. However, Prnp^{-/-} mice accumulated lower amount of aggregates compared to Prnp^{+/+} mice (Fig. 11D). In the Prnp^{-/-} mice, the number of α-Syn aggregates was significantly lower in four distinct brain areas (cortex, striatum, thalamus and hippocampus) (Fig. 11D). Generally, Prnp^{+/+} and Prnp^{-/-} animals inoculated within the striatum accumulated less α-Syn aggregates compared to those injected within the SNpc. In both cases the α-Syn-positive LB-like deposits were mainly ipsilateral; still, several α-Syn aggregates were present also in the contralateral regions to the injection site.

α-Syn amyloid fibril injection in the SNpc induced strong front and hind limb clasping in Prnp^{+/+} mice, while in the case of injection within the striatum only one Prnp^{+/+} mouse was clasping. On the contrary, clasping was never observed in Prnp^{-/-} or control mice.

Another hallmark of synucleinopathies is the presence of phosphorylated α-Syn deposits at residue S129 (pS129) (29). As shown in Fig. 12A and B, immunohistochemical analysis for pS129-α-Syn revealed a noticeable accumulation of large interstitial aggregates in Prnp^{+/+} mice and fewer pS129-α-Syn deposits in Prnp^{-/-} mice (Fig. 12A, B).

Next, astroglial activation was assessed. To this end, brain slices were blocked in 5% NGS, 1% BSA, 1% Triton-X100 in PBS and incubated ON with anti Glial Fibrillary Acidic Protein (GFAP, ab7260, 1:1000). Antibody staining was revealed after incubation with the appropriate secondary antibody Alexa 488 (Life Technologies, 1:500). 4',6-diamidino-2-phenylindole (DAPI, Sigma-Aldrich, SIGMAFAST™) was used for nuclear staining. Slides were coverslipped with VECTASHIELD Antifade Mounting Medium (H-1000, Vector Laboratories). Fluorescent images (1024x1024 pixels) were acquired with the C1 Nikon confocal. For the GFAP fluorescence a 20X objective was used and stacks of z-sections with an interval of 0.25 µm were sequentially scanned, to obtain representative images of the hippocampus.

Results are shown in Fig. 12C. α-Syn amyloid injection and the ensuing accumulation were accompanied by a strong astrogliosis that was more prominent in Prnp^{+/+} and in a lesser extend in Prnp^{-/-} mice (Fig. 12C).

To investigate levels of tyrosine hydroxylase (TH), brain slices were blocked in 5% NGS, 1% BSA, 1% Triton-X100 in PBS and incubated ON with the primary antibody anti Tyrosine Hydroxylase (TH, ab112, 1:1000). Antibody staining was revealed after incubation with the appropriate secondary antibody Alexa 488 (Life Technologies, 1:500). 4',6-diamidino-2-phenylindole (DAPI, Sigma-Aldrich, SIGMAFAST™) was used for nuclear staining. Slides were coverslipped with VECTASHIELD Antifade Mounting Medium (H-1000, Vector Laboratories). Fluorescent images (1024x1024 pixels) were acquired with the C1 Nikon confocal. TH labelled slides were sequentially scanned as 20 z-sections with an interval of 0.25µm) for all the area of interest (distance from Bregma: -2.92). TH positive (TH+) cells were counted with an automatic protocol with the Volocity 5.4 3D imaging software (PerkinElmer, Coventry, United Kingdom).

Results shown in Fig. 12D and 13 reveal that α-Syn aggregates deposition was accompanied by the gradual loss of tyrosine hydroxylase (TH) immunoreactivity, suggesting that α-Syn accumulation is linked to loss of DA neurons (Fig. 12D, Fig. 13).

### Example 5: Inhibition of uptake of α-Syn fibrils by anti-PrP antibodies

To investigate the influence of anti-PrP antibodies on binding of PrP to α-Syn and on α-Syn uptake, thirty thousand cells were cultured on coverslips and treated with non-specific mouse IgG or different amounts of W226 or SAF34 anti-PrP antibodies 30 min before addition of α-Syn amyloids.

Cells were then treated with α-Syn amyloid fibrils (2 µM) for 24 h before quantification. For quantification cells were fixed with 4% formaldehyde in PBS for 30 min. Cells were then washed three times with PBS (1X) followed by blocking in 5% Normal Goat Serum (NGS, ab7481, Abcam)/0.3% Triton X-100 for 1h, at room temperature (R.T.) Cells were incubated with primary antibodies diluted in 1% of blocking buffer (anti-α-Syn Ab C-20-R, Santa Cruz, 1 :1,000), followed by three washings with PBS and secondary antibody incubation (goat antimouse Alexa488, and goat anti-rabbit Alexa594, Life Technologies). To ensure that α-Syn preparations were within the cell cytoplasm a specific dye that labels the entire cell was used (HCS CellMask™ dye). Cells were mounted in Aqua Poly/Mount (Polysciences), and images were acquired using Leica confocal microscope (Leica TCS SP2, Wetzlar, Germany).

The uptake quantification was performed in blind using Oil Immersion 63X objective on more than 200 cells per one single independent experiment (in total of *N*=3). Random fields per coverslips at 63x magnification were captured using Leica confocal microscope (Leica TCS SP2, Wetzlar, Germany). To observe internalized α-Syn fibrils, the coverslips were double-labeled with anti- α-Syn antibody and whole cytoplasmic dye CellMask. Cells considered α-Syn positive were those in which the aggregates were found in perinuclear zone. The images were acquired as 20-30 z-stacks of 0.22 µm, 1024 × 1024, and analyzed using Orthogonal Views function in Image J (NIH). Data are represented as % of total cell counted in three independent experiments.

Results are shown in Fig. 14. Both antibodies, W226 and SAF34 were effective in reducing uptake of α-Syn amyloid fibrils. In particular, anti-PrP antibody W226 was effective in reducing α-Syn amyloid fibrils uptake by 50% compared to control experiments. These results show that anti-PrP antibodies are able to significantly reduce uptake and spreading of α-Syn amyloid fibrils. Accordingly, the data suggest that anti-PrP antibodies can be useful in the treatment of synucleinopathies.

**TABLE OF SEQUENCES AND SEQ ID NUMBERS (SEQUENCE LISTING):**

| SEQ ID NO | Sequence | Remarks |
|---|---|---|
| SEQ ID NO: 1 | | human PrP |
| SEQ ID NO: 2 | MANLGYWLLALFVTMWTDVGLCKKRPKPGGW NTGGSRYPGQGSPGGNRYPPQGGTWGQPHG GGWGQPHGGSWGQPHGGSWGQPHGGGW GQGGGTHNQWNKPSKPKTNLKHVAGAAAAG AVVGGLGGYMLGSAMSRPMIHFGNDWEDRYY RENMYRYPNQVYYRPVDQYSNQNNFVHDCV NITIKQHTVTTTTKGENFTETDVKMMERVVEQM CVTQYQKESQAYYDGRRSSSTVLFSSPPVILLISFLI FLIVG | mouse PrP |
| SEQ ID NO: 3 | KKRPKPGGWNTGGSRYPGQGSPGGNRYPPQG GGGWGQPHGGGWGQPHGGGWGQPHGG GWGQPHGGGWGQGGGTHSQWNKPSKPKT NMKHMAGAAAAGAVVGGLGGYMLGSAMSRP IIHFGSDYEDRYYRENMHRYPNQVYYRPMDEYS NQNNFVHDCVNITIKQHTVTTTTKGENFTETDV KMMERVVEQMCITQYERESQAYYQRGS | human PrP, amino acids 23 - 230 |
| SEQ ID NO: 4 | TFFYGGSRGKRNNFKTEEY | AN-2 peptide |
| SEQ ID NO: 5 | GAGACCGATGTGAAGATGATGGA | Prnp FW primer |
| SEQ ID NO: 6 | TAATAGGCCTGGGACTCCTTCTG | Prnp RV primer |
| SEQ ID NO: 7 | GTTGCGTTACACCCTTTCTTG | ACTB FW primer |
| SEQ ID NO: 8 | CCTGCACCACCAACTGCTTA | GAPDH FW primer |

## Claims

1. A ligand capable of binding to prion protein (PrP) for use in the prevention and/or treatment of a synucleinopathy.

2. The ligand for use according to claim 1, wherein the ligand is capable of binding to cellular prion protein (PrP^{C}).

3. The ligand for use according to claim 1 or 2, wherein the ligand is capable of binding to the N-terminal part and/or to the C-terminal part of the (cellular) prion protein.

4. The ligand for use according to any one of claims 1 - 3, wherein the ligand is capable of binding to two distinct epitopes of the (cellular) prion protein.

5. The ligand for use according to claim 4, wherein the ligand is capable of binding to an epitope in the N-terminal part of the cellular prion protein and to an epitope in the C-terminal part of the cellular prion protein.

6. The ligand for use according to any one of claims 1 - 5, wherein the ligand is not (neuro)toxic.

7. The ligand for use according to any one of claims 1 - 6, wherein the ligand is an anti-prion protein antibody, or an antigen-binding fragment thereof.

8. The ligand for use according to claim 7, wherein the ligand is a multispecific antibody or antigen-binding fragment thereof, preferably a bispecific antibody or antigen-binding fragment thereof.

9. The ligand for use according to claim 7 or 8, wherein the ligand is a monoclonal antibody or antigen-binding fragment thereof.

10. The ligand for use according to any one of claims 7 - 9, wherein the ligand is a human or humanized antibody or antigen-binding fragment thereof.

11. Conjugate comprising the ligand as defined in any one of claims 1 - 10 and an agent facilitating the passage across the blood-brain barrier for use in the prevention and/or treatment of a synucleinopathy.

12. Nucleic acid molecule comprising a polynucleotide encoding the ligand as defined in any one of claims 1 - 10 for use in the prevention and/or treatment of a synucleinopathy, wherein the ligand is a (poly)peptide, in particular an anti-prion protein antibody or an antigen-binding fragment thereof.

13. Pharmaceutical composition comprising
(i) the ligand as defined in any one of claims 1 - 10, the conjugate as defined in claim 11, the nucleic acid molecule as defined in claim 12, and
(ii) optionally, a pharmaceutically acceptable carrier, diluent and/or excipient for use in the prevention and/or treatment of a synucleinopathy.

14. The ligand for use according to any one of claims 1 - 10, the conjugate for use according to claim 11, the nucleic acid molecule for use according to claim 12 or the pharmaceutical composition for use according to claim 13, wherein the synucleinopathy is selected from Parkinson's disease, dementia with Lewy bodies, and multiple systems atrophy.

15. The ligand, the conjugate, the nucleic acid molecule or the pharmaceutical composition for use according to claim 14, wherein the synucleinopathy is Parkinson's disease.

16. The ligand, the conjugate, the nucleic acid molecule or the pharmaceutical composition for use according to any one of claims 1 - 15, wherein the ligand, the nucleic acid molecule or the pharmaceutical composition is administered intravenously or intramuscularly.

17. The ligand, the conjugate, the nucleic acid molecule or the pharmaceutical composition for use according to any one of claims 1 - 16, wherein the ligand, the nucleic acid molecule or the pharmaceutical composition is administered once or repeatedly.

18. A combination of
(i) the ligand as defined in any one of claims 1 - 10, the conjugate as defined in claim 11, the nucleic acid molecule as defined in claim 12 or the pharmaceutical composition as defined in claim 13, and
(ii) an antiparkinson medication
for use in the prevention and/or treatment of a synucleinopathy, in particular of Parkinson's disease.

19. The combination for use according to claim 18, wherein the ligand, the conjugate, the nucleic acid molecule or the pharmaceutical composition is administered intravenously or intramuscularly and the antiparkinson medication is administered orally.

20. The combination for use according to claim 18 or 19, wherein (i) the ligand, the conjugate, the nucleic acid molecule or the pharmaceutical composition and (ii) the antiparkinson medication are administered separately.

21. The combination for use according to any one of claims 18 - 20, wherein the antiparkinson medication is selected from dopaminergic precursors, COMT inhibitors, peripheral aromatic L-amino acid decarboxylase inhibitors, selective monoamine oxidase B inhibitors, dopamine receptor agonists, anticholinergics, positive allosteric modulators of mGluR4, and anti-α-synuclein antibodies.

22. The combination for use according to claim 21, wherein the antiparkinson medication is a dopaminergic precursor, preferably levodopa (L-DOPA).

23. A kit comprising
(i) the ligand as defined in any one of claims 1 - 10, the conjugate as defined in claim 11, the nucleic acid molecule as defined in claim 12 or the pharmaceutical composition as defined in claim 13; and
(ii) an antiparkinson medication.

24. The kit according to claim 23, wherein the antiparkinson medication is selected from dopaminergic precursors, COMT inhibitors, peripheral aromatic L-amino acid decarboxylase inhibitors, selective monoamine oxidase B inhibitors, dopamine receptor agonists, anticholinergics, positive allosteric modulators of mGluR4, and anti-α-synuclein antibodies.

25. The kit according to claim 23 or 24, wherein the antiparkinson medication is a dopaminergic precursor, preferably levodopa (L-DOPA).

26. Method for treating and/or preventing a synucleinopathy comprising administering to a subject the ligand as defined in any one of claims 1 - 10, the conjugate as defined in claim 11, the nucleic acid molecule as defined in claim 12 or the pharmaceutical composition as defined in claim 13.

27. Method according to claim 26, wherein the synucleinopathy is selected from Parkinson's disease, dementia with Lewy bodies, and multiple systems atrophy.

28. Method for reducing uptake of α-synuclein fibrils comprising administering to a subject the ligand as defined in any one of claims 1 - 10, the conjugate as defined in claim 11, the nucleic acid molecule as defined in claim 12 or the pharmaceutical composition as defined in claim 13.


SEQUENCE LISTING

<110>  Scuola Internazionale Superiore di Studi Avanzati

<120>  LIGANDS BINDING TO PRION PROTEIN FOR USE IN THE TREATMENT OF
       SYNUCLEINOPATHIES

<130>  ref. GLP G5-0623

<160>  8

<170>  PatentIn version 3.5

<210>  1
<211>  253
<212>  PRT
<213>  Homo sapiens PrP

<400>  1

<210>  2
<211>  254
<212>  PRT
<213>  Mus musculus PrP

<400>  2

Met Ala Asn Leu Gly Tyr Trp Leu Leu Ala Leu Phe Val Thr Met Trp
1               5                   10                  15

Thr Asp Val Gly Leu Cys Lys Lys Arg Pro Lys Pro Gly Gly Trp Asn
            20                  25                  30

Thr Gly Gly Ser Arg Tyr Pro Gly Gln Gly Ser Pro Gly Gly Asn Arg
        35                  40                  45

Tyr Pro Pro Gln Gly Gly Thr Trp Gly Gln Pro His Gly Gly Gly Trp
    50                  55                  60

Gly Gln Pro His Gly Gly Ser Trp Gly Gln Pro His Gly Gly Ser Trp
65                  70                  75                  80

Gly Gln Pro His Gly Gly Gly Trp Gly Gln Gly Gly Gly Thr His Asn
                85                  90                  95

Gln Trp Asn Lys Pro Ser Lys Pro Lys Thr Asn Leu Lys His Val Ala
            100                 105                 110

Gly Ala Ala Ala Ala Gly Ala Val Val Gly Gly Leu Gly Gly Tyr Met
        115                 120                 125

Leu Gly Ser Ala Met Ser Arg Pro Met Ile His Phe Gly Asn Asp Trp
    130                 135                 140

Glu Asp Arg Tyr Tyr Arg Glu Asn Met Tyr Arg Tyr Pro Asn Gln Val
145                 150                 155                 160

Tyr Tyr Arg Pro Val Asp Gln Tyr Ser Asn Gln Asn Asn Phe Val His
                165                 170                 175

Asp Cys Val Asn Ile Thr Ile Lys Gln His Thr Val Thr Thr Thr Thr
            180                 185                 190

Lys Gly Glu Asn Phe Thr Glu Thr Asp Val Lys Met Met Glu Arg Val
        195                 200                 205

Val Glu Gln Met Cys Val Thr Gln Tyr Gln Lys Glu Ser Gln Ala Tyr
    210                 215                 220

Tyr Asp Gly Arg Arg Ser Ser Ser Thr Val Leu Phe Ser Ser Pro Pro
225                 230                 235                 240

Val Ile Leu Leu Ile Ser Phe Leu Ile Phe Leu Ile Val Gly
                245                 250

<210>  3
<211>  208
<212>  PRT
<213>  Homo sapiens PrP

<400>  3

Lys Lys Arg Pro Lys Pro Gly Gly Trp Asn Thr Gly Gly Ser Arg Tyr
1               5                   10                  15

Pro Gly Gln Gly Ser Pro Gly Gly Asn Arg Tyr Pro Pro Gln Gly Gly
            20                  25                  30

Gly Gly Trp Gly Gln Pro His Gly Gly Gly Trp Gly Gln Pro His Gly
        35                  40                  45

Gly Gly Trp Gly Gln Pro His Gly Gly Gly Trp Gly Gln Pro His Gly
    50                  55                  60

Gly Gly Trp Gly Gln Gly Gly Gly Thr His Ser Gln Trp Asn Lys Pro
65                  70                  75                  80

Ser Lys Pro Lys Thr Asn Met Lys His Met Ala Gly Ala Ala Ala Ala
                85                  90                  95

Gly Ala Val Val Gly Gly Leu Gly Gly Tyr Met Leu Gly Ser Ala Met
            100                 105                 110

Ser Arg Pro Ile Ile His Phe Gly Ser Asp Tyr Glu Asp Arg Tyr Tyr
        115                 120                 125

Arg Glu Asn Met His Arg Tyr Pro Asn Gln Val Tyr Tyr Arg Pro Met
    130                 135                 140

Asp Glu Tyr Ser Asn Gln Asn Asn Phe Val His Asp Cys Val Asn Ile
145                 150                 155                 160

Thr Ile Lys Gln His Thr Val Thr Thr Thr Thr Lys Gly Glu Asn Phe
                165                 170                 175

Thr Glu Thr Asp Val Lys Met Met Glu Arg Val Val Glu Gln Met Cys
            180                 185                 190

Ile Thr Gln Tyr Glu Arg Glu Ser Gln Ala Tyr Tyr Gln Arg Gly Ser
        195                 200                 205

<210>  4
<211>  19
<212>  PRT
<213>  Artificial Sequence

<220>
<223>  AN-2 peptide

<400>  4

Thr Phe Phe Tyr Gly Gly Ser Arg Gly Lys Arg Asn Asn Phe Lys Thr
1               5                   10                  15

Glu Glu Tyr

<210>  5
<211>  23
<212>  DNA
<213>  Artificial Sequence

<220>
<223>  Prnp FW primer

<400>  5
gagaccgatg tgaagatgat gga                                              23

<210>  6
<211>  23
<212>  DNA
<213>  Artificial Sequence

<220>
<223>  Prnp RV primer

<400>  6
taataggcct gggactcctt ctg                                              23


<210>  7
<211>  21
<212>  DNA
<213>  Artificial Sequence

<220>
<223>  ACTB FW primer

<400>  7
   gttgcgttac acccttcctt g                                                 21


<210>  8
<211>  20
<212>  DNA
<213>  Artificial Sequence

<220>
<223>  GAPDH FW primer

<400>  8
cctgcaccac caactgctta                                                  20